# EUROPÄISCHE PATENTSCHRIFT

(11) **EP 1 530 574 B1**
(45) Veröffentlichungstag und Bekanntmachung des Hinweises auf die Patenterteilung: **22.11.2006**
(21) Anmeldenummer: 03797225.4
(22) Anmeldetag: 05.08.2003
(51) Int. Cl.: C07D 513/08, C07D 515/08, C07D 513/18, C07D 487/08, C07D 498/08, A61K 31/529, A61P 35/00

(54) **MAKROZYKLISCHE PYRIMIDINE, DEREN HERSTELLUNG UND VERWENDUNG ALS ARZNEIMITTEL**
MACROCYCLIC PYRIMIDINES, THE PRODUCTION THEREOF AND THE USE OF THE SAME AS MEDICAMENTS
PYRIMIDINES MACROCYCLIQUES, LEUR PRODUCTION ET LEUR UTILISATION COMME MEDICAMENT

(30) Priorität: 21.08.2002 DE 10239042
(43) Veröffentlichungstag der Anmeldung: 18.05.2005
(73) Patentinhaber: Schering Aktiengesellschaft, 13342 Berlin (DE)
(72) Erfinder: LÜCKING, Ulrich, 10245 Berlin (DE); SIEMEISTER, Gerhard, 13503 Berlin (DE); SCHÄFER, Martina, 13187 Berlin (DE); BRIEM, Hans, 28279 Bremen (DE)
(86) Internationale Anmeldenummer: PCT/EP2003/008664
(87) Internationale Veröffentlichungsnummer: WO 2004/026881

(56) Entgegenhaltungen:
- WO-A-00/39101
- WO-A-02/04429
- WO-A-02/20512
- US-A- 4 138 558
- JHAUMEER-LAULLOO, S.; WITVROUW, M.: "Synthesis and anti-HIV activity of novel macrocyclic benzamides with a disulfide bridge" INDIAN J. CHEM., Bd. 39B, Nr. 11, 2000, Seiten 842-846, XP009020915
- DATABASE CROSSFIRE BEILSTEIN [Online] Beilstein Institut zur Förderung der Chemischen Wissenschaften, Frankfurt am Main, DE; XP002261263 & BULL. SOC. SCI. PHOTOGR. JPN., Bd. 19, 1969, Seite 41

## Beschreibung

Die vorliegende Erfindung betrifft makrozyklische Pyrimidinderivate, deren Verfahren zur Herstellung sowie deren Verwendung als Medikament zur Behandlung verschiedener Erkrankungen.

Die Zyklin-abhängigen Kinasen (cyclin-dependent kinase, CDK) sind eine Enzymfamilie, die eine wichtige Rolle bei der Regulation des Zellzyklus spielt und somit ein besonders interessantes Ziel für die Entwicklung kleiner inhibitorischer Moleküle darstellt. Selektive Inhibitoren der CDKs können zur Behandlung von Krebs oder anderen Erkrankungen, die Störungen der Zellproliferation zur Ursache haben, verwendet werden.

Rezeptortyrosinkinasen und deren Liganden, die spezifisch die Funktion von Endothelzellen regulieren, sind in entscheidender Weise an der physiologischen, wie auch der pathogenen Angiogenese beteiligt. Von besonderer Bedeutung ist hier das Vascular Endothelial Growth Factors (VEGF) / VEGF-Rezeptor System. In pathologischen Situationen, die mit einer verstärkten Neovaskularisation einhergehen, wie z.B. Tumorerkrankungen, wurde eine erhöhte Expression von angiogenen Wachstumsfaktoren und ihrer Rezeptoren gefunden. Inhibitoren des VEGF/VEGF-Rezeptorsystems können die Ausbildung eines Blutgefäßsystems im Tumor inhibieren, damit den Tumor von der Sauerstoff- und Nährstoffversorgung abscheiden und somit das Tumorwachstum inhibieren.

Pyrimidine und Analoga sind bereits als Wirkstoffe beschrieben, wie beispielsweise die 2-Anilino-Pyrimidine als Fungizide (DE 4029650) oder substituierte Pyrimidinderivate zur Behandlung von neurologischen oder neurodegenerativen Erkrankungen (WO 99/19305). Als CDK-Inhibitoren werden unterschiedlichste Pyrimidinderivate beschrieben, beispielsweise Bis(anilino)pyrimidinderivate (WO 00/12486), 2-Amino-4-substituierte Pyrimidine (WO 01/14375), Purine (WO 99/02162), 5-Cyano-Pyrimidine (WO 02/04429), Anilinopyrimidine (WO 00/12486) und 2-Hydroxy-3-N,N-dimethylaminopropoxy-Pyrimidine (WO 00/39101).

Die Aufgabe der vorliegenden Erfindung ist es Verbindungen bereitzustellen, die verbesserte Eigenschaften als die bereits bekannten Verbindungen haben. Überraschenderweise wurde nun gefunden, dass die erfindungsgemäßen Substanzen entweder Zyklin-abhängige Kinasen und VEGF-Rezeptortyrosinkinasen oder Zyklin-abhängige Kinasen oder VEGF-Rezeptortyrosinkinasen bereits im nanomolaren Bereich inhibieren und somit die Proliferation der Tumorzellen und/oder die Tumorangiogenese inhibieren können. Damit unterscheiden sie sich deutlich von anderen bereits bekannten CDK-, oder VEGF-R Inhibitoren, wie z.B. Olomoucin und Roscovitin.

Es wurde nun gefunden, dass Verbindungen der Ansprüche 1 bis 3 sowie deren Diastereomeren, Enantiomeren und Salze die bekannten Nachteile überwinden.

Unter Alkyl ist jeweils ein geradkettiger oder verzweigter Alkylrest, wie beispielsweise Methyl, Ethyl, Propyl, Isopropyl, Butyl, Isobutyl, sek. Butyl, tert. Butyl, Pentyl, Isopentyl, Hexyl, Heptyl, Octyl, Nonyl und Decyl, zu verstehen.

Unter Alkoxy ist jeweils ein geradkettiger oder verzweigter Alkoxyrest, wie beispielsweise Methyloxy, Ethyloxy, Propyloxy, Isopropyloxy, Butyloxy, Isobutyloxy, sek. Butyloxy, Pentyloxy, lsopentyloxy, Hexyloxy, Heptyloxy, Octyloxy, Nonyloxy, Decyloxy, Undecyloxy oder Dodecyloxy zu verstehen.

Unter Alkylthio ist jeweils ein geradkettiger oder verzweigter Alkylthiorest, wie beispielsweise Methylthio, Ethylthio, Propylthio, Isopropylthio, Butylthio, Isobutylthio, sek. Butylthio, tert.-Butylthio, Pentylthio, lsopentylthio oder Hexylthio zu verstehen.

Unter Cycloalkyl sind monocyclische Alkylringe wie Cyclopropyl, Cyclobutyl, Cyclopentyl, Cyclohexyl oder Cycloheptyl, Cyclooctyl, Cyclononyl oder Cyclodecyl, aber auch bicyclische Ringe oder tricyclische Ringe wie zum Beispiel Adamantanyl zu verstehen.

Heterocycloalkyl steht für einen 3 - 12 Kohlenstoffatome umfassenden Alkylring, der anstelle des Kohlenstoffes ein oder mehrere, gleich oder verschiedene Heteroatome, wie z. B. Sauerstoff, Schwefel oder Stickstoff enthält.
Als Heterocycloalkyle seien z. B. genannt: Oxiranyl, Oxethanyl, Aziridinyl, Azetidinyl, Tetrahydrofuranyl, Pyrrolidinyl, Dioxolanyl, Imidazolidinyl, Pyrazolidinyl, Dioxanyl, Piperidinyl, Morpholinyl, Dithianyl, Thiomorpholinyl, Piperazinyl, Trithianyl, Chinuclidinyl etc.

Unter den Ringsystemen, bei denen gegebenenfalls ein- oder mehrere mögliche Doppelbindungen im Ring enthalten sein können, sind zum Beispiel Cycloalkenyle wie Cyclopropenyl, Cyclobutenyl, Cyclopentenyl, Cyclohexenyl, Cycloheptenyl zu verstehen, wobei die Anknüpfung sowohl an der Doppelbindung wie auch an den Einfachbindungen erfolgen kann.

Unter Halogen ist jeweils Fluor, Chlor, Brom oder Jod zu verstehen.

Die Alkenyl- und Alkinyl-Substituenten sind jeweils geradkettig oder verzweigt, wobei beispielsweise folgenden Reste gemeint sind: Vinyl, Propen-1-yl, Propen-2-yl, But-1-en-1-yl, But-1-en-2-yl, But-2-en-1-yl, But-2-en-2-yl, 2-Methyl-prop-2-en-1-yl, 2-Methyl-prop-1-en-1-yl, But-1-en-3-yl, Ethinyl, Prop-1-in-1-yl, But-1-in-1-yl, But-2-in-1-yl, But-3-en-1-yl, Allyl.

Der Arylrest hat jeweils 6 - 12 Kohlenstoffatome wie beispielsweise Naphthyl, Biphenyl und insbesondere Phenyl.

Der Heteroarylrest umfaßt jeweils 3 - 18 Ringatome und kann anstelle des Kohlenstoffs ein- oder mehrere, gleiche oder verschiedene Heteroatome, wie Sauerstoff, Stickstoff oder Schwefel im Ring enthalten, und kann mono-, bi- oder tricyclisch sein, und kann zusätzlich jeweils benzokondensiert sein.

### Beispielsweise seien genannt:

Thiophenyl, Furanyl, Pyrrolyl, Oxazolyl, Thiazolyl, Imidazolyl, Pyrazolyl, Isoxazolyl, Isothiazolyl, Oxadiazolyl, Triazolyl, Thiadiazolyl, etc. und Benzoderivate davon, wie z. B. Benzofuranyl, Benzothienyl, Benzoxazolyl, Benzimidazolyl, Indazolyl, Indolyl, Isoindolyl, etc.; oder Pyridyl, Pyridazinyl, Pyrimidinyl, Pyrazinyl, Triazinyl, etc. und Benzoderivate davon, wie z. B. Chinolyl, Isochinolyl, etc.; oder Azocinyl, Indolizinyl, Purinyl, etc. und Benzoderivate davon; oder Cinnolinyl, Phthalazinyl, Chinazolinyl, Chinoxalinyl, Naphthyridinyl, Pteridinyl, Carbazolyl, Acridinyl, Phenazinyl, Phenothiazinyl, Phenoxazinyl, Xanthenyl, Oxepinyl, 1,4-Benzodioxan etc.

Ist eine saure Funktion enthalten, sind als Salze die physiologisch verträglichen Salze organischer und anorganischer Basen geeignet, wie beispielsweise die gut löslichen Alkali- und Erdalkalisalze sowie N-Methyl-glukamin, Dimethyl-glukamin, Ethyl-glukamin, Lysin, 1,6-Hexadiamin, Ethanolamin, Glukosamin, Sarkosin, Serinol, Tris-hydroxy-methyl-amino-methan, Aminopropandiol, Sovak-Base, 1-Amino-2,3,4-butantriol.

Ist eine basische Funktion enthalten, sind die physiologisch verträglichen Salze organischer und anorganischer Säuren geeignet wie Salzsäure, Schwefelsäure, Phosphorsäure, Zitronensäure, Weinsäure u.a.

Soweit die Herstellung der erfindungsgemäßen Verbindungen der Ansprüche 1 bis 3 nicht beschrieben ist, erfolgt diese analog bekannter Methoden.

Die Strukturaufklärung der makrozyklischen Riechstoffe *Muskon* und *Zibeton* durch *Ruzicka* ((a) Ruzicka, L. *Helv. Chim. Acta* 1926, *9*, 715. (b) Ruzicka, L. *Helv. Chim. Acta* 1926, 9, 249) im Jahre 1926 markiert den Beginn der Chemie der Makrozyklen.

Im allgemeinen werden mittlere (8 - 11-gliedrige) und große (≥ 12-gliedrige) Ringe als Makrozyklen bezeichnet. Die etablierten Verfahren zur Synthese von Makrozyklen beruhen zum Teil auf Ringerweiterungsreaktionen (Hesse, M. Ring Enlargement in Organic Chemistry, VCH, Weinheim, 1991), seltener auf Ringkontraktionen (Hayashi, T. *J*. *Org. Chem.* 1984, *49*, 2326).
Die am häufigsten angewandte Methode ist die Zyklisierung von bifunktionellen azyklischen Vorläufern (Reviews zur Synthese von Makrozyklen: (a) Roxburgh, C.J. *Tetrahedron* 1995, *51*, 9767. (b) Meng, Q. *Top. Curr. Chem.* 1991, *161*, 107. (c) Paterson, I. *Tetrahedron* 1985, *41*, 3569. (d) Masamune, S. *Angew. Chem.* 1977, *89*, 602. (e) Nicolaou, K.C. *Tetrahedron* 1977, 33, 683. (f) Ruggli, P. Liebigs Ann. Chem. 1912, 92).

Schnell und mit sehr guten Ausbeuten kann die Herstellung der erfindungsgemäßen Verbindungen der Ansprüche 1 bis 3 durch den Ringschluss über die 2-Position des Pyrimidins azyklischer Vorläufer der Formeln (VIII) oder (IX) erfolgen, indem man
a) Verbindungen der Formel VIII 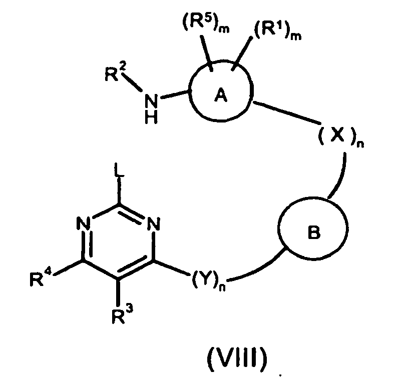 in der R¹, R², R³, R⁴, R⁵, X, Y, A, B, m und n die in den Verbindungen der Ansprüche 1 bis 3 verwirklichten Bedeutungen haben und L für eine Abgangsgruppe steht, mit einer geeigneten Säure zu Verbindungen der Ansprüche 1 bis 3 zyclisiert, oder
b) den azyclischen Vorläufer der Formel (IX) 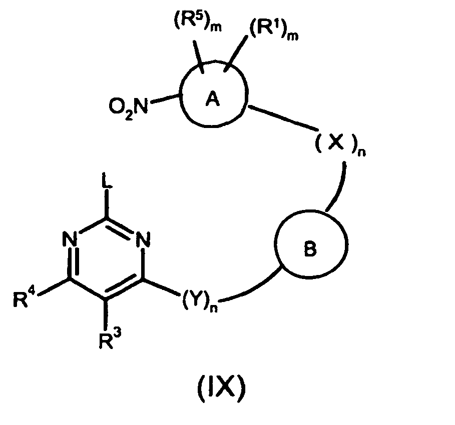 in der in der R¹, R³, R⁴, R⁵, X, Y, A, B, m und n die in den Verbindungen der Ansprüche 1 bis 3 verwirklichten Bedeutungen haben und L für eine Abgangsgruppe steht, in einem geeigneten Lösungsmittel und einem geeignete Reduktionsmittel bei 0 °C bis Reflux zunächst zum Amin reduziert und anschließend das intermediär gebildete Amin zu den Verbindungen der Ansprüche 1 bis 3 zyclisiert.

Die Herstellung der erfindungsgemäßen Verbindungen der Ansprüche 1 bis 3 ist ebenfalls Gegenstand der vorliegenden Erfindung.

Geeignete Lösungsmittel sind zum Beispiel einfache Ketone wie Aceton, Alkohole wie z.B. Ethanol oder Butanol, Ester wie zum Beispiel Essigester, aromatische Lösungsmittel wie zum Beispiel Toluol oder Benzol sowie polar aprotische Lösungsmittel wie Acetonitril, DMSO, DMF oder N-Methylpyrrolidine oder Gemische dieser Lösungsmittel, auch unter Zusatz von Wasser.

Geeignete Reduktionsmittel sind zum Beispiel Ti(III)Cl oder Sn(II)Cl.

Unter Abgangsgruppen in der Bedeutung von L sind zum Beispiel eine Halogeno- oder Sulphonyloxy-Gruppe, wie Fluor, Chlor, Brom, lod, Methansulphonyloxy, Toluol-4-sulphonyloxy, Trifluoromethylsulphonyloxy, etc. zu verstehen.
Für die Zyklisierung zur Anwendung kommende Säuren sind zum Beispiel geeignete Lewis Säuren, wie anorganische Säuren wie Hydrogenchlorid, Hydrogenbromid, Schwefelsäure, organische Säuren wie Essigsäure, Ameisensäure, BBr₃, Metallsalze wie Ti(III)Cl, Sn(II)Cl, Ln(III)Otf, etc.

Die für die Herstellung der erfindungsgemäßen Verbindungen der Ansprüche 1 bis 3 vorzugsweise verwendeten Zwischenprodukte der Formeln II, III, IV, V, VI und VII 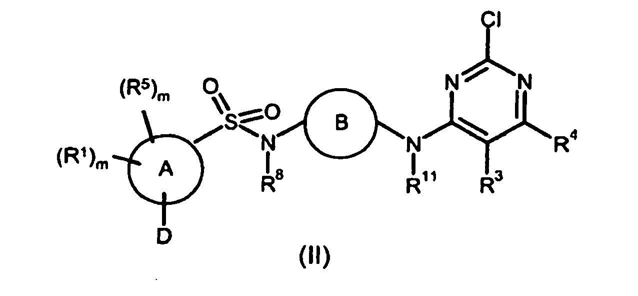 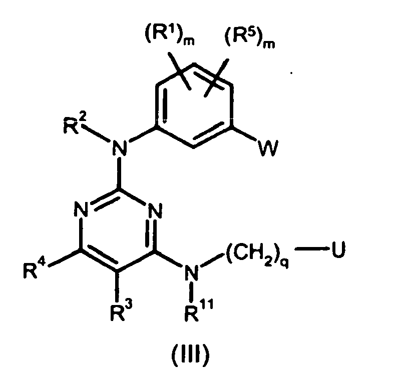 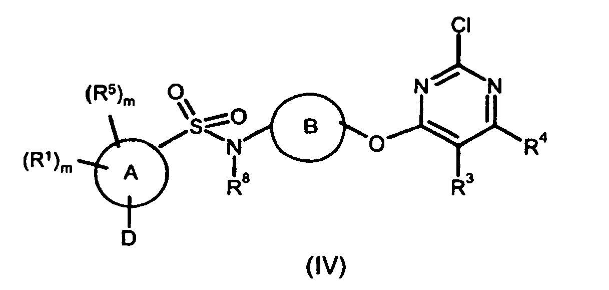 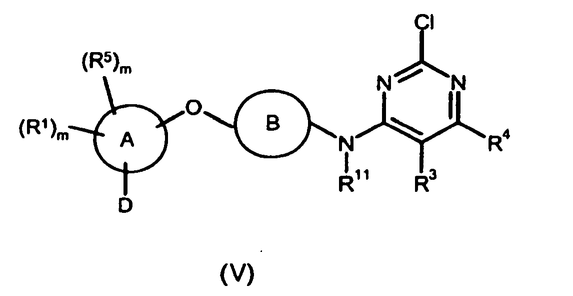 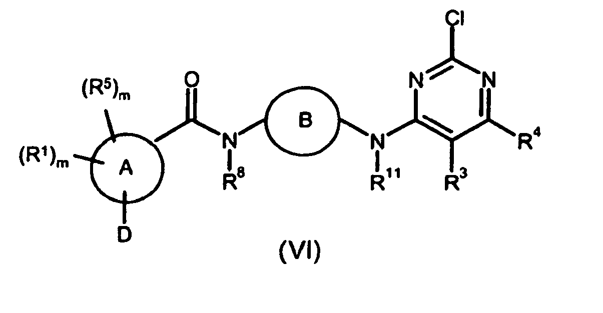 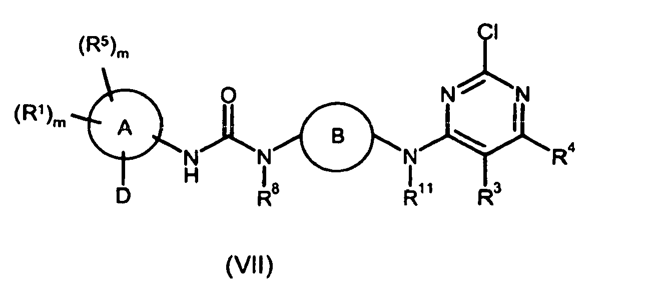 oder

in denen R¹, R², R³, R⁴, R⁵, R⁸, R¹¹, A, B und m die in den Verbindungen der Ansprüche 1 bis 3 verwirklichten Bedeutungen haben und D für -NH₂, -NAc oder - NO₂, q für 1 bis 12, U für die Gruppe -OH, -CO₂H, -CO₂-C1-C₆-Alkyl, -SO₂Cl, -SO₂F, -SO₃H oder 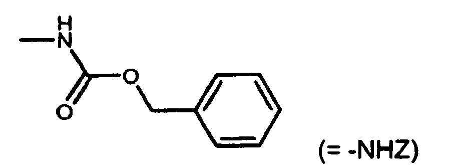 und W für die Gruppe -OH -OH, -CO₂H, -CO₂-C1-C₆-Alkyl, -SO₂Cl, -SO₂F oder - SO₃H steht,
sowie deren Diastereomeren, Enantiomeren und Salze sind ebenfalls Gegenstand der vorliegenden Erfindung.

Insbesondere werden vorzugsweise solche Zwischenprodukte der Formeln II, III, IV, V, VI und VII verwendet, in denen
A für Phenylen oder Thiophenylen steht und
R¹, R², R³, R⁴, R⁵, R⁸, R¹¹ und m die in den Verbindungen der Ansprüche 1 bis 3 verwirklichten Bedeutungen haben und D für-NH₂, -NAc oder-NO₂, q für 1 bis 12,
U für die Gruppe -OH, -CO₂H, -CO₂-C1-C₆-Alkyl, -SO₂Cl, -SO₂F, -SO₃H oder 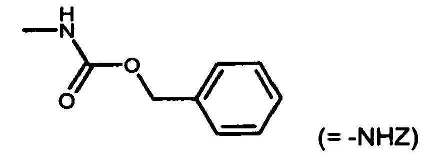 und
W für die Gruppe -OH -OH, -CO₂H, -CO₂-C1-C₆-Alkyl, -SO₂Cl, -SO₂F oder -SO₃H steht,
sowie deren Diastereomeren, Enantiomeren und Salze.

Die erfindungsgemäßen Verbindungen können zum einen Zyklin-abhängige Kinasen inhibieren. Der eukaryote Zellteilungszyklus stellt die Duplikation des Genoms und seine Verteilung auf die Tochterzellen sicher, indem er eine koordinierte und regulierte Abfolge von Ereignissen durchläuft. Der Zellzyklus wird in vier aufeinanderfolgende Phasen eingeteilt: die G1 Phase repräsentiert die Zeit vor der DNA-Replikation, in der die Zelle wächst und für externe Stimuli empfänglich ist. In der S Phase repliziert die Zelle ihre DNA, und in der G2 Phase bereitet sie sich auf den Eintritt in die Mitose vor. In der Mitose (M Phase) wird die replizierte DNA getrennt und die Zellteilung vollzogen.

Die Zyklin-abhängigen Kinasen (CDKs), eine Familie von Serin/Threonin-Kinasen, deren Mitglieder die Bindung eines Zyklins (Cyc) als regulatorische Untereinheit zu ihrer Aktivierung benötigen, treiben die Zelle durch den Zellzyklus. Unterschiedliche CDK/Cyc Paare sind in den verschiedenen Phasen des Zellzyklus aktiv. Für die grundlegende Funktion des Zellzyklus bedeutende CDK/Cyc Paare sind beispielsweise CDK4(6)/CycD, CDK2/CycE, CDK2/CycA, CDK1/CycA und CDK1/CycB. Einige Mitglieder der CDK-Enzymfamilie haben eine regulatorische Funktion indem sie die Aktivität der vorgenannten Zellzyklus-CDKs beeinflussen, während anderen Mitgliedern der CDK-Enzymfamlie noch keine bestimmte Funktion zugeordnet werden konnte. Eine von diesen, CDK5, zeichnet sich dadurch aus, dass sie eine atypische, von den Zyklinen abweichende, regulatorische Untereinheit besitzt (p35), und ihre Aktivität im Gehirn am höchsten ist.

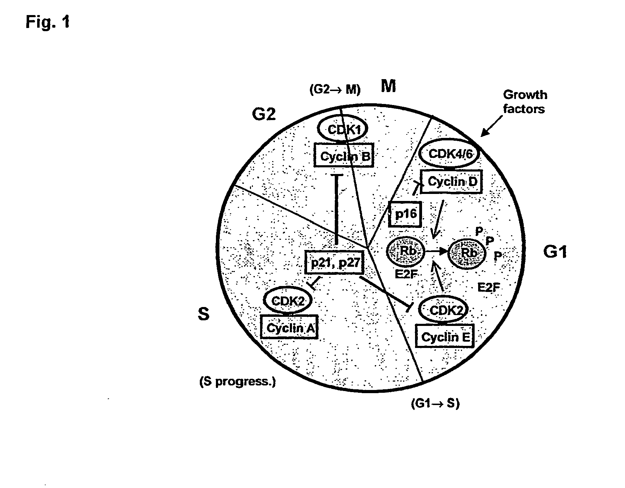

Der Eintritt in den Zellzyklus und das Durchlaufen des "Restriction Points", der die Unabhängigkeit einer Zelle von weiteren Wachstumssignalen für den Abschluß der begonnenen Zellteilung markiert, werden durch die Aktivität der CDK4(6)/CycD und CDK2/CycE Komplexe kontrolliert. Das wesentliche Substrat dieser CDK-Komplexe ist das Retinoblastoma-Protein (Rb), das Produkt des Retinoblastoma Tumorsuppressor Gens. Rb ist ein transkriptionelles Ko-Repressor Protein. Neben anderen noch weitgehend unverstandenen Mechanismen, bindet und inaktiviert Rb Transkriptionsfaktoren vom E2F-Typ, und bildet transkriptionelle Repressorkomplexe mit Histon-Deacetylasen (HDAC) (Zhang H.S. *et al*. (2000). Exit from G1 and S phase of the cell cycle is regulated by repressor complexes containing HDAC-Rb-hSWI/SNF and Rb-hSWI/SNF. *Cell* 101, 79-89). Durch die Phosphorylierung des Rb durch CDKs werden gebundene E2F Transkriptionsfaktoren freigesetzt und führen zu transkriptioneller Aktivierung von Genen, deren Produkte für die DNA Synthese und die Progression durch die S-Phase benötigt werden. Zusätzlich bewirkt die Rb-Phosphorylierung die Auflösung der Rb-HDAC Komplexe, wodurch weitere Gene aktiviert werden. Die Phosphorylierung von Rb durch CDK's ist mit dem Überschreiten des "Restriction Points" gleichzusetzen. Für die Progression durch die S-Phase und deren Abschluß ist die Aktivität der CDK2/CycE und CDK2/CycA Komplexe notwendig, z. B. wird die Aktivität der Transkriptionsfaktoren vom E2F-Typ mittels Phosphorylierung durch CDK2/CycA abgeschaltet, sobald die Zellen in die S-Phase eingetreten sind. Nach vollständiger Replikation der DNA steuert die CDK1 im Komplex mit CycA oder CycB den Eintritt und das Durchlaufen der Phasen G2 und M (Fig. 1).

Entsprechend der außerordentlichen Bedeutung des Zellteilungszyklus ist das Durchlaufen des Zyklus streng reguliert und kontrolliert. Die Enzyme, die für die Progression durch den Zyklus notwendig sind, müssen zu dem richtigen Zeitpunkt aktiviert werden, und auch wieder abgeschaltet werden sobald die entsprechende Phase durchlaufen ist. Entsprechende Kontrollpunkte ("Checkpoints") arretieren die Progression durch den Zellzyklus falls DNA-Schäden detektiert werden, oder die DNA-Replikation, oder der Aufbau des Spindelapparates noch nicht beendet ist. Die Aktivität der CDKs wird durch verschiedene Mechanismen, wie Synthese und Degradation der Zykline, Komplexierung der CDKs mit den entsprechenden Zyklinen, Phosphorylierung und Dephosphorylierung regulatorischer Threonin- und Tyrosin-Reste, und die Bindung natürlicher inhibitorischer Proteine, direkt kontrolliert. Während die Proteinmenge der CDKs in einer proliferierenden Zelle relativ konstant ist, oszilliert die Menge der einzelnen Zykline mit dem Durchlaufen des Zyklus. So wird zum Beispiel die Expression von CycD während der frühen G1 Phase durch Wachstumsfaktoren stimuliert, und die Expression von CycE wird nach Überschreiten des "Restriction Points" durch die Aktivierung der Transkriptionsfaktoren vom E2F-Typ induziert. Die Zykline selbst werden durch Ubiquitin-vermittelte Proteolyse abgebaut. Aktivierende und inaktivierende Phosphorylierungen regulieren die Aktivität der CDK's, zum Beispiel phosphorylieren CDK-aktivierende Kinasen (CAKs) Thr160/161 der CDK1, wohingegen die Familie der Wee1/Myt1 Kinasen CDK1 durch Phosphorylierung von Thr14 und Tyr15 inaktivieren. Diese inaktivierenden Phosphorylierungen können durch cdc25 Phosphatasen wieder aufgehoben werden. Sehr bedeutsam ist die Regulation der Aktivität der CDK/Cyc-Komplexe durch zwei Familien natürlicher CDK Inhibitorproteine (CKIs), den Proteinprodukten der p21 Genfamilie (p21, p27, p57) und der p16 Genfamilie (p15, p16, p18, p19). Mitglieder der p21 Familie binden an Zyklin-Komplexe der CDKs 1,2,4,6, inhibieren aber nur Komplexe die CDK1 oder CDK2 enthalten. Mitglieder der p16 Familie sind spezifische Inhibitoren der CDK4- und CDK6-Komplexe.

Oberhalb dieser komplexen, direkten Regulation der Aktivität der CDKs liegt die Ebene der Kontrollpunkt-Regulation. Kontrollpunkte erlauben der Zelle das geordnete Ablaufen der einzelnen Phasen während des Zellzykluses zu verfolgen. Die wichtigsten Kontrollpunkte liegen am Übergang von G1 nach S und von G2 nach M. Der G1-Kontrollpunkt stellt sicher, dass die Zelle keine DNA-Synthese beginnt falls sie nicht entsprechend ernährt ist, mit anderen Zellen oder dem Substrat korrekt interagiert, und ihre DNA intakt ist. Der G2/M Kontrollpunkt stellt die vollständige Replikation der DNA und den Aufbau der mitotischen Spindel sicher, bevor die Zelle in die Mitose eintritt. Der G1 Kontrollpunkt wird von dem Genprodukt des p53 Tumorsuppressorgens aktiviert. p53 wird nach Detektion von Veränderungen im Metabolismus oder der genomischen Integrität der Zelle aktiviert und kann entweder einen Stopp der Zellzyklusprogression oder Apoptose auslösen. Dabei spielt die transkriptionelle Aktivierung der Expression des CDK Inhibitorproteins p21 durch p53 eine entscheidende Rolle. Ein zweiter Zweig des G1 Kontrollpunktes umfaßt die Aktivierung der ATM und Chk1 Kinasen nach DNA-Schädigung durch UV-Licht oder ionisierende Strahlung und schließlich die Phosphorylierung und den nachfolgenden proteolytischen Abbau der cdc25A Phosphatase (Mailand N. *et al*. (2000). Rapid destruction of human cdc25A in response to DNA damage. *Science* 288, 1425-1429). Daraus resultiert eine Arretierung des Zellzykluses, da die inhibitorische Phosphorylierung der CDKs nicht entfernt wird. Nach Aktivierung des G2/M Kontrollpunktes durch Schädigung der DNA sind beide Mechanismen in ähnlicher Weise daran beteiligt, die Progression durch den Zellzyklus zu stoppen.

Der Verlust der Regulation des Zellzyklusses und der Verlust der Funktion der Kontrollpunkte sind Charakteristika von Tumorzellen. Der CDK-Rb-Signalweg ist in über 90% humaner Tumorzellen von Mutationen betroffen. Diese Mutationen, die schließlich zur inaktivierenden Phosphorylierung des RB führen, schließen die Überexpression von D- und E-Zyklinen durch Genamplifikation oder chromosomale Translokationen, inaktivierende Mutationen oder Deletionen von CDK-Inhibitoren des p16-Typs, sowie erhöhten (p27) oder verminderten (CycD) Proteinabbau ein. Die zweite Gruppe von Genen, die durch Mutationen in Tumorzellen getroffen sind, kodiert für Komponenten der Kontrollpunkte. So ist p53, das essentiell für die G1 und G2/M Kontrollpunkte ist, das am häufigsten mutierte Gen in humanen Tumoren (ca. 50%). In Tumorzellen, die p53 ohne Mutation exprimieren, wird es häufig aufgrund einer stark erhöhten Proteindegradation inaktiviert. In ähnlicher Weise sind die Gene anderer für die Funktion der Kontrollpunkte notwendiger Proteine von Mutationen betroffen, zum Beispiel ATM (inaktivierende Mutationen) oder cdc25 Phosphatasen (Überexpression).

Überzeugende experimentelle Daten deuten darauf hin, daß CDK2/Cyc-Komplexe eine entscheidende Position während der Zellzyklusprogression einnehmen: (1) Sowohl dominant-negative Formen der CDK2, wie die transkriptionelle Repression der CDK2 Expression durch anti-sense Oligonukleotide bewirken einen Stopp der Zellzyklusprogression. (2) Die Inaktivierung des CycA Gens in Mäusen ist letal. (3) Die Störung der Funktion des CDK2/CycA Komplexes in Zellen mittels zellpermeabler Peptide führte zur Tumorzell-selektiven Apoptose (Chen Y.N.P. *et al*. (1999). Selective killing of transformed cells by cyclin/cyclin-dependent kinase 2 antagonists. *Proc. Natl. Acad. Sci. USA* 96, 4325-4329).

Veränderungen der Zellzykluskontrolle spielen nicht nur bei Krebserkrankungen ein Rolle. Der Zellzyklus wird durch eine Reihe von Viren, sowohl durch transformierende, wie durch nicht-transformierende, aktiviert um die Vermehrung der Viren in der Wirtszelle zu ermöglichen. Der fälschliche Eintritt in den Zellzyklus von normalerweise post-mitotischen Zellen wird mit verschiedenen neurodegenerativen Erkrankungen in Zusammenhang gebracht.
Die Mechanismen der Zellzyklusregulation, ihrer Veränderungen in Krankheiten und eine Vielzahl von Ansätzen zur Entwicklung von Inhibitoren der Zellzyklusprogression und speziell der CDKs wurden bereits in mehreren Publikationen ausführlich zusammenfassend beschrieben (Sielecki T.M. *et al.* (2000). Cyclin-dependent kinase inhibitors: useful targets in cell cycle regulation. *J*. *Med. Chem.* 43, 1-18; Fry D.W. & Garrett M.D. (2000). Inhibitors of cyclin-dependent kinases as therapeutic agents for the treatment of cancer. *Curr. Opin. Oncol. Endo. Metab. Invest. Drugs* 2, 40-59; Rosiania G.R. & Chang Y.T. (2000). Targeting hyperproliferative disorders with cyclin dependent kinase inhibitors. *Exp. Opin. Ther. Patents* 10, 215-230; Meijer L. *et al.* (1999). Properties and potential applications of chemical inhibitors of cyclin-dependent kinases. *Pharmacol. Ther.* 82, 279-284; Senderowicz A.M. & Sausville E.A. (2000). Preclinical and clinical development of cyclin-dependent kinase modulators. *J. Natl. Cancer Inst*. 92, 376-387).

Erfindungsgemäße Verbindungen der Ansprüche 1 bis 3 können zum anderen auch Rezeptortyrosinkinasen und deren Liganden, die spezifisch die Funktion von Endothelzellen regulieren, inhibieren. Rezeptortyrosinkinasen und deren Liganden, die spezifisch die Funktion von Endothelzellen regulieren, sind in entscheidender Weise an der physiologischen, wie auch der pathogenen Angiogenese beteiligt. Von besonderer Bedeutung ist hier das VEGF/VEGF-Rezeptor System. In pathologischen Situationen die mit einer verstärkten Neovaskularisation einhergehen wurde eine erhöhte Expression von angiogenen Wachstumsfaktoren und ihrer Rezeptoren gefunden. So exprimieren die meisten soliden Tumoren große Mengen an VEGF, und die Expression der VEGF-Rezeptoren ist vorzugsweise in den Endothelzellen, die in der Nähe der Tumoren liegen oder durch diese hindurchführen, deutlich erhöht (Plate et al., Cancer Res. 53, 5822-5827, 1993). Die Inaktivierung des VEGFNEGF-Rezeptorsystems durch VEGF-neutralisierende Antikörper (Kim et al., Nature 362, 841-844, 1993), retrovirale Expression dominant-negativer VEGF-Rezeptorvarianten (Millauer et al., Nature 367, 576-579, 1994), rekominanter VEGF-neutralisierender Rezeptorvarianten (Goldman et al., Proc. Natl. Acad. Sci. USA 95, 8795-8800, 1998), oder niedermolekularer Inhibitoren der VEGF-Rezeptortyrosinkinase (Fong et al., Cancer Res. 59, 99-106, 1999; Wedge et al., Cancer Res. 60, 970-975, 2000; Wood et al., Cancer Res. 60, 2178-2189, 2000) resultierten in einem verringerten Tumorwachstum und einer verringerten Tumorvaskularisierung. Somit ist die Hemmung der Angiogenese ein möglicher Behandlungsmodus für Tumorerkrankungen.

Erfindungsgemäße Verbindungen können demensprechend entweder Zyklin-abhängigen Kinasen, wie CDK1, CDK2, CDK3, CDK4, CDK5, CDK6, CDK7, CDK8 und CDK9, sowie der Glycogen-Synthase-Kinase (GSK-3β) und VEGF-Rezeptortyrosinkinasen oder Zyklin-abhängigen Kinasen oder VEGF-Rezeptortyrosinkinasen inhibieren. Diese Wirkungen tragen dazu bei, dass die erfindungsgemäßen Verbindungen verwendet werden können bei der Behandlung von Krebs, Angiofribroma, Arthritis, Augenerkrankungen, Autoimmunerkrankungen, Chemotherapeutika-induzierter Alopezie und Mukositis, Crohn-Krankheit, Endometriose, fibrotische Erkrankungen, Hämangioma, kardiovaskulären Erkrankungen, infektiösen Erkrankungen, nephrologischen Erkrankungen, chronischen und akuten neurodegenerativen Erkrankungen, sowie von Verletzungen des Nervengewebes, viralen Infektionen, zur Hemmung der Reocclusion von Gefäßen nach Ballonkatheterbehandlung, bei der Gefäßprothetik oder nach dem Einsetzen von mechanischen Vorrichtungen zum Offenhalten von Gefäßen, wie z. B. Stents, als Immunsuppressiva, zur Unterstützung der narbenfreien Wundheilung, bei Altersflecken und bei Kontaktdermatitis, wobei
unter Krebs solide Tumoren, Tumor- oder Metastasenwachstum, Kaposis Sarkom, Morbus Hodgkin und Leukämie,
unter Arthritis, rheumatoide Arthritis, unter Augenericrankungen, diabetische Retinopathie, Neovaskulares Glaukom,
unter Autoimmunerkrankungen Psoriasis, Alopezie und Multiple Sklerose, unter fibrotische Erkrankungen, Leberzirrhose, mesangialzellproliferative Erkrankungen, Arteriosklerose,
unter infektiösen Erkrankungen durch unizelluläre Parasiten hervorgerufene Erkrankungen,
unter kardiovaskulären Erkrankungen Stenosen, wie z. B. Stent-induzierte Restenose, Arteriosklerosen und Restenosen,
unter nephrologischen Erkrankungen Glomerulonephritis, diabetische Nephropatie, maligne Nephrosklerose, thrombische mikroangiopatische Syndrome, Transplantationsabstoßungen und Glomerulopathie,
unter chronisch neurodegenerativen Erkrankungen Huntington's Erkrankung, amyotrophe Lateralsklerose, Parkinsonsche Erkrankung, AIDS Dementia und Alzheimer'sche Erkrankung,
unter akut neurodegenerativen Erkrankungen Ischämien des Gehirns und Neurotraumata,
und unter viralen Infektionen Cytomegalus-Infektionen, Herpes, Hepatitis B oder C, und HIV Erkrankungen zu verstehen sind.

Zur Verwendung der erfindungsgemäßen Verbindungen als Arzneimittel werden diese in die Form eines pharmazeutischen Präparats gebracht, das neben dem Wirkstoff für die enterale oder parenterale Applikation geeignete pharmazeutische, organische oder anorganische inerte Trägermaterialien, wie zum Beispiel, Wasser, Gelatine, Gummi arabicum, Milchzucker, Stärke, Magnesiumstearat, Talk, pflanzliche Öle, Polyalkylenglykole usw. enthält. Die pharmazeutischen Präparate können in fester Form, zum Beispiel als Tabletten, Dragees, Suppositorien, Kapseln oder in flüssiger Form, zum Beispiel als Lösungen, Suspensionen oder Emulsionen vorliegen. Gegebenenfalls enthalten sie darüber hinaus Hilfsstoffe, wie Konservierungs-, Stabilisierungs-, Netzmittel oder Emulgatoren; Salze zur Veränderung des osmotischen Drucks oder Puffer.
Diese pharmazeutischen Präparate sind ebenfalls Gegenstand der vorliegenden Erfindung.

Für die parenterale Anwendung sind insbesondere Injektionslösungen oder Suspensionen, insbesondere wäßrige Lösungen der aktiven Verbindungen in polyhydroxyethoxyliertem Rizinusöl, geeignet.

Als Trägersysteme können auch grenzflächenaktive Hilfsstoffe wie Salze der Gallensäuren oder tierische oder pflanzliche Phospholipide, aber auch Mischungen davon sowie Liposomen oder deren Bestandteile verwendet werden.

Für die orale Anwendung sind insbesondere Tabletten, Dragees oder Kapseln mit Talkum und/oder Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt ist.

Die enteralen, parenteralen und oralen Applikationen sind ebenfalls Gegenstand der vorliegenden Erfindung.

Die Dosierung der Wirkstoffe kann je nach Verabfolgungsweg, Alter und Gewicht des Patienten, Art und Schwere der zu behandelnden Erkrankung und ähnlichen Faktoren variieren. Die tägliche Dosis beträgt 0,5-1000 mg, vorzugsweise 50-200 mg, wobei die Dosis als einmal zu verabreichende Einzeldosis oder unterteilt in 2 oder mehreren Tagesdosen gegeben werden kann.

Ebenfalls Gegenstand der vorliegenden Erfindung ist die Verwendung der Verbindungen der Ansprüche 1 bis 3, zur Herstellung eines Arzneimittels zur Behandlung von Krebs, Augenerkrankungen, Autoimmunerkrankungen, Arthritis, Endometriose, fibrotische Erkrankungen, kardiovaskulären Erkrankungen, Chemotherapeutika-induzierter Alopezie und Mukositis, infektiösen Erkrankungen, nephrologischen Erkrankungen, chronischen und akuten neurodegenerativen Erkrankungen, sowie von Verletzungen des Nervengewebes, viralen Infektionen, Hämangioma, Angiofribroma, Crohn-Krankheit, zur Hemmung der Reocclusion von Gefäßen nach Ballonkatheterbehandlung, z.B. bei der Gefäßprothetik oder nach dem Einsetzen von mechanischen Vorrichtungen zum Offenhalten von Gefäßen, wie z. B. Stents, als Immunsuppressiva, zur Unterstützung der narbenfreien Wundheilung, bei Altersflecken und bei Kontaktdermatitis,
wobei unter Krebs solide Tumoren, Tumor- oder Metastasenwachstum, Kaposis Sarkom, Morbus Hodgkin und Leukämie,
unter Autoimmunerkrankungen Psoriasis, Alopezie und Multiple Sklerose, unter kardiovaskulären Erkrankungen Stenosen, wie z. B. Stent-induzierte Restenose, Arteriosklerosen und Restenosen, unter infektiösen Erkrankungen durch unizelluläre Parasiten hervorgerufene Erkrankungen,
unter nephrologischen Erkrankungen Glomerulonephritis, diabetische Nephropatie, maligne Nephrosklerose, thrombische mikroangiopatische Syndrome, Transplantationsabstoßungen und Glomerulopathie,
unter chronisch neurodegenerativen Erkrankungen Huntington's Erkrankung, amyotrophe Lateralsklerose, Parkinsonsche Erkrankung, AIDS Dementia und Alzheimer'sche Erkrankung,
unter akut neurodegenerativen Erkrankungen Ischämien des Gehirns und Neurotraumata,
unter Arthritis, rheumatoide Arthritis,
unter Augenerkrankungen, diabetische Retinopathie, Neovaskulares Glaukom, unter fibrotische Erkrankungen, Leberzirrhose, mesangialzellproliferative Erkrankungen, Arteriosklerose,
und unter viralen Infektionen Cytomegalus-Infektionen, Herpes, Hepatitis B oder C, und HIV Erkrankungen zu verstehen sind.

Ebenfalls Gegenstand der vorliegenden Erfindung sind Arzneimittel zur Behandlung der oben aufgeführten Erkrankungen, die mindestens eine Verbindung gemäß der Ansprüche 1 bis 3 enthalten, sowie Arzneimittel mit geeigneten Formulierungs- und Trägerstoffen.

Die erfindungsgemäßen Verbindungen der Ansprüche 1 bis 3 sind entweder gute gute Inhibitoren von Zyklin-abhängigen Kinasen, wie CDK1, CDK2, CDK3, CDK4, CDK5, CDK6, CDK7, CDK8 und CDK9, sowie der Glycogen-Synthase-Kinase (GSK-3β), und der VEGF-Rezeptortyrosinkinasen oder von Inhibitoren der Zyklin-abhängigen Kinasen oder gute Inhibitoren von VEGF-Rezeptortyrosinkinasen.

Soweit die Herstellung der Ausgangsverbindungen nicht beschrieben wird, sind diese bekannt oder analog zu bekannten Verbindungen oder hier beschriebenen Verfahren herstellbar. Es ist ebenfalls möglich, alle hier beschriebenen Umsetzungen in Parallel-Reaktoren oder mittels kombinatorischer Arbeitstechniken durchzuführen. Die Isomerengemische können nach üblichen Methoden wie beispielsweise Kristallisation, Chromatographie oder Salzbildung in die Enantiomeren bzw. E/Z-Isomeren aufgetrennt werden.
Die Herstellung der Salze erfolgt in üblicher Weise, indem man eine Lösung der Verbindung der der Ansprüche 1 bis 3 mit der äquivalenten Menge oder einem Überschuß einer Base oder Säure, die gegebenenfalls in Lösung ist, versetzt und den Niederschlag abtrennt oder in üblicher Weise die Lösung aufarbeitet.

### Herstellung der erfindungsgemäßen Verbindungen

Die nachfolgenden Beispiele erläutern die Herstellung der erfindungsgemäßen Verbindungen, ohne den Umfang der beanspruchten Verbindungen auf diese Beispiele zu beschränken.

Die erfindungsgemäßen Verbindungen der Ansprüche 1 bis 3 lassen sich neben dem bereits oben beschriebenen erfindungsgemäßen Eintopfverfahren auch gemäß den folgenden allgemeinen Verfahrensvarianten herstellen:

### Herstellung der 5-Brom-Derivate

### Verfahrensvariante 1a

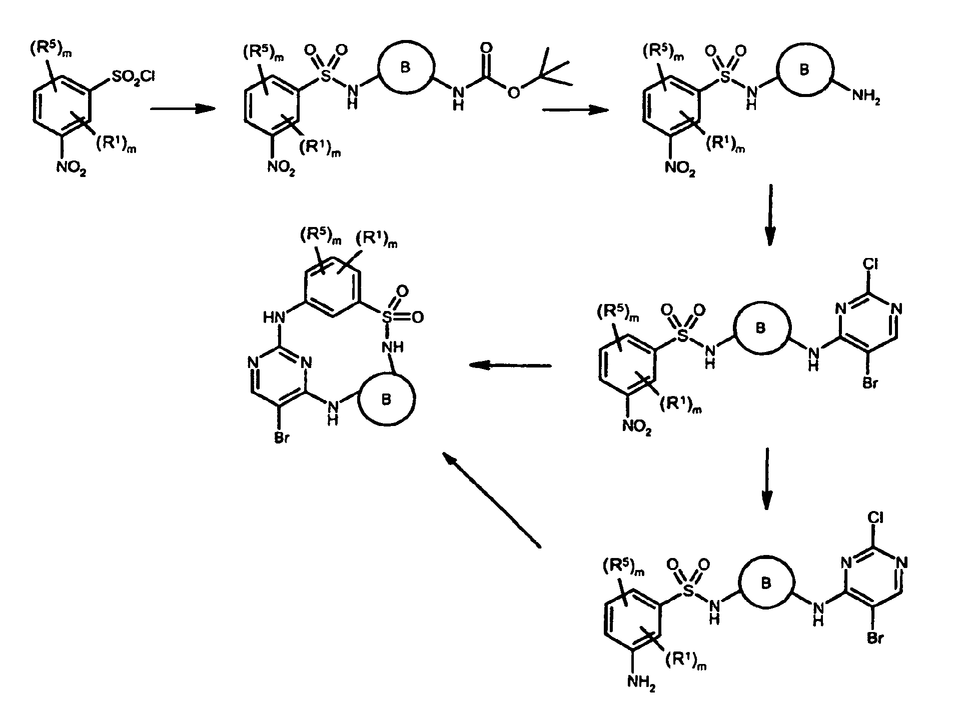

In den Formeln haben R¹, R⁵, B und m die in den Verbindungen der Ansprüche 1 bis 3 verwirklichten Bedeutungen.

### Beispiel 1.0

### Herstellung von 1⁵-Brom-4-thia-2,5,11-triaza-1(2,4)-pyrimidina-3(1,3)-benzenacycloundecaphane 4,4-dioxide

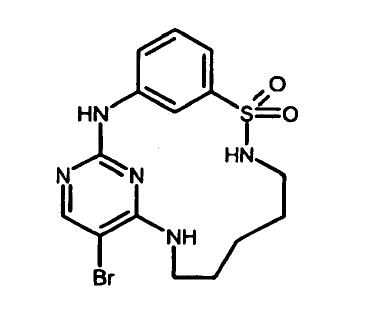

Eine Lösung von 100 mg (0,22 mmol) 3-Amino-*N*-[5-(5-brom-2-chlor-pyrimidin-4-ylamino)-pentyl]-benzolsutfonamid in Acetonitril / Wasser / 2-Butanol (8,5 ml / 1,5 ml / 0,5 ml) wird mittels Spritzenpumpe innerhalb von 2 Stunden zu einer refluxierenden Lösung von Acetonitril/ / Wasser / 4 molare Lösung von Chlorwasserstoff in Dioxan (45 ml / 5 ml / 0,6 ml) gegeben. Nach weiteren 60 min wird das Acetonitril am Rotationsverdampfer abgezogen und der Rückstand mit Wasser (30 ml) versetzt. Man extrahiert mit Essigester (3x). Die vereinten organischen Phasen werden mit 1 M NaHCO₃-Lösung, 10% Zitronensäure, 1 M NaHCO₃ Lösung gewaschen, getrocknet (Na₂SO₄), filtriert und eingeengt. Man erhält 83 mg (0,20 mmol, entsprechend 90 % der Theorie) des Produktes.

¹H-NMR (DMSO): 9.65 (s, 1H), 8.78 (s, 1H), 8.03 (s, 1H), 7.43 (m, 2H), 7.30 (m, 2H), 7.22 (t, 1H), 3.42 (m, 2H), 2.75 (m, 2H), 1.65 (m, 2H), 1.42 (m, 4H).
¹³C-NMR (DMSO): 158.5s, 158.3s, 156.1d, 140.9s, 139.7s, 130.0d, 122.7d, 118.8d, 117.9d, 93.1 s, 66.7t, 41.0t, 27.0t, 26.1 t, 22.8t.
MS: 412 (ES).

### Herstellung der Zwischenprodukte nach Verfahrensvariante 1a

### 1a) Herstellung von [5-(3-Nitro-benzolsulfonylamino)-pentyl]-carbamidsäure-tert-butylester

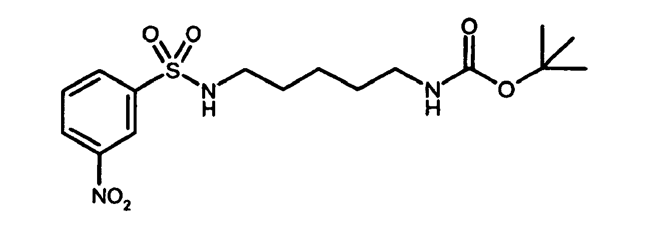

Zu einer Lösung von 3,21 g (14,5 mmol) 3-Nitrobenzolsulfonylchlorid und 3,0 ml (14,4 mmol) N-Boc-1,5-diaminopentan in 50 ml Aceton und 15 ml Wasser werden 4,2 ml (30,1 mmol) Triethylamin gegeben. Das Reaktionsgemisch wird eine Stunde bei Raumtemperatur gerührt. Anschließend wird das Aceton am Rotationsverdampfer abgezogen. Nach der Zugabe von Wasser (20 ml) wird mit Essigester extrahiert (2x). Die vereinten organischen Phasen werden getrocknet (Na₂SO₄), filtriert und eingeengt. Man erhält 5,00 g (12,9 mmol, entsprechend 90% der Theorie) des Produktes als hell gelbes Öl.
¹H-NMR (DMSO): 8.49 (m, 2H), 8.19 (dd, 1H), 7.88 (m, 2H), 6.72 (t, 1H), 2.82 (m, 4H), 1.32 (m, 15H).

### 1b) Herstellung von N-(5-Amino-pentyl)-3-nitro-benzolsulfonamid

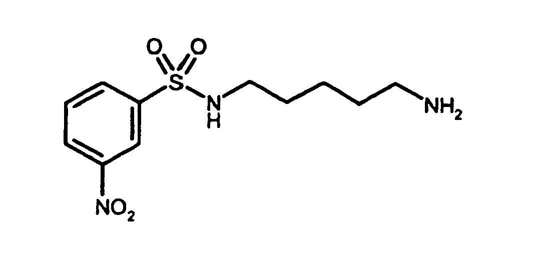

5,00 g (12,9 mmol) [5-(3-Nitro-benzolsulfonylamino)-pentyl]-carbamidsäure-*tert-*butylester werden mit 15 ml Trifluoressigsäure versetzt und 90 min bei Raumtemperatur gerührt. Das Reaktionsgemisch wird eingeengt und der Rückstand mit gesättigter NaHCO₃ Lösung basisch gestellt. Anschließend wird mit Essigester extrahiert (2x). Die vereinten organischen Phasen werden mit gesättigter NaCl Lösung gewaschen, getrocknet (Na₂SO₄), filtriert und eingeengt. Man erhält 3,4 g (11,8 mmol, entsprechend 91% der Theorie) des Produktes.
¹H-NMR (DMSO): 8.49 (m, 2H), 8.19 (dd, 1H), 7.90 (t, 1H), 7.60 (br, 3H), 2.73 (m, 4H), 1.35 (m, 6H).

### 1c) Herstellung von N-[5-(5-Brom-2-chlor-pyrimidin-4-ylamino)-pentyl]-3-nitro-benzolsulfonamid

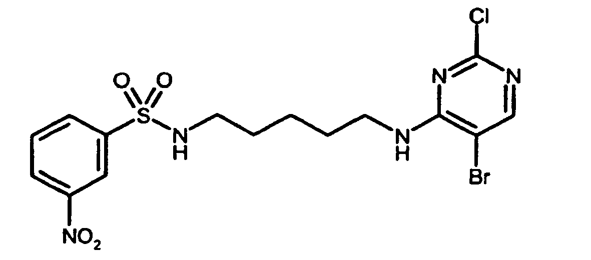

Eine Lösung von 1,2 g (5,3 mmol) 5-Brom-2,4-dichlor-pyrimidin in 30 ml Acetonitril wird zu einer Lösung von 1,5 g (5,2 mmol) *N*-(5-Amino-pentyl)-3-nitro-benzolsulfonamid in 50 ml Acetonitril gegeben. Das Reaktionsgemisch wird mit 1,0 ml (7,2 mmol) Triethylamin versetzt und 17 Stunden bei Raumtemperatur gerührt. Nach der Zugabe von Wasser (50 ml) wird mit Essigester extrahiert (2x). Die vereinten organischen Phasen werden getrocknet (Na₂SO₄), filtriert und eingeengt. Der verbleibende Rückstand wird chromatographisch gereinigt (Hexan/Essigester 2:1, Flashmaster II). Man erhält 1,5 g (3,1 mmol, entsprechend 60% der Theorie) des Produktes.
¹H-NMR (DMSO): 8.49 (m, 2H), 8.19 (m, 2H), 7.88 (m, 2H), 7.68 (t, 1H), 3.30 (m, 2H), 2.79 (m, 2H), 1.45 (m, 4H), 1.21 (m, 2H).
MS: 478 (ES).

### 1d) Herstellung von 3-Amino-N-[5-(5-brom-2-chlor-pyrimidin-4-ylamino)-pentyl]-benzolsulfonamid

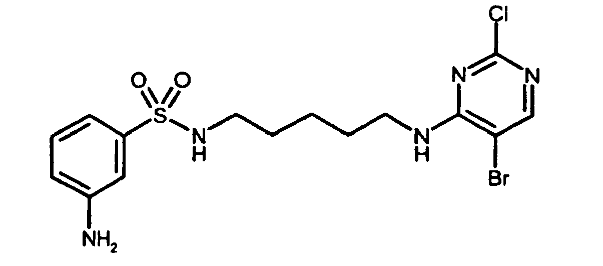

Eine Lösung von 300 mg (0,63 mmol) *N*-[5-(5-Brom-2-chlor-pyrimidin-4-ylamino)-pentyl]-3-nitro-benzolsulfonamid in 6 ml Ethanol wird mit 600 mg Zinn(II)chlorid versetzt und 30 min bei 70°C gerührt. Nach dem Abkühlen wird das Reaktionsgemisch vorsichtig auf Eiswasser gegeben und mit gesättigter NaHCO₃ Lösung basisch gestellt. Man extrahiert mit Essigester (3x). Die vereinten organischen Phasen werden getrocknet (Na₂SO₄), filtriert und eingeengt. Der verbleibende Rückstand wird chromatographisch gereinigt (Essigester/Hexan 4:1). Man erhält 112 mg (0,25 mmol, entsprechend 40% der Theorie) des Produktes.
¹H-NMR (DMSO): 8.20 (s, 1H), 7.70 (br, 1H), 7.31 (br, 1H), 7.15 (t, 1H), 6.94 (m, 1H), 6.85 (m, 1H), 6.71 (m, 1H), 5.52 (s, 2H), 3.30 (m, 2H), 2.71 (m, 2H), 1.45 (m, 4H), 1.21 (m, 2H).
MS: 448 (ES).

### Beispiel 1.1

### Herstellung von 1⁵-Bromo-4-thia-2,5,9-triaza-1(2,4)-pyrimidina-3(1,3)-benzenacyclononaphane-4,4-dioxide

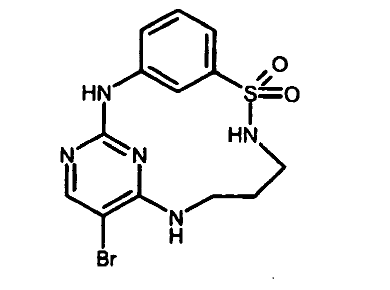

### Methode A

Eine Lösung von 200 mg (0,48 mmol) 3-Amino-*N*-[3-(5-brom-2-chlor-pyrimidin-4-ylamino)-propyl]-benzolsulfonamid in Acetonitril / Wasser / 2-Butanol (9,0 ml / 1,0 ml / 0,3 ml) wird mittels Spritzenpumpe innerhalb von 2.5 Stunden zu einer refluxierenden Lösung von Acetonitril / Wasser / 4 molare Lösung von Chlorwasserstoff in Dioxan (45 ml / 5 ml / 0,6 ml) gegeben. Nach weiteren 3 Stunden unter Rückfluss wird das Ölbad abgeschaltet und die Reaktionslösung über Nacht bei Raumtemperatur gerührt. Der gebildete Niederschlag wird abfiltriert, mit Wasser gewaschen und anschließend im Vakuum getrocknet. Man erhält 112 mg (0,31 mmol) des Produktes. Das Filtrat wird am Rotationsverdampfer eingeengt. Der gebildete Niederschlag wird mit Wasser gewaschen und abfiltriert. Nach dem Trocknen erhält man weitere 45 mg (0,12 mmol) des Produktes. Die Gesamtausbeute an Produkt beträgt somit 157 mg (0,41 mmol, entsprechend 85% der Theorie).

### Methode B

Eine Lösung von 450 mg (1,00 mmol) *N*-[3-(5-Brom-2-chlor-pyrimidin-4-ylamino)-propyl]-3-nitro-benzolsulfonamid in 9,5 ml Ethanol wird mit 960 mg Zinn(II)chlorid versetzt und 30 min bei 70°C gerührt. Nach dem Abkühlen wird das Reaktionsgemisch vorsichtig auf Eiswasser gegeben und mit 1 N NaOH Lösung basisch gestellt. Man extrahiert mit Essigester (3x). Die vereinten organischen Phasen werden getrocknet (Na₂SO₄), filtriert und eingeengt. Der verbleibende Rückstand wird chromatographisch gereinigt (Essigester / Hexan 4:1). Man erhält 72 mg des Rohproduktes. Man versetzt mit 1 N HCl und extrahiert mit Essigester. Aus der wässrigen Phase fällt ein farbloser Feststoff aus. Der Feststoff wird abfiltriert und getrocknet. Man erhält 20 mg (0,05 mmol, entsprechend 5% der Theorie) des Produktes.
¹H-NMR (DMSO): 10.45 (s, 1H), 9.07 (s, 1H), 8.35 (br, 1H), 8.18 (s, 1H), 7.78 (t, 1H), 7.45 (m, 2H), 7.32 (m, 1H), 3.44 (m, 2H), 3.28 (m, 2H), 1.82 (m, 2H).
MS: 384 (ES).

### Herstellung des Zwischenproduktes nach Verfahrensvariante 1a

### 1e) Herstellung von 3-Amino-N-[3-(5-brom-2-chlor-pyrimidin-4-ylamino)-propyl]-benzolsulfonamid

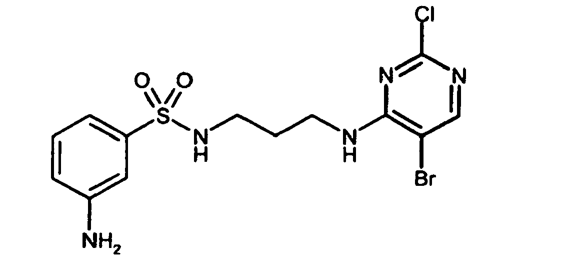

Eine Lösung von 1,35 g (2,99 mmol) *N*-[3-(5-Brom-2-chlor-pyrimidin-4-ylamino)-propyl]-3-nitro-benzolsulfonamid in 100 ml Tetrahydrofuran wird unter Argon bei Raumtemperatur mit 15 ml einer 15%igen Lösung von Ti(III)Cl in etwa 10%iger Salzsäure versetzt. Nach 17 Stunden wird die Reaktionslösung erneut mit 1 ml der Ti(III)Cl-Lösung versetzt und weitere 3 h gerührt. Der Ansatz wird mit 1 N NaOH Lösung basisch gestellt und anschließend filtriert. Der Filterkuchen wird 2x mit jeweils 100 ml Essigester / MeOH (30 ml / 20 ml) nachgewaschen. Das Filtrat wird am Rotationsverdampfer eingeengt und danach mit Essigester extrahiert (2x). Die vereinten organischen Phasen werden mit NaCl-Lösung gewaschen, getrocknet (Na₂SO₄), filtriert und eingeengt. Der verbleibende Rückstand wird chromatographisch gereinigt (Dichlormethan / MeOH 95:5, Flashmaster II). Man erhält 624 mg (1,48 mmol, entsprechend 49% der Theorie) des Produktes.
¹H-NMR (DMSO): 8.21 (s, 1H), 7.63 (t, 1H), 7.38 (t, 1H), 7.13 (t, 1H), 6.97 (m, 1H), 6.83 (m, 1H), 6.71 (m, 1H), 5.53 (s, 2H), 3.30 (m, 2H), 2.75 (m, 2H), 1.65 (m, 2H).

### Beispiel 1.2

### Herstellung von rac-1⁵-Bromo-4-thia-2,5,9-triaza-1(2,4)-pyrimidina-3(1,3)-benzenacyclononaphan-7-ol-4,4-dioxide

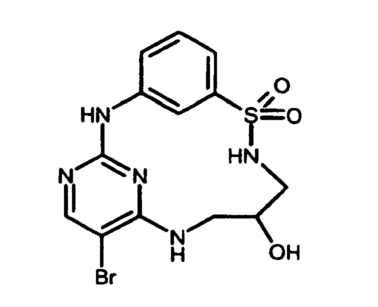

Eine Lösung von 150 mg (0,34 mmol) 3-Amino-*N*-[3-(5-brom-2-chlor-pyrimidin-4-ylamino)-2-hydroxy-propyl]-benzolsulfonamid in Acetonitril / Wasser (9,0 ml / 1,0 ml) wird mittels Spritzenpumpe innerhalb von 2,5 Stunden zu einer refluxierenden Lösung von Acetonitril / Wasser / 4 molare Lösung von Chlorwasserstoff in Dioxan (45 ml / 5 ml / 0,6 ml) gegeben. Nach weiteren 4 Stunden unter Rückfluss wird das Ölbad abgeschaltet und die Reaktionslösung über Nacht bei Raumtemperatur gerührt. Der gebildete Niederschlag wird abfiltriert, mit MeCN gewaschen und anschließend im Vakuum getrocknet. Man erhält 125 mg (0,31 mmol, entsprechend 91 % der Theorie) des Produktes.
¹H-NMR (DMSO): 10.65 (br, 1H), 9.03 (s, 1H), 8.41 (br, 1H), 8.22 (s, 1H), 7.93 (m, 1H), 7.46 (m, 2H), 7.34 (m, 1H), 4.14 (m, 1H), 3.94 (dd, 1H), 3.49 (m, 1H), 2.88 (m, 2H).
MS: 402 (ES).

### Herstellung der Zwischenprodukte nach Verfahrensvariante 1a

### 1f) 3-Amino-N-[3-(5-brom-2-chlor-pyrimidin-4-ylamino)-2-hydroxy-propyl]-benzolsulfonamid

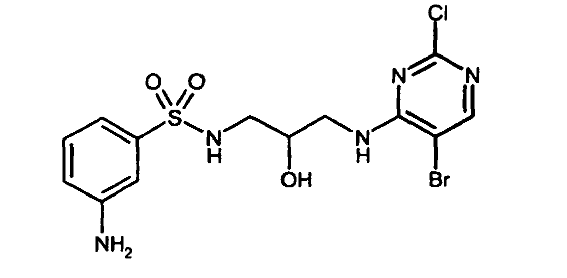

Eine Lösung von 258 mg (0,553 mmol) *N*-[3-(5-Brom-2-chlor-pyrimidin-4-ylamino)-2-hydroxy-propyl]-3-nitro-benzolsulfonamid in 20 ml Tetrahydrofuran wird unter Argon bei Raumtemperatur mit 2,6 ml einer 15%igen Lösung von Ti(III)Cl in etwa 10%iger Salzsäure versetzt. Nach 2 Stunden wird die Reaktionslösung erneut mit 0,2 ml der Ti(III)Cl-Lösung versetzt und weitere 60 min gerührt. Der Ansatz wird mit 1 M NaOH Lösung basisch gestellt und anschließend filtriert. Der Filterkuchen wird 2x mit jeweils 50 ml Essigester / MeOH (30 ml / 20 ml) nachgewaschen. Das Filtrat wird am Rotationsverdampfer eingeengt und danach mit Essigester extrahiert (2x). Die vereinten organischen Phasen werden mit NaCl-Lösung gewaschen, getrocknet (Na₂SO₄), filtriert und eingeengt. Der verbleibende Rückstand wird chromatographisch gereinigt (Dichlormethan / MeOH 95:5, Flashmaster II). Man erhält 155 mg (0,36 mmol, entsprechend 64% der Theorie) des Produktes.
¹H-NMR (DMSO): 8.25 (s, 1H), 7.43 (t, 1H), 7.36 (t, 1H), 7.13 (t, 1H), 6.96 (m, 1H), 6.86 (m, 1H). 6.71 (m, 1H), 5.53 (s, 2H), 5.14 (d, 1H), 3.70 (m, 1H), 3.30 (m, 2H), 2.72 (m, 2H).

### Herstellung der Zwischenprodukte nach Verfahrensvariante 1b

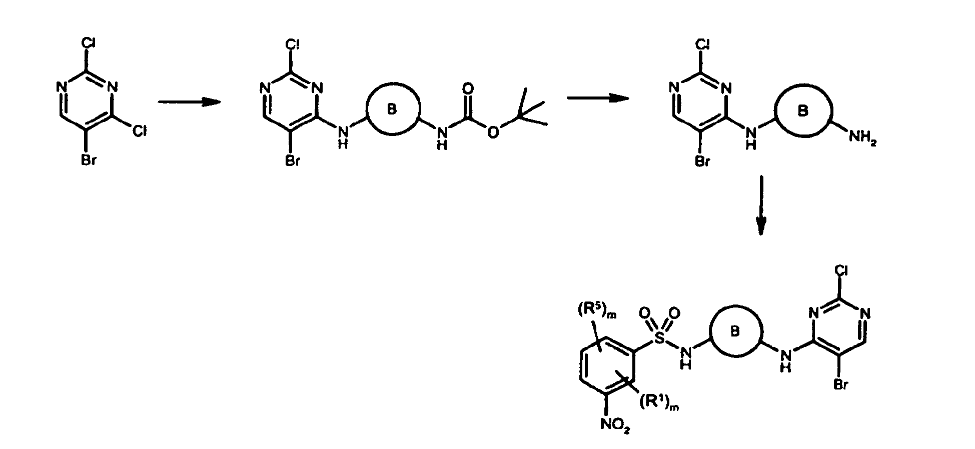

In den Formeln haben R¹, R⁵, B und m die in den Verbindungen der Ansprüche 1 bis 3 verwirklichten Bedeutungen .

### 1g) Herstellung von [3-(5-Brom-2-chlor-pyrimidin-4-ylamino)-propyl]-carbaminsäure-tert-butyl-ester

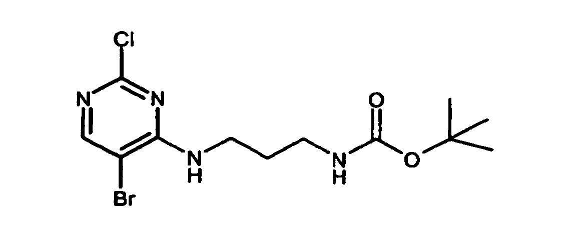

Eine Lösung von 6,1 g (26,6 mmol) 5-Brom-2,4-dichlor-pyrimidin in 100 ml Acetonitril wird sukzessive mit 5,0 g (28.7 mmol) *N*-Boc-1,3-diaminopropan und 4,5 ml (32,4 mmol) Triethylamin versetzt und 3.5 Stunden bei Raumtemperatur gerührt. Der Ansatz wird mit 200 ml Essigester verdünnt. Man wäscht mit gesättigter NaCl Lösung, Zitronensäure (10%), gesättigter NaHCO₃ Lösung sowie gesättigter NaCl Lösung. Die vereinten organischen Phasen werden getrocknet (Na₂SO₄), filtriert und eingeengt. Man erhält 9,7 g (26,6 mmol, entsprechend 100 % der Theorie) des Produktes.
¹H-NMR (DMSO): 8.22 (s, 1H), 7.63 (t, 1H), 6.79 (t, 1H), 3.30 (m, 2H), 2.94 (m, 2H), 1.63 (m, 2H), 1.35 (s, 9H).

### 1h) Herstellung von N-(5-Brom-2-chlor-pyrimidin-4-yl)-propan-1,3-diamin Hydrochlorid

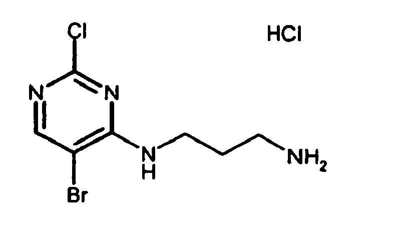

Eine Lösung von 5,0 g (13,7 mmol) [3-(5-Brom-2-chlor-pyrimidin-4-ylamino)-propyl]-carbaminsäure-*tert*-butyl-ester in 150 ml Acetonitril wird mit 25 ml einer 4 molaren Lösung von Chlorwasserstoff in Dioxan versetzt und bei Raumtemperatur gerührt. Nach 4 h wird das Lösungsmittel am Rotationsverdampfer abgezogen und der Rückstand im Trockenschrank getrocknet. Man erhält 4.1 g (13,7 mmol, entsprechend 100 % der Theorie) des Produktes.
¹H-NMR (DMSO): 8.26 (s, 1H), 7.95 (m, 5H), 3.42 (m, 2H), 2.79 (m, 2H), 1.96 (m, 2H).
MS: 265 (ES).

### 1i) Herstellung von N-[3-(5-Brom-2-chlor-pyrimidin-4-ylamino)-propyl]-3-nitro-benzolsulfonamid

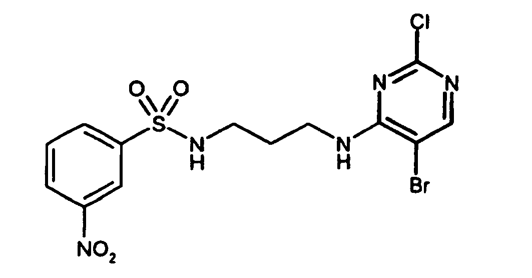

Eine Lösung von 530 mg (1,76 mmol) *N*-(5-Brom-2-chlor-pyrimidin-4-yl)-propan-1,3-diamin Hydrochlorid und 352 mg (1,60 mmol) 3-Nitrobenzolsulfonylchlorid in 20 ml Aceton / 6 ml Wasser wird bei Raumtemperatur mit 1 ml Triethylamin versetzt. Nach 2.5 h wird das organische Lösungsmittel am Rotationsverdampfer abgezogen. Nach der Zugabe von Wasser (20 ml) wird mit Essigester extrahiert. Die vereinten organischen Phasen werden mit Zitronensäure (10%), gesättigter NaHCO₃ Lösung sowie gesättigter NaCl Lösung gewaschen, getrocknet (Na₂SO₄), filtriert und eingeengt. Man erhält 633 mg (1,41 mmol, entsprechend 87 % der Theorie) des Produktes.
¹H-NMR (DMSO): 8.48 (m, 2H), 8.19 (m, 2H), 8.00 (t, 1H), 7.88 (t, 1H), 7.63 (t, 1H), 3.30 (m, 2H), 2.88 (t, 2H), 1.67 (m, 2H).

### Beispiel 1.3

### Herstellung von rac-1⁵- Bromo-4-thia-2,5,9-triaza-1(2,4)-pyrimidina-3(1,3)-benzenacyclonaphane-8-methanol-4,4-dioxide hydrochloride

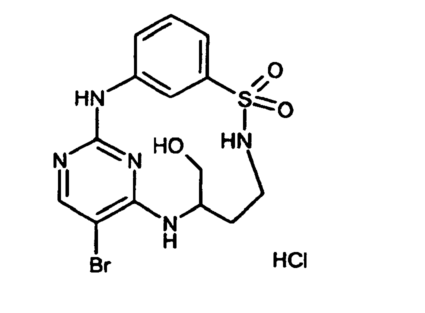

Eine Lösung von 145 mg (0,33 mmol) 3-Amino-*N*-[3-(5-brom-2-chlor-pyrimidin-4-ylamino)-4-hydroxy-butyl]-benzolsulfonamid in Acetonitril / Methanol / Wasser (9,0 ml / 2,0 ml / 1,0 ml) wird mittels Spritzenpumpe innerhalb von 3 h zu einer refluxierenden Lösung von Acetonitril / Wasser / 4 molare Lösung von Chlorwasserstoff in Dioxan (45 ml / 5 ml / 0,8 ml) gegeben. Nach weiteren 14 h unter Rückfluss werden am Rotationsverdampfer ca. 20 ml Acetonitril abgezogen. Nach dem Erkalten wird der gebildete Niederschlag abgesaugt, mit Wasser und Diisopropylether nachgewaschen und getrocknet. Man erhält 97 mg (0,22 mmol, entsprechend 67 % der Theorie) des Produktes in Form des Hydrochlorides.
¹H-NMR (DMSO): 10.22 (s, 1H), 8.99 (m, 1H), 8.19 (s, 1H), 7.69 (t, 1H), 7.40 (m, 3H), 6.95 (br, 1H), 4,04 (m, 1H), 3,70 (m, 2H), 3,40 (m, 2H), 2.19 (m, 1H), 1.61 (m, 1H).
MS: 414 (ES).

Das Racemat wird mittels chiraler HPLC präperativ in die Enantiomere getrennt:
- Säule:: Chiralpak AD (20 µm); 250 x 60 mm
- Eluenten:: Hexan / Ethanol 80 / 20 + 0.1 % DEA
- Fluß:: 100 ml / min
- Detektor:: UV 280 nm
- Temperatur:: RT
- Retention:: Enantiomer (+): 38,5 min, 1.3 (+)-Enantiomer
Enantiomer (-): 59,1 min, 1.3 (-)-Enantiomer

### Beispiel 1.4

### Herstellung von 1⁵-Bromo-4-thia-2,5,9-triaza-1(2,4)-pyrimidina-3(1,3)-benzenacyclononaphan-3⁵-amine- 4,4-dioxide

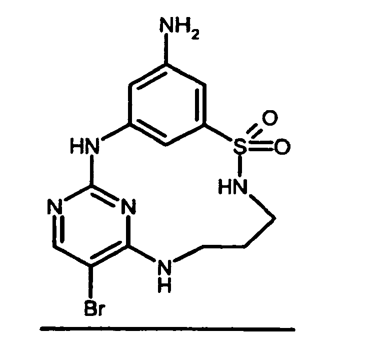

Eine Lösung von 46 mg (0.11 mmol) 1⁵-Bromo-3⁵-nitro-4-thia-2,5,9-triaza-1(2,4)-pyrimidina-3(1,3)-benzenacyclononaphane-4,4-dioxide hydochlorid in 1 ml THF wird bei Raumtemperatur mit 0.4 ml einer 15%igen Lösung von Ti(III)Cl in etwa 10%iger Salzsäure versetzt. Nach 67 h wird erneut mit 0.2 ml einer 15%igen Lösung von Ti(III)Cl in etwa 10%iger Salzsäure versetzt und weitere 21 h gerührt. Der Ansatz wird mit 2N NaOH Lösung basisch gestellt und gegen Essigester extrahiert. Die vereinten organischen Phasen werden mit NaCl Lösung gewaschen und anschließend mittels Whatman Filter filtriert und eingeengt. Der gebildete Rückstand wird aus Methanol / Diisopropylether umkristallisiert. Man erhält 24 mg (0.06 mmol, entsprechend 55 % der Theorie) des Produktes.
¹H-NMR (DMSO): 9.45 (br, 1H), 8.51 (br, 1H), 8.02 (br, 1H), 7.53 (br, 1H), 7.31 (br, 1H), 6.60 (br, 1H), 6.48 (br, 1H), 5.42 (s, 2H), 3.30 (m, 4H), 1.78 (m, 2H).
MS: 399 (ES).

### Beispiel 1.5

### Herstellung von 1⁵-Bromo-3⁵-nitro-4-thia-2,5,9-triaza-1(2,4)-pyrimidina-3(1,3)-benzenacyclononaphane- 4,4-dioxide hydrochloride

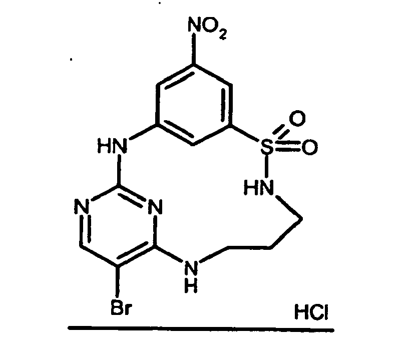

Eine Lösung von 420 mg (0,90 mmol) 3-Amino-*N*-[3-(5-brom-2-chlor-pyrimidin-4-ylamino)-propyl]-5-nitro-benzolsulfonamid in Acetonitril / DMF (7.0 ml / 3.0 ml) wird mittels Spritzenpumpe innerhalb von 2 h zu einer refluxierenden Lösung von Acetonitril / 4 molare Lösung von Chlorwasserstoff in Dioxan (150.0 ml / 2.5 ml) gegeben. Nach dem Abkühlen wird der gebildete Niederschlag abgesaugt. Das Filtrat wird eingeengt und Rückstand mit Methanol digeriert. Man erhält 151 mg (0,36 mmol, entsprechend 40 % der Theorie) des Produktes in Form des Hydrochlorides.
¹H-NMR (DMSO): 10.68 (s, 1H), 9.51 (s, 1H), 8.15 (m, 4H), 8.02 (m, 1H), 3.41 (m, 4H), 1.83 (m, 2H).
MS: 429 (ES).

### Herstellung der Zwischenprodukte nach Verfahrensvariante 1a

### 1j) Herstellung von 3-Amino-N-[3-(5-brom-2-chlor-pyrimidin-4-ylamino)-propyl]-5-nitro-benzolsulfonamid

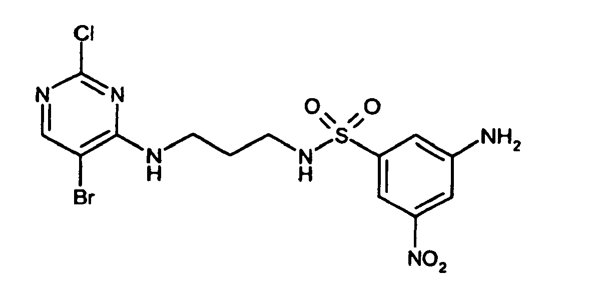

Eine Lösung von 602 mg (1.28 mmol) *N*-[3-(5-Brom-2-chlor-pyrimidin-4-ylamino)-propyl]-3,5-dinitro-benzolsulfonamid in 10 ml THF wird bei Raumtemperatur mit 4.2 ml einer 15%igen Lösung von Ti(III)Cl in etwa 10%iger Salzsäure versetzt. Nach 2 h wird erneut mit 3.0 ml einer 15%igen Lösung von Ti(III)Cl in etwa 10%iger Salzsäure versetzt und weitere 16 h gerührt. Der Ansatz wird mit 2N NaOH Lösung basisch gestellt und filtriert. Der Filterkuchen wird mit THF und Wassser nachgewaschen. Das THF des Filtrats wird am Rotationsverdampfer abgezogen und der Rückstand gegen Essigester extrahiert. Die vereinten organischen Phasen werden mittels Whatman Filter filtriert und eingeengt. Man erhält 440 mg (0.95 mmol, entsprechend 74 % der Theorie) des Produktes.
MS: 465 (ES).

### Herstellung von N-Alkyl-Derivate

### Verfahrensvariante 2

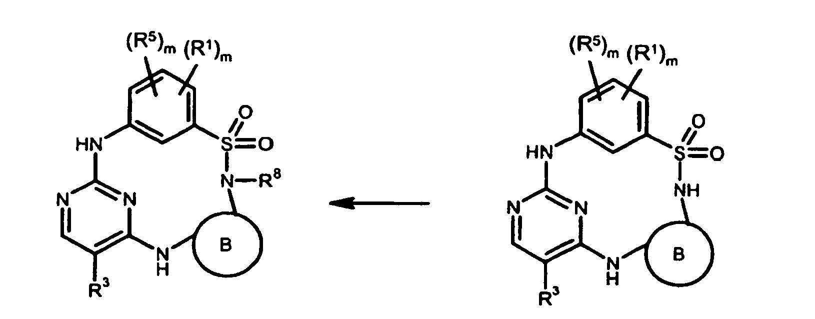

In den Formeln haben R¹, R³, R⁵, R⁸, B und m die in den Verbindungen der Ansprüche 1 bis 3 verwirklichten Bedeutungen .

### Beispiel 2.0

### Herstellung von 1⁵-Bromo-5-methyl-4-thia-2,5,9-triaza-1(2,4)-pyrimidina-3(1,3)-benzenacyclononaphane- 4,4-dioxide

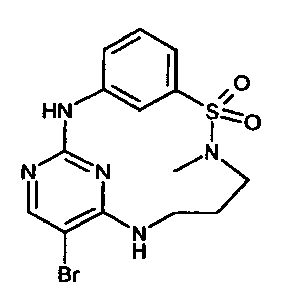

Eine Lösung von 35 mg (0.09 mmol) 1⁵-Bromo-4-thia-2,5,9-triaza-1(2,4)-pyrimidina-3(1,3)-benzenacyclononaphane-4,4-dioxide in 4 ml DMSO wird bei Raumtemperatur mit 6 mg (0,15 mmol) einer 60 %igen Dispersion von Natriumhydrid in Mineralöl versetzt und 10 min gerührt. Anschließend erfolgt die Zugabe von 7 µl Methyliodid. Nach 4 h wird erneut mit 6 mg einer 60 %igen Dispersion von Natriumhydrid in Mineralöl sowie 7 µl Methyliodid versetzt und über Nacht gerührt. Der Ansatz wird mit Essigester verdünnt und mit gesättigter NaCl Lösung gewaschen. Die vereinten organischen Phasen werden getrocknet (Na₂SO₄), filtriert und eingeengt. Der verbleibende Rückstand wird mit MTB-Ether digeriert. Man erhält 10 mg (0.03 mmol, entsprechend 27% der Theorie) des Produktes.
¹H-NMR (DMSO): 9.78 (s, 1H), 9.08 (m, 1H), 8.02 (s, 1H), 7.40 (m, 4H), 3.44 (m, 4H), 2.68 (s, 3H), 1.95 (m, 2H).
MS: 398 (ES).

### Herstellung der 4-Oxo-Derivate

### Verfahrensvariante 3a

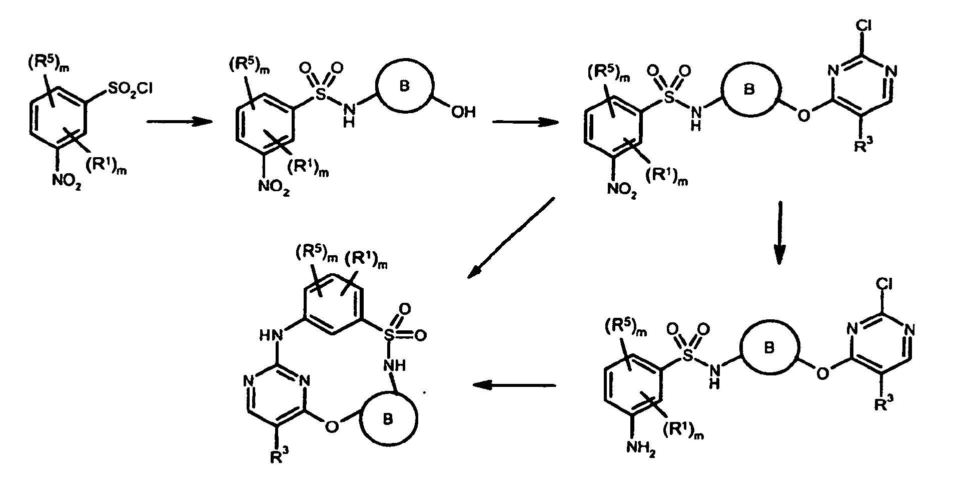

In den Formeln haben R¹, R³, R⁵, B und m die in den Verbindungen der Ansprüche 1 bis 3 verwirklichten Bedeutungen.

### Beispiel 3.0

### Herstellung von 1⁵-Bromo-9-oxa-4-thia-2,5-diaza-1(2,4)-pyrimidina-3(1,3)-benzenacyclononaphane-4,4-dioxide

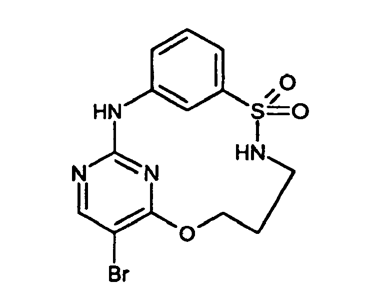

Eine Lösung von 30 mg (0,07 mmol) 3-Amino-*N*-[3-(5-brom-2-chlor-pyrimidin-4-yloxy)-propyl]-benzolsulfonamid in Acetonitril / DMSO (9,5 ml / 0,5 ml) wird mittels Spritzenpumpe innerhalb von 2 Stunden zu einer refluxierenden Lösung von Acetonitril / Wasser / 4 molare Lösung von Chlorwasserstoff in Dioxan (22,5 ml / 2,5 ml / 0,3 ml) gegeben. Nach weiteren 16 h wird das Acetonitril am Rotationsverdampfer abgezogen und der Rückstand mit 1 M NaHCO₃ Lösung versetzt. Man extrahiert mit Essigester (2x). Die vereinten organischen Phasen werden getrocknet (Na₂SO₄), filtriert und eingeengt. Der erhaltene Rückstand wird chromatographisch gereinigt (DCM / EtOH 9:1). Man erhält 8 mg (0,02 mmol, entsprechend 30% der Theorie) des Produktes.
¹H-NMR (DMSO): 10.23 (s, 1H), 9.08 (m, 1H), 8.41 (s, 1H), 7.88 (br, 1H), 7.36 (m, 3H), 4.58 (m, 2H), 3.30 (m, 2H), 2.07 (m, 2H).
MS: 385 (ES).

### Herstellung der Zwischenprodukte nach Verfahrensvariante 3a

### 3a) Herstellung von N-[3-(5-Brom-2-chlor-pyrimidin-4-yloxy)-propyl]-3-nitro-benzolsulfonamid

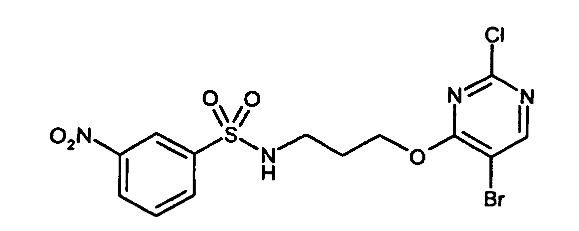

Eine Lösung von 272 mg (1,05 mmol) N-(3-Hydroxy-propyl)-3-nitro-benzolsulfonamid in 5 ml DMF wird mit 49 mg einer 60 %igen Dispersion von Natriumhydrid in Mineralöl (1,22 mmol) versetzt und 5 min bei Raumtemperatur gerührt. Man versetzt mit einer Lösung von 220 mg (0.97 mmol) 5-Brom-2,4-dichlor-pyrimidin in 5 ml DMF und rührt weitere 2 Stunden. Der Ansatz wird mit gesättigter NaCl Lösung versetzt und anschließend mit Essigester extrahiert (2x). Die vereinten organischen Phasen werden mit NaCl Lösung gewaschen, getrocknet (Na₂SO₄), filtriert und eingeengt. Der verbleibende Rückstand wird chromatographisch gereinigt (Hexan / Essigester 1:1, Flashmaster II). Man erhält 75 mg (0,16 mmol, entsprechend 16% der Theorie) des Produktes.
¹H-NMR (DMSO): 8.66 (s, 1H), 8.47 (m, 2H), 8.16 (m, 1H), 8.06 (m, 1H), 7. 88 (t, 1H), 4.37 (t, 2H), 3.00 (m, 2H), 1.96 (m, 2H).
MS: 451 (ES).

### 3b) Herstellung von 3-Amino-N-[3-(5-brom-2-chlor-pyrimidin-4-yloxy)-propyl]-benzolsulfonamid

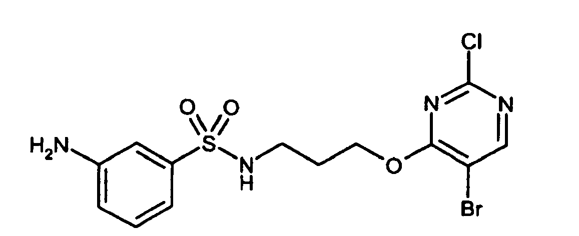

Eine Lösung von 70 mg (0.16 mmol) *N*-[3-(5-Brom-2-chlor-pyrimidin-4-yloxy)-propyl]-3-nitro-benzolsulfonamid in 5 ml THF wird bei 0 °C mit 1,0 ml einer 15%igen Lösung von Ti(III)Cl in etwa 10%iger Salzsäure versetzt. Nach 2 Stunden wird die Reaktionslösung erneut mit 0,2 ml der Ti(III)Cl-Lösung versetzt und eine weitere Stunde gerührt. Der Ansatz wird mit 1 N NaOH Lösung basisch gestellt und anschließend filtriert. Der Filterkuchen wird 2x mit jeweils 50 ml Essigester / MeOH (30 ml / 20 ml) nachgewaschen. Das Filtrat wird am Rotationsverdampfer eingeengt und danach mit Essigester extrahiert (2x). Die vereinten organischen Phasen werden mit NaCl Lösung gewaschen, getrocknet (Na₂SO₄), filtriert und eingeengt. Der verbleibende Rückstand wird chromatographisch gereinigt (Hexan / Essigester 1:1, Flashmaster II). Man erhält 32 mg (0,08 mmol, entsprechend 49% der Theorie) des Produktes.
¹H-NMR (DMSO): 8.67 (s, 1H), 7.49 (t, 1H), 7.12 (t, 1H), 6.96 (m, 1H), 6.81 (m, 1H), 6.68 (m, 1H), 5.54 (s, 1H), 4.39 (m, 2H), 2.96 (m, 2H), 1.87 (m, 2H).
MS: 421 (ES). ,

### Herstellung der 5-Carboxamid-Derivate

### Verfahrensvariante 4

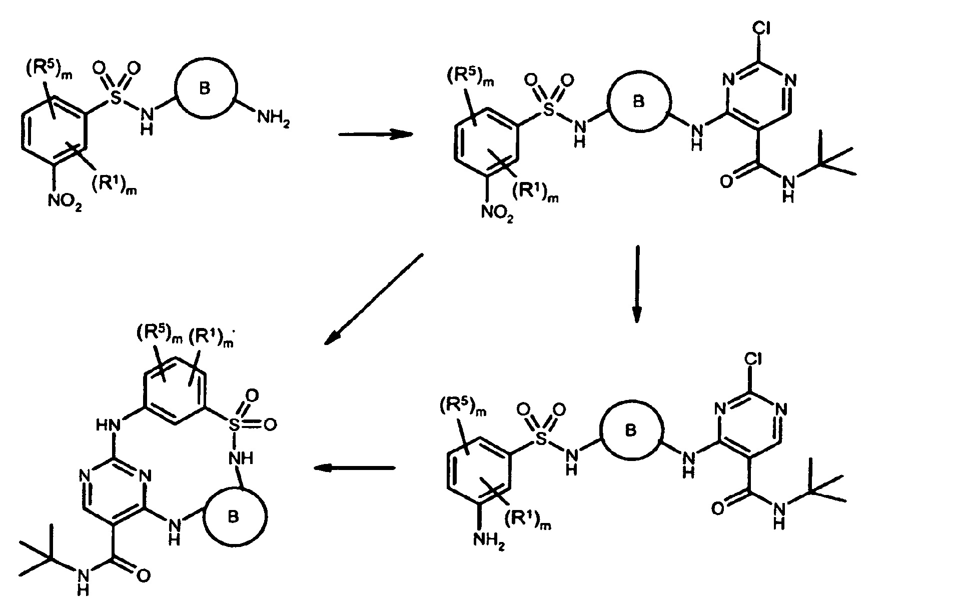

In den Formeln haben R¹, R⁵, B und m die in den Verbindungen der Ansprüche 1 bis 3 verwirklichten Bedeutungen .

### Beispiel 4.0

### Herstellung von N-tert-Butyl-4-thia-2,5,9-triaza-1(2,4)-pyrimidina-3(1,3)-benzenacyclononaphan-1⁵-carboxamid-4,4-dioxid

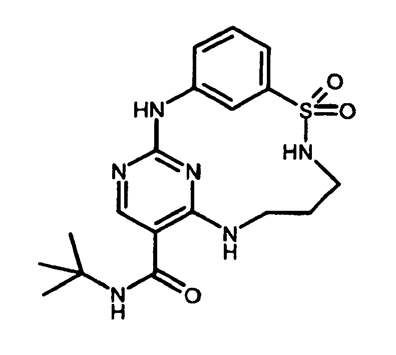

Eine Lösung von 150 mg (0,32 mmol) 2-Chlor-4-[3-(3-nitro-benzolsulfonylamino)-propylamino]-pyrimidin-5-carboxylsäure-*tert*-butylamid in 10 ml THF wird unter Argon bei Raumtemperatur mit 1,6 ml einer 15%igen Lösung von Ti(III)Cl in etwa 10%iger Salzsäure versetzt. Nach 17 Stunden wird die Reaktionslösung erneut mit 0,3 ml der Ti(III)Cl-Lösung versetzt und weitere 4 Stunden gerührt. Der Ansatz wird mit 1M NaOH Lösung basisch gestellt und anschließend filtriert. Der Filterkuchen wird 2x mit jeweils 50 ml Essigester / MeOH (30 ml / 20 ml) nachgewaschen. Das Filtrat wird am Rotationsverdampfer eingeengt und danach mit Essigester extrahiert (2x). Die vereinten organischen Phasen werden getrocknet (Na₂SO₄), filtriert und eingeengt. Beim Einengen fällt ein farbloser Feststoff aus, der abfiltriert und getrocknet wird. Man erhält 25 mg (0,06 mmol, entsprechen 18% der Theorie) des Produktes.
¹H-NMR (DMSO): 9.95 (s, 1H), 9.45 (s, 1H), 8.82 (t, 1H), 8.49 (s, 1H), 7.78 (t, 1H), 7.58 (s, 1H), 7.38 (m, 3H), 3.50 (m, 2H), 3.30 (m, 2H), 1.86 (m, 2H).
MS: 405 (ES).

### Herstellung der Zwischenprodukte nach Verfahrensvariante 4

### 4a) Herstellung von 2,4-Dichlor-pyrimidin-5-carbonylchlorid

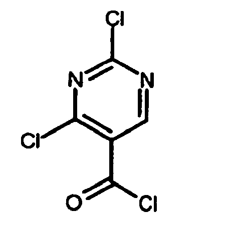

Eine Suspension von 21,7 g (139 mmol) 2,4-Dihydroxy-5-carbonsäure-pyrimidin, 96,7 g (463 mmol) Phosphorpentachlorid und 33 ml (348 mmol) Phosphoroxydchlorid wird 5 Stunden bei 115 °C gerührt. Nach dem Abkühlen wird das Reaktionsgemisch am Rotationsverdampfer eingeengt. Der gebildete Rückstand wird durch Vakuumdestillation (K_{p 0.1mbar}: 68°C) gereinigt. Man erhält 24,9 g (117 mmol, entsprechend 84 % der Theorie) des Produktes.
¹H-NMR (DMSO): 9.11 (s, 1H).

### 4b) Herstellung von 2,4-Dichlor-pyrimidin-5-carbonsäure-tert-butylamide

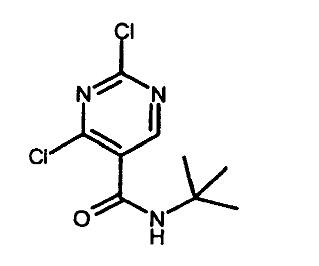

Eine Lösung von 24,85 g (117,5 mmol) 2,4-Dichlor-pyrimidin-5-carbonylchlorid in 125 ml THF wird auf -15 °C abgekühlt. Man versetzt langsam mit einer Lösung von 13.2 ml (124,5 mmol) *tert*-Buylamin und 17,4 ml (125,7 mmol) Triethylamin in 50 ml THF, so dass die Temperatur des Reaktionsgemisches kleiner -10 °C bleibt. Es wird weitere 2 Stunden bei -10 °C gerührt, dann wird das Kühlbad entfernt und das Reaktionsgemisch unter Rühren auf Raumtemperatur erwärmt. Nach 1 Stunde wird der gebildete Niederschlag abfiltriert und das Filtrat vollständig eingeengt. Der erhaltene Rückstand wird chromatographisch gereinigt (Hexan / Essigester 4:1). Man erhält 14,01 g (56,6 mmol, entsprechend 50% der Theorie) des Produktes.
¹H-NMR (DMSO): 8.81 (s, 1H), 8.34 (s, 1H), 1.36 (s, 9H).

### 4c) 2-Chlor-4-[3-(3-nitro-benzolsulfonylamino)-propylamino]-pyrimidin-5-carbonsäure-tert-butylamid

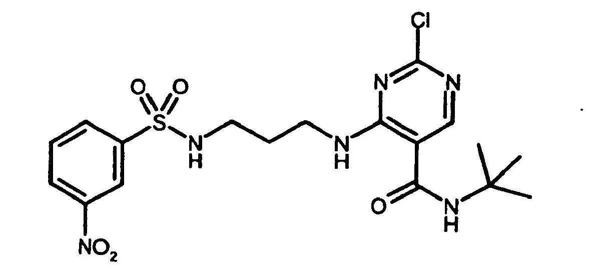

Eine Lösung von 0,95 g (3,83 mmol) 2,4-Dichlor-pyrimidin-5-carbonsäure-*tert-*butylamid in 6 ml THF wird bei Raumtemperatur unter Rühren mit einer Suspension aus 1,00 g (3,86 mmoi) *N*-(3-Amino-propyt)-3-nitro-benzolsulfonamid in 9 ml THF / 0,55 ml Triethylamin versetzt. Nach 19 Stunden wird der gebildete Niederschlag abgesaugt und mit Essigester gewaschen. Das Filtrat wird einrotiert und der gebildete Rückstand chromatographisch gereinigt (Hexan / Essigester 2:1, Flashmaster II). Man erhält 0,79 g (1,67 mmol, entsprechend 44% der Theorie) des Produktes.
¹H-NMR (DMSO): 8.74 (t, 1H), 8.47 (m, 3H), 8.18 (dd, 1H), 8.04 (t, 1H), 7.98 (s, 1H), 7.85 (t, 1H), 3.30 (m, 2H), 2.87 (m, 2H), 1.68 (m, 2H), 1.36 (s, 9H).

### Herstellung der 5-Cyano-Derivate

### Verfahrensvariante 5

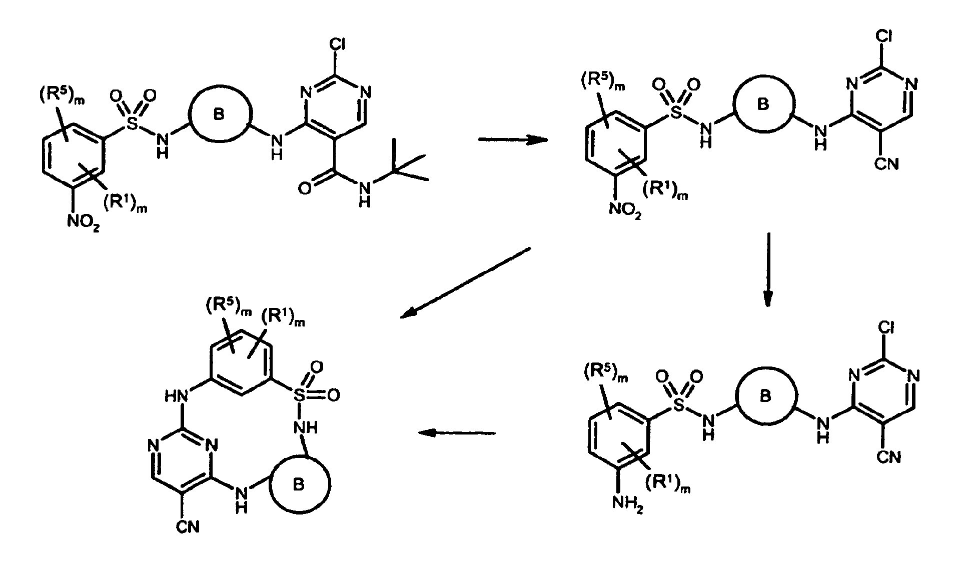

In den Formeln haben R¹, R⁵, B und m die in den Verbindungen der Ansprüche 1 bis 3 verwirklichten Bedeutungen .

### Beispiel 5.0

### Herstellung von 1⁵-Cyano-4-thia-2,5,9-triaza-1(2,4)-pyrimidina-3(1,3)-benzenacyclononaphan 4,4-dioxide

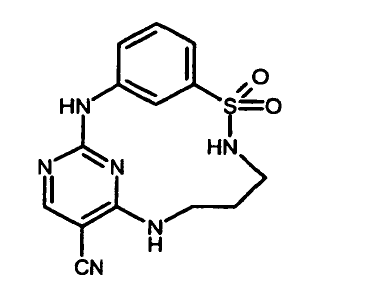

Eine Lösung von 100 mg (0,25 mmol) *N*-[3-(2-Chlor-5-cyano-pyrimidin-4-ylamino)-propyl]-3-nitro-benzolsulfonamid in 10 ml THF wird unter Argon bei Raumtemperatur mit 1,2 ml einer 15%igen Lösung von Ti(III)Cl in etwa 10%iger Salzsäure versetzt. Nach 3,5 Stunden wird der Ansatz mit Essigester verdünnt, mit 1 M NaOH Lösung basisch gestellt (pH 13) und anschließend filtriert. Der Filterkuchen wird mit 50 ml Essigester / MeOH (30 ml / 20 ml) und 70 ml Essigester / MeOH / 1 N NaOH (40 ml / 20 ml / 10 ml) nachgewaschen. Das Filtrat wird am Rotationsverdampfer eingeengt und danach mit Essigester extrahiert (2x). Die vereinten organischen Phasen werden getrocknet (Na₂SO₄), filtriert und eingeengt. Beim Einengen fällt das Produkt als farbloser Feststoff aus, der abfiltriert wird. Man erhält 30 mg (0,09 mmol, entsprechend 36% der Theorie) des Produktes.
¹H-NMR (DMSO): 10.29 (s, 1H), 9.29 (s, 1H), 8.39 (s, 1H), 8.15 (br, 1H), 7.78 (br, 1H), 7.38 (m, 3H), 3.30 (m, 4H), 1.85 (m, 2H).
MS: 331 (ES).

### Herstellung der Zwischenpodukte nach Verfahrensvariante 5

### 5a) Herstellung von N-[3-(2-Chlor-5-cyano-pyrimidin-4-ylamino)-propyl]-3-nitro-benzolsulfonamid

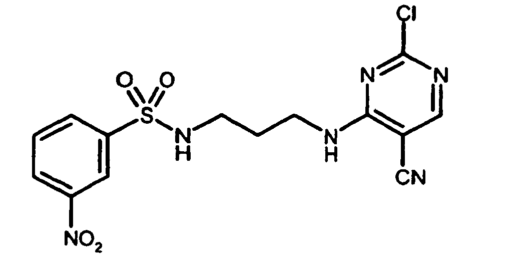

125 mg (0,27 mmol) 2-Chlor-4-[3-(3-nitro-benzolsulfonylamino)-propylamino]-pyrimidin-5-carbonsäure-*tert*-butylamid werden mit 4 ml Thionylchlorid versetzt und 19 Stunden unter Rückfluss gerührt. Das Reaktionsgemisch wird eingeengt. Man versetzt mit Wasser und Toluol und engt am Rotationsverdampfer bis zur Trockene ein. Man erhält 110 mg (0,27 mmol, entsprechend 100% der Theorie) des Produktes.
¹H-NMR (DMSO): 8.50 (m, 4H), 8.19 (d, 1H), 8.01 (t, 1H), 7.88 (t, 1H), 3.30 (m, 2H), 2.87 (m, 2H), 1.71 (m, 2H).

### Herstellung der Thiophen-Derivate

### Verfahrensvariante 6a

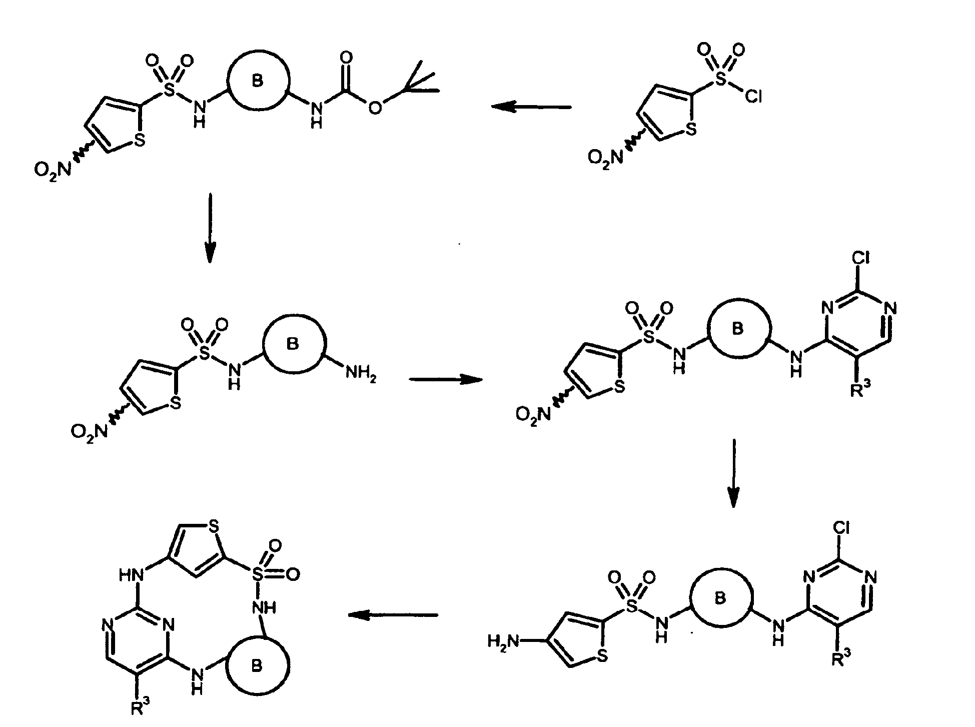

In den Formeln haben R³ und B die in den Verbindungen der Ansprüche 1 bis 3 verwirklichten Bedeutungen.

### Beispiel 6.0

### Herstellung von 1⁵-Bromo-4-thia-2,5,8-triaza-1(2,4)-pyrimidina-3(4,2)-thiophenacyclooctaphan-4,4-dioxide

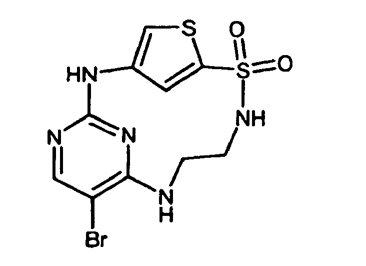

Eine Lösung von 170 mg (0,41 mmol) 4-Amino-thiophenylen-2-sulfonsäure-[2-(5-brom-2-chlor-pyrimidin-4-ylamino)-ethyl]-amid in Acetonitril / Wasser (12,m0 ml / 1,5 ml) wird mittels Spritzenpumpe innerhalb von 2 Stunden zu einer refluxierenden Lösung von Acetonitril / Wasser / 4 molare Lösung von Chlorwasserstoff in Dioxan (64 ml / 7 ml / 0,8 ml) gegeben. Nach Beendigung der Zugabe wird das Reaktionsgemisch für weitere 6 Stunden unter Rückfluss gerührt. Nach dem Abkühlen wird das Lösungsmittel am Rotationsverdampfer abgezogen. Der Rückstand wird mit 2N NaOH versetzt und mit Essigester extrahiert (2x). Die vereinten organischen Phasen werden mittels Whatman-Filter filtriert und eingeengt. Der verbleibende Rückstand wird aus MeOH / Disopropylether kristallisiert. Man erhält 41 mg (0,11 mmol, entsprechend 27% der Theorie) des Produktes.
¹H-NMR (DMSO): 9.03 (s, 1H), 7.92 (s, 1H), 7.68 (d, 1H), 7.48 (br, 1H), 7.38 (d, 1H), 7.08 (t, 1H), 2.91 (m, 4H).
MS: 376 (ES).

### Herstellung der Zwischenprodukte nach Verfahrensvariante 6a

### 6a) Herstellung von 4-Nitro-thiophen-2-sulfonylchlorid (A) und 5-Nitro-thiophen-2-sulfonylchlorid (B)

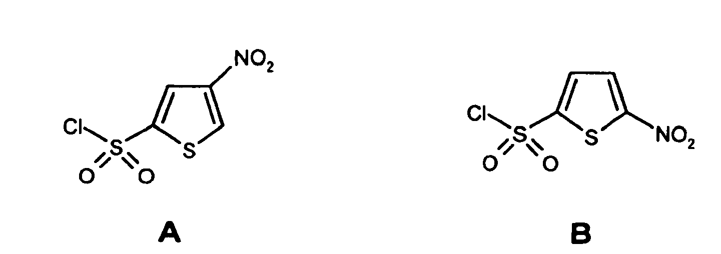

Eine Lösung von 25 g (137 mmol) Thiophen-2-sulfonylchlorid in 20 ml Dichlormethan wird langsam unter Rühren zu 98 ml konz. Salpetersäure getropft. Das Reaktionsgemisch wird 2 Stunden bei 40 °C gerührt und anschließend auf Eis gegeben. Man extrahiert mit Dichlormethan (2x). Die vereinten organischen Phasen werden über MgSO₄ getrocknet, filtriert und eingeengt. Man erhält 24 g (105 mmol, entsprechend 77 % der Theorie) eines Gemisches der Produkte A und B im Verhältnis 2 /1.
¹H-NMR (DMSO): 8.63 (d, 1H, **A**), 7.93 (d, 1H, **B**), 7.54 (d, 1H, **A**), 7.18 (d, 1H, **B**).

### 6b) Herstellung von [2-(4-Nitro-thiophen-2-sulfonylamino)-ethyl]-carbaminsäure-tert-butylester (A) und [2-(5-Nitro-thiophen-2-sulfonylamino)-ethyl]-carbaminsäure-tert-butyl-ester (B)

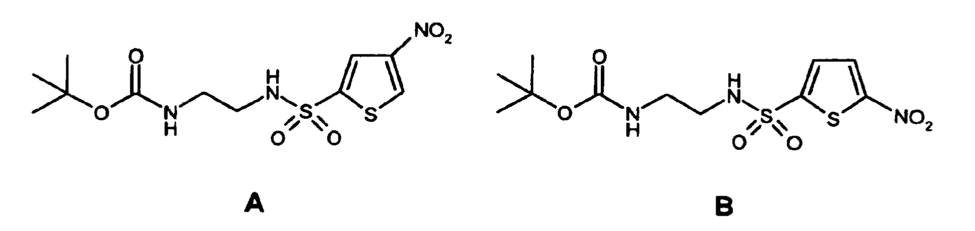

Zu einer Lösung von 2,27 g (10 mmol) eines Gemisches von 4-Nitro-thiophen-2-sulfonylchlorid und 5-Nitro-thiophen-2-sulfonylchlorid im Verhältnis 2 / 1 sowie 1,64 g (10 mmol) (2-Amino-ethyl)-carbaminsäure-*tert*-butylester in 40 ml Aceton und 10 ml Wasser werden 2,8 ml (20 mmol) Triethylamin gegeben. Das Reaktionsgemisch wird 3 Stunden bei Raumtemperatur gerührt und anschließend das Aceton am Rotationsverdampfer abgezogen. Nach der Zugabe von Wasser (20 ml) wird mit Essigester extrahiert (2x). Die vereinten organischen Phasen werden über einen Whatman-Filter filtriert und eingeengt. Man erhält 2,65 g (7,5 mmol, entsprechend 75% der Theorie) eines Gemisches der Verbindungen **A** und **B** im Verhältnis 1 / 1.
¹H-NMR (DMSO): 9.02 (d, 1H, **A**), 8.85 (t, 1H), 8.15 (m, 2H), 8.02 (d, 1H, **A**), 7.63 (d, 1H, **B**), 6.78 (m, 2H), 2.92 (m, 8H), 1.40 (s, 18H).

### 6c) Herstellung von 4-Nitro-thiophen-2-sulfonsäure-[2-(5-brom-2-chlor-pyrimidin-4-ylamino)-ethyl]-amid (A) und 5-Nitro-thiophen-2-sulfonsäure-[2-(5-brom-2-chlor-pyrimidin-4-ylamino)-ethyl]-amid (B)

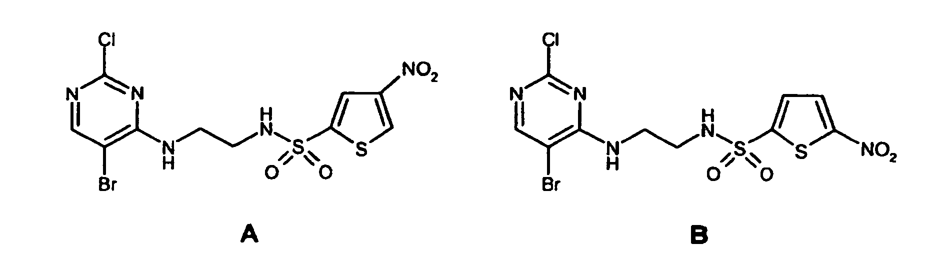

2,65 g (7,54 mmol) eines Gemisches von [2-(4-Nitro-thiophen-2-sulfonylamino)-ethyl]-carbaminsäure-*tert*-butylester und [2-(5-Nitro-thiophen-2-sulfonylamino)-ethyl]-carbaminsäure-*tert*-butylester im Verhältnis 1 / 1 werden mit 9 ml TFA versetzt und 2,5 Stunden bei Raumtemperatur gerührt. Das Reaktionsgemisch wird am Rotationsverdampfer eingeengt und mit Wasser und 1 N NaOH (pH 13) versetzt. Man extrahiert mit Essigester (2x). Die vereinten organischen Phasen werden über einen Whatman-Filter filtriert und eingeengt. Der verbleibende Rückstand wird chromatographisch (Dichlormethan / MeOH 1:1) aufgereinigt. Das erhaltene Rohprodukt wird in 3 ml Acetonitril aufgenommen und mit einer Lösung von 1,37 g (3 mmol) 5-Brom-2,4-dichlor-pyrimidine / 1 ml Triethylamin (7 mmol) in 3 ml Acetonitril versetzt. Nach 16 Stunden wird das Reaktionsgemisch am Rotationsverdampfer eingeengt und der verbleibende Rückstand chromatographisch (Hexan / Essigester 2:1, Flashmaster II) gereinigt. Man erhält 0,87 g (1,97 mmol, entsprechend 26 % der Theorie) eines Gemisches der Regioisomeren A und B im Verhältnis 10 / 6.
¹H-NMR (DMSO): 8.98 (d, 1H, A), 8.50 (t, 1H, B), 8.32 (t, 2H, A), 8.20 (s, 2H, A+B), 8.05 (d, 1H, B), 7.98 (d, 1H, A), 7.63 (m, 3H, A+B), 3.47 (m, 4H, A+B), 3.20 (m, 4H, A+B).
MS: 4.42 (ES)

### 6d) 4-Amino-thiophen-2-sulfonsäure-[2-(5-brom-2-chlor-pyrimidin-4-ylamino)-ethyl]-amid

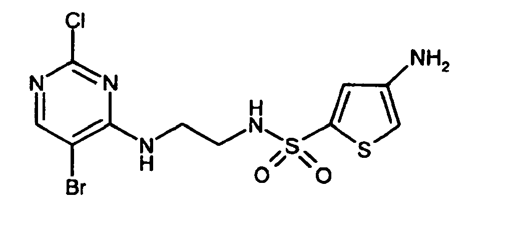

Eine Lösung von 600 mg (1,35 mmol) eines Gemisches von 4-Nitro-thiophen-2-sulfonsäure [2-(5-brom-2-chlor-pyrimidin-4-ylamino)-ethyl]-amid und 5-Nitro-thiophen-2-sulfonsäure-[2-(5-brom-2-chlor-pyrimidin-4-ylamino)-ethyl]-amid (Verhältnis 10 / 6) in 40 ml THF wird unter Argon bei Raumtemperatur mit 6,4 ml einer 15%igen Lösung von Ti(III)Cl in etwa 10%iger Salzsäure versetzt. Nach 46 Stunden wird die Reaktionslösung erneut mit 2,0 ml der Ti(III)Cl-Lösung versetzt und weitere 7 Stunden gerührt. Der Ansatz wird mit 2N NaOH Lösung basisch gestellt und anschließend filtriert. Der Filterkuchen wird 2x mit jeweils 50 ml Essigester / MeOH (30 ml / 20 ml) nachgewaschen. Das Filtrat wird am Rotationsverdampfer eingeengt und danach mit Essigester extrahiert (2x). Die vereinten organischen Phasen werden über einem Whatman-Filter filtriert und eingeengt. Der Rückstand wird chromatographisch (Hexan / Essigester 1 : 4) gereinigt. Man erhält 178 mg (0,43 mmol, entsprechend 32 % der Theorie) des Produktes.
¹H-NMR (DMSO): 8.21 (s, 1H), 7.82 (t, 1H), 7.65 (t, 1H), 7.03 (d, 1H), 6.31 (d, 1H), 5.21 (br, 2H), 3.44 (m, 2H), 3.02 (m, 2H).
MS: 412 (ES).

### Herstellung der Zwischenprodukte nach Verfahrensvariante 6b

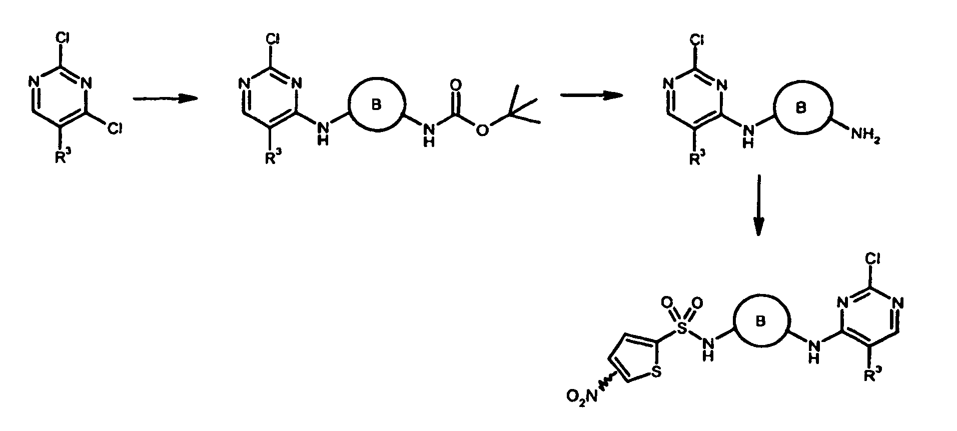

In den Formeln haben R³ und B die in den Verbindungen der Ansprüche 1 bis 3 verwirklichten Bedeutungen.

### 6e) Herstellung von 4-Nitro-thiophen-2-sulfonsäure-[3-(5-brom-2-chlor-pyrimidin-4-ylamino)-propyl]-amid (A) und 5-Nitro-thiophen-2-sulfonsäure-[3-(5-brom-2-chlor-pyrimidin-4-ylamino)-propyl]-amid

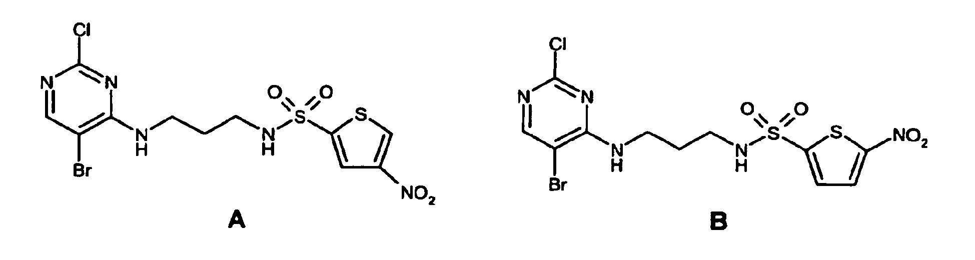

Eine Lösung von 995 mg (3,3 mmol) *N*-(5-Brom-2-chlor-pyrimidin-4-yl)-propan-1,3-diamin Hydrochlorid und 700 mg (3,1 mmol) eines Gemisches von 4-Nitro-thiophen-2-sulfonylchlorid und 5-Nitro-thiophen-2-sulfonylchlorid im Verhältnis 1 / 1 in 40 ml Aceton / 10 ml Wasser wird bei Raumtemperatur mit 2 ml (14,4 mmol) Triethylamin versetzt. Nach 15 min wird das organische Lösungsmittel am Rotationsverdampfer abgezogen. Man versetzt mit 150 ml Essigester und wäscht mit Zitronensäure (10%), gesättigter NaHCO₃ Lösung sowie gesättigter NaCl Lösung. Die organische Phase wird getrocknet (Na₂SO₄), filtriert und eingeengt. Man erhält 860 mg (1,9 mmol, entsprechend 62 % der Theorie) eines Gemisches der Produkte A und B im Verhältnis 1 / 1.
¹H-NMR (DMSO): 9.00 (d, 1H, **A**), 8.39 (t, 1H), 8.20 (m, 4H), 8.12 (d, 1H, **B**), 8.02 (d, 1H, **A**), 7.68 (t, 1H), 7.61 (d, 1H, **B**), 3.30 (m, 4H), 2.90 (m, 4H), 1.75 (m, 4H).

### Herstellung der Oxa-phane und Einführung von Sulfamoylgruppierungen

### Verfahrensvariante 7

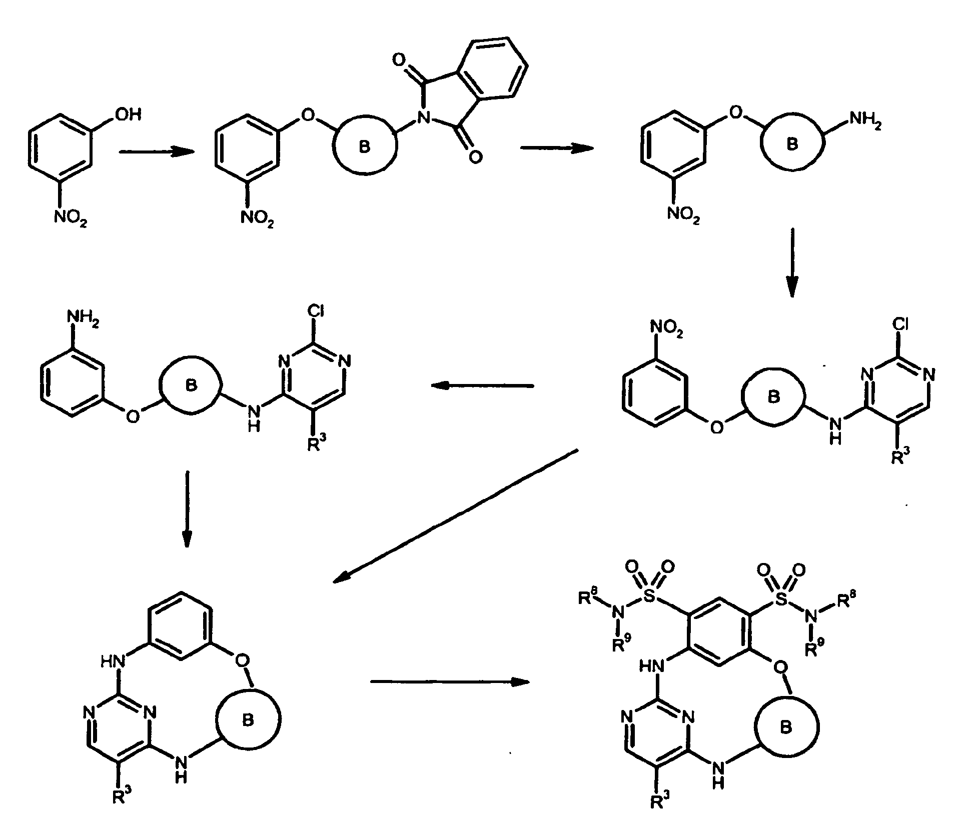

In den Formeln haben R³, R⁸, R⁹ und B die in den Verbindungen der Ansprüche 1 bis 3 verwirklichten Bedeutungen .

### Beispiel 7.0

### Herstellung von 1⁶-Bromo-N,N'-dimethyl-4-oxa-2,9-diaza-1(2,4)-pyrimidina-3(1,3)-benzenacyclononaphan-3⁴,3⁶-disulfonamide

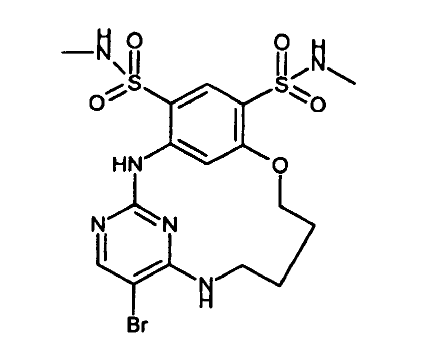

10 ml Chlorsulfonsäure werden unter Kühlung (4°C) vorsichtig mit 75 mg Phosphorpentachlorid versetzt. Man gibt 60 mg (0,18 mmol) 1⁵-Bromo-4-oxa-2,9-diaza-1(2,4)-pyrimidina-3(1,3)-benzenacyclononaphane hinzu und rührt 3 Stunden bei Raumtemperatur. Das Reaktionsgemisch wird vorsichtig auf Eiswasser gegeben und 1 Stunde gerührt. Der gebildete Feststoff wird abgesaugt und in 1 ml THF aufgenommen. Man versetzt mit 2 ml einer Lösung von Methylamin in Ethanol und rührt 12 Stunden bei Raumtemperatur. Das Reaktionsgemisch wird eingeengt und der verbleibende Rückstand chromatographisch (Dichlormethan / Methanol 1 : 1) gereinigt. Man erhält 8 mg (0,02 mmol, entsprechend 10% der Theorie) des Produktes.
¹H-NMR (DMSO): 9.43 (s, 1H), 9.17 (s, 1H), 8.15 (s, 1H), 8.03 (s, 1H), 7.95 (q, 1H), 7.72 (t, 1H), 7.15 (q, 1H), 4.55 (br, 2H), 3.42 (br, 2H), 2.45 (m, 6H), 1.91 (m, 4H).
MS: 521 (ES).

### Beispiel 7.1

### Herstellung von 1⁵-Bromo-4-oxa-2,9-diaza-1(2,4)-pyrimidina-3(1,3)-benzenacyclononaphane

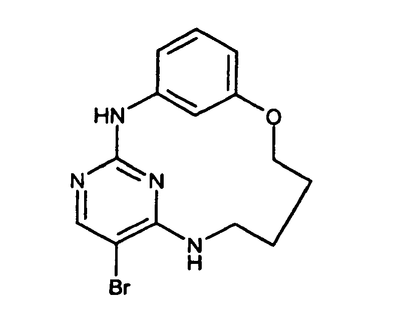

Eine Lösung von 295 mg (0,79 mmol) [4-(3-Amino-phenoxy)-butyl]-(5-brom-2-chlor-pyrimidin-4-yl)-amin in Acetonitril / Wasser (18 ml / 2 ml) wird mittels Spritzenpumpe innerhalb von 4,5 Stunden zu einer refluxierenden Lösung von Acetonitril / Wasser / 4 molare Lösung von Chlorwasserstoff in Dioxan (105 ml / 12 ml / 1,4 ml) gegeben. Nach weiteren 60 min unter Rückfluss wird das Ölbad abgeschaltet und die Reaktionslösung über Nacht bei Raumtemperatur gerührt. Das Reaktionsgemisch wird mit 2N NaOH basisch gestellt und mit Essigester extrahiert (3x). Die vereinten organischen Phasen werden mit NaCl Lösung gewaschen, getrocknet (MgSO₄), filtriert und eingeengt. Der verbleibende Rückstand wird mit Methanol digeriert. Man erhält 65 mg (0,19 mmol, entsprechend 24% der Theorie) des Produktes.
¹H-NMR (DMSO): 9.31 (s, 1H), 8.82 (s, 1H), 8.01 (s, 1H), 7.37 (br, 1H), 7.02 (t, 1H), 6.63 (d, 1H), 6.36 (dd, 1H), 4.34 (br, 2H), 3.30 (m, 2H), 1.85 (m, 4H).
MS: 335 (ES).

### Herstellung der Zwischenprodukte nach Verfahrensvariante 7

### 7a) Herstellung von 2-[4-(3-Nitro-phenoxy)-butyl]-isoindol-1,3-dion

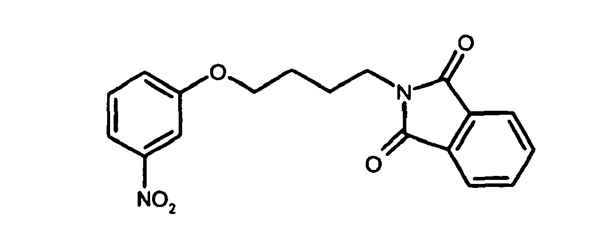

Zu einer Lösung von 6,96 g (50 mmol) 3-Nitrophenol in 500 ml DMF werden 9,67 g (70 mmol) Kaliumcarbonat gegeben und anschließend 10 min bei Raumtemperatur gerührt. Man versetzt mit 14,1 g (50 mmol) 2-(4-Brom-butyl)-isoindol-1,3-dion und rührt 4 Stunden bei 60 °C. Nach dem Erkalten wird mit Wasser versetzt und mit Essigester extrahiert. Die vereinten organischen Phasen werden getrocknet (MgSO₄), filtriert und eingeengt. Man erhält 17,2 g (50 mmol, entsprechend 100% der Theorie) des Produktes.
¹H-NMR (CDCl₃): 7.85 (m, 3H), 7.70 (m, 3H), 7.40 (t, 1H), 7.18 (dd, 1H), 4.06 (m, 2H), 3.80 (m, 2H), 1.95 (m, 4H).

### 7b) Herstellung von 4-(3-Nitro-phenoxy)-butylamin

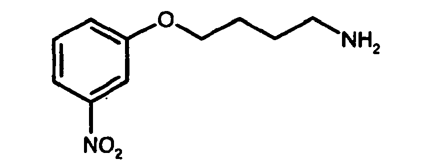

Eine Lösung von 17,0 g (50 mmol) 2-[4-(3-Nitro-phenoxy)-butyl]-isoindol-1,3-dion in 1000 ml Ethanol wird mit 25 ml Hydrazin versetzt und 2 Stunden bei 70 °C gerührt. Nach dem Abkühlen wird der gebildete Niederschlag abgesaugt und das Filtrat einrotiert. Der Rückstand wird in Dichlormethan aufgenommen. Es wird erneut filtriert und das Filtrat vollständig eingeengt. Man erhält 5,8 g (28 mmol, entsprechend 56 % der Theorie) des Produktes.
¹H-NMR (CDCl₃): 7.79 (dd, 1H), 7.71 (t, 1H), 7.39 (t, 1H), 7.20 (dd, 1H), 4.03 (m, 2H), 2.79 (m, 2H), 1.85 (m, 2H), 1.70 (m, 2H).

### 7c) Herstellung von (5-Brom-2-chlor-pyrimidin-4-yl)-[4-(3-nitro-phenoxy)-butyl]-amin

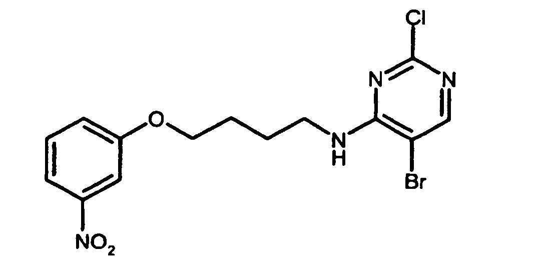

Eine Lösung 2,28 g (10 mmol) 5-Brom-2,4-dichtor-pyrimidin und 1,4 ml Triethylamin (10 mmol) in 32 ml Acetonitril wird bei 4 °C unter Rühren mit einer Lösung von 2,1 g (10 mmol) 4-(3-Nitro-phenoxy)-butylamin in 5 ml Acetonitril versetzt. Nach 12 Stunden wird mit Essigester verdünnt und filtriert. Das Filtrat wird am Rotationsverdampfer eingeengt und der verbleibende Rückstand chromatographisch (Hexan / Essigester 1:1, Flashmaster II) gereinigt. Man erhält 2,7 g (7 mmol, entsprechend 70 % der Theorie) des Produktes.
¹H-NMR (CDCl₃): 8.11 (s, 1H), 7.81 (dd, 1H), 7.71 (t, 1H), 7.44 (t, 1H), 7.20 (dd, 1H), 5.62 (br, 1H), 4.11 (m, 2H), 3.62 (m, 2H), 1.92 (m, 4H).

### 7d) Herstellung von 4-(3-Amino-phenoxy)-butyl]-(5-brom-2-chlor-pyrimidin-4-yl)-amin

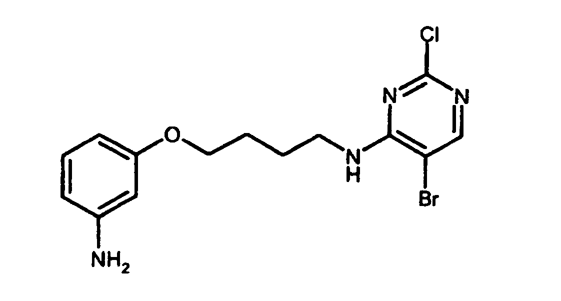

Eine Lösung von 401 mg (1,00 mmol) (5-Brom-2-chlor-pyrimidin-4-yl)-[4-(3-nitro-phenoxy)-butyl]-amin in 30 ml THF wird unter Argon bei Raumtemperatur mit 4,5 ml einer 15%igen Lösung von Ti(III)Cl in etwa 10%iger Salzsäure versetzt. Nach 3,5 Stunden wird die Reaktionslösung erneut mit 0,2 ml der Ti(III)Cl-Lösung versetzt und weitere 12 Stunden gerührt. Der Ansatz wird mit 2N NaOH Lösung basisch gestellt und anschließend filtriert. Der Filterkuchen wird 2x mit jeweils 50 ml Essigester / MeOH (30 ml / 20 ml) nachgewaschen. Das Filtrat wird am Rotationsverdampfer eingeengt und danach mit Essigester extrahiert (2x). Die vereinten organischen Phasen werden mit NaCl Lösung gewaschen, getrocknet (MgSO₄), filtriert und eingeengt. Der verbleibende Rückstand wird chromatographisch gereinigt (Hexan / Essigester 1 : 1, Flashmaster II). Man erhält 300 mg (0,81 mmol, entsprechend 81 % der Theorie) des Produktes.
¹H-NMR (CDCl₃): 8.10 (s, 1H), 7.03 (t, 1H), 6.36 (m, 3H), 5.66 (br, 1H), 3.97 (m, 2H), 3.60 (m, 2H), 1.86 (m, 2H).
MS: 371 (ES).

### Herstellung der Sulfonamid-Oxa-Cyclophane

### Verfahrensvariante 8a

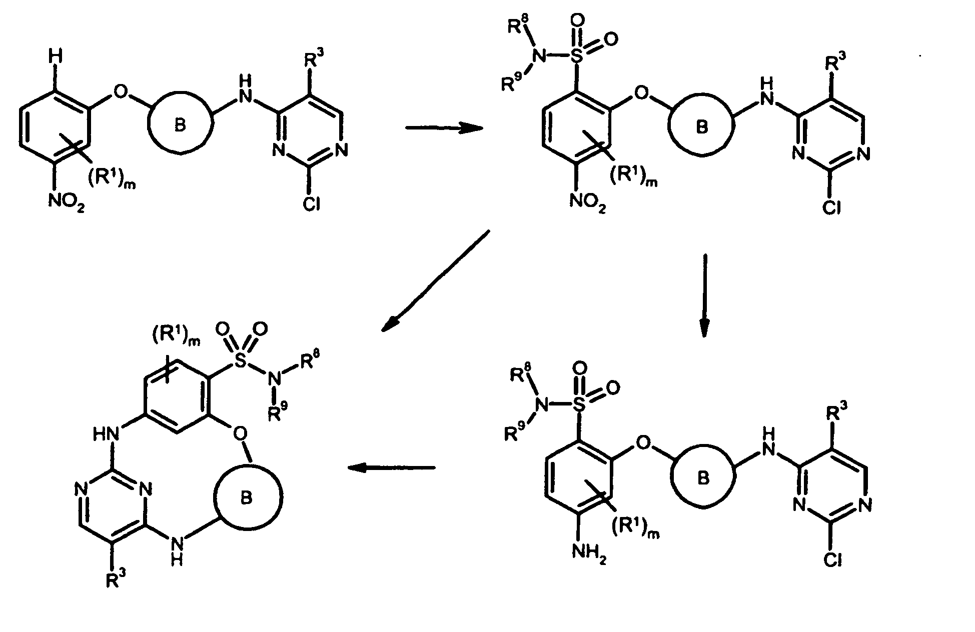

In den Formeln haben R¹, R³, R⁸, R⁹, B und m die in den Verbindungen der Ansprüche 1 bis 3 verwirklichten Bedeutungen .

### Beispiel 8.0

### Herstellung von 1⁵-Bromo-4-oxa-2,9-diaza-1(2,4)-pyrimidina-3(1,3)-benzenacyclononaphane-3⁴-sulfonamide

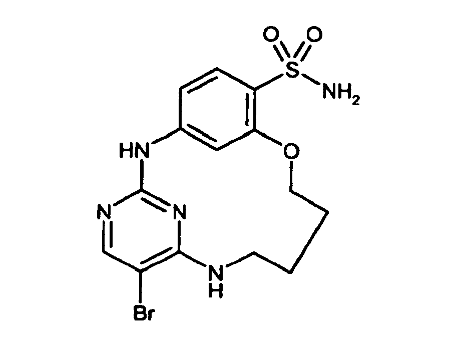

Eine Lösung von 66 mg (0,15 mmol) 4-Amino-2-[4-(5-brom-2-chlor-pyrimidin-4-ylamino)-butoxy]-benzolsulfonamid in Acetonitril / Wasser / 2-Butanol (8 ml / 1 ml / 1 ml) wird mittels Spritzenpumpe innerhalb von 3,5 Stunden zu einer refluxierenden Lösung von Acetonitril / Wasser / 4 molare Lösung von Chlorwasserstoff in Dioxan (45 ml / 5 ml / 0,6 ml) gegeben. Nach weiteren 16 Stunden unter Rückfluss wird das Reaktionsgemisch am Rotationsverdampfer eingeengt. Der Ansatz wird mit 1 N NaOH basisch gestellt (pH 13) und mit Essigester extrahiert (2x). Die vereinten organischen Phasen werden getrocknet (MgSO₄), filtriert und eingeengt. Der verbleibende Rückstand wird mit Hexan und *tert*-Butylmethylether digeriert. Man erhält 55 mg (0,13 mmol, entsprechend 87% der Theorie) des Produktes.
¹H-NMR (DMSO): 9.70 (s, 1H), 9.10 (d, 1H), 8.06 (s, 1H), 7.49 (m, 2H), 6.82 (s, 2H), 6.67 (dd, 1H), 4.45 (br, 2H), 3.40 (m, 2H), 1.85 (m, 4H).
MS: 414 (ES).

### Herstellung der Zwischenprodukten nach Verfahrensvariante 8a

### 8a) Herstellung von 2-[4-(5-Brom-2-chlor-pyrimidin-4-ylamino)-butoxy]-4-nitro-benzolsulfonamid (A) und 4-[4-(5-Brom-2-chlor-pyrimidin-4-ylamino)-butoxy]-2-nitro-benzolsulfonamid (B)

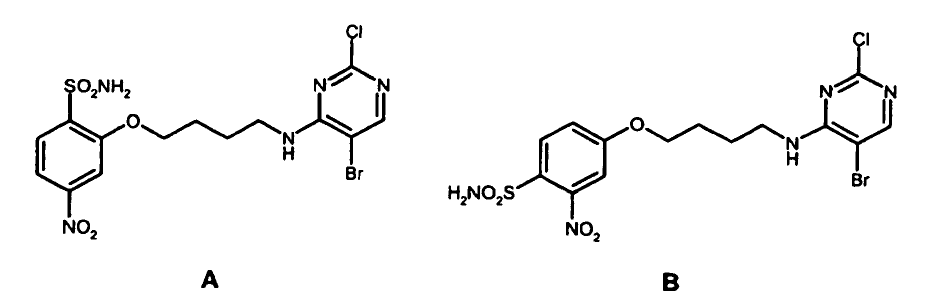

402 mg (1,01 mmol) (5-Brom-2-chlor-pyrimidin-4-yl)-[4-(3-nitro-phenoxy)-butyl]-amin werden portionsweise in 4 ml eiskalter Chlorsufonsäure (Kühlung: Eis / Methanol) eingetragen und anschließend 3 Stunden bei Raumtemperatur gerührt. Der Ansatz wird vorsichtig unter Rühren auf Eiswasser gegeben. Der gebildete Niederschlag wird abgesaugt, in Aceton aufgenommen und mit konz. Ammoniak versetzt. Man rührt für 2 Stunden bei Raumtemperatur und engt den Ansatz am Rotationsverdampfer ein. Man versetzt mit Wasser und extrahiert mit Essigester (2x). Die vereinten organischen Phasen werden getrocknet (MgSO₄), filtriert und eingeengt. Der verbleibende Rückstand wird chromatographisch (Hexan / Essigester 1 : 1) gereinigt. Man erhält 190 mg (0,40 mmol, entsprechend 40 % der Theorie) des Produktes A und 110 mg (0,23 mmol, entsprechend 23 % der Theorie) des Produktes B.
2-[4-(5-Brom-2-chlor-pyrimidin-4-ylamino)-butoxy]-4-nitro-benzolsulfonamid (A): ¹H-NMR (DMSO): 8.25 (s, 1H), 7.99 (d, 1H), 7.91 (m, 2H), 7.72 (br, 1H), 7.35 (br, 2H), 4.34 (m, 2H), 3.42 (m, 2H), 1.82 (m, 4H).
MS : 480 (ES).

4-[4-(5-Brom-2-chlor-pyrimidin-4-ylamino)-butoxy]-2-nitro-benzolsulfonamid (B):
¹H-NMR (DMSO): 8.22 (s, 1H), 7.93 (d, 1H), 7.78 (t, 1H), 7.67 (s, 2H), 7.51 (d, 1H), 7.34 (dd, 1H), 4.16 (m, 2H), 3.45 (m, 2H), 1.73 (m, 4H).
MS : 480 (ES).

### 8b) Herstellung von 4-Amino-2-[4-(5-brom-2-chlor-pyrimidin-4-ylamino)-butoxy]-benzolsulfonamid

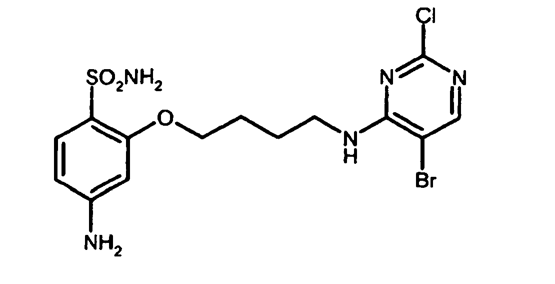

Eine Lösung von 160 mg (0,33 mmol) 2-[4-(5-Brom-2-chlor-pyrimidin-4-ylamino)-butoxy]-4-nitro-benzolsulfonamid in 10 ml THF wird unter Argon bei Raumtemperatur mit 1,4 ml einer 15%igen Lösung von Ti(III)CI in etwa 10%iger Salzsäure versetzt. Nach 4 Stunden wird die Reaktionslösung erneut mit 0,2 ml der Ti(III)Cl-Lösung versetzt und weitere 14 Stunden gerührt. Der Ansatz wird mit 2N NaOH Lösung basisch gestellt und anschließend filtriert. Der Filterkuchen wird 2x mit jeweils 50 ml Essigester / MeOH (30 ml / 20 ml) nachgewaschen. Das Filtrat wird am Rotationsverdampfer eingeengt und danach mit Essigester extrahiert (2x). Die vereinten organischen Phasen werden mit NaCl Lösung gewaschen, getrocknet (MgSO₄), filtriert und eingeengt. Der verbleibende Rückstand wird chromatographisch gereinigt (Dichlormethan / Methanol 9 : 1; Flashmaster II). Man erhält 70 mg (0,81 mmol, entsprechend 47 % der Theorie) des Produktes.
¹H-NMR (DMSO): 8.22 (s, 1H), 7.73 (t, 1H), 7.36 (d, 1H), 6.44 (s, 2H), 6.27 (d, 1H), 6.13 (dd, 1H), 5.78 (s, 2H), 4.04 (m, 2H), 3.45 (m, 2H), 1.76 (m, 4H).
MS: 450 (ES).

### Beispiel 8.1

### Herstellung von 1⁵-Bromo-N-(dimethylaminomethylen)-4-oxa-2,9-diaza -1(2,4)-pyrimidina-3(1,3)-benzenacyclononaphane-3⁴-sulfonamide

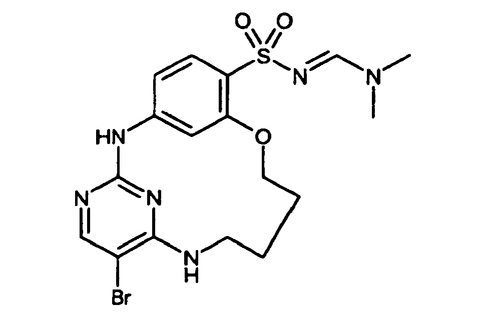

Eine Suspension von 40 mg (0.096 mmol) 1⁵-Bromo-4-oxa-2,9-diaza-1(2,4)-pyrimidina-3(1,3)-benzenacyclononaphan-3⁴-sulfonamide in 1 ml DMF wird bei Raumtemperatur mit 0.02 ml N,N-Dimethylformamiddimethylacetal versetzt und über Nacht gerührt. Das Lösungsmittel wird abgezogen und der verbleibende Rückstand mit MTB-Ether digeriert. Man erhält 40 mg (0.085, entsprechend 88 % der Theorie) des Produktes.
¹H-NMR (DMSO): 9.70 (s, 1H), 8.98 (m, 1H), 8.17 (s, 1H), 8.04 (s, 1H), 7.53 (m, 1H), 7.45 (t, 1H), 6.69 (m, 1H), 4.35 (m, 2H), 3.35 (m, 2H), 3.18 (s, 3H), 2.88 (s, 3H), 1.80 (m, 4H).
MS: 469 (ES).

### Herstellung der Sulfonamid-Oxa-Cyclophane nach Vervahrensvariante 8b)

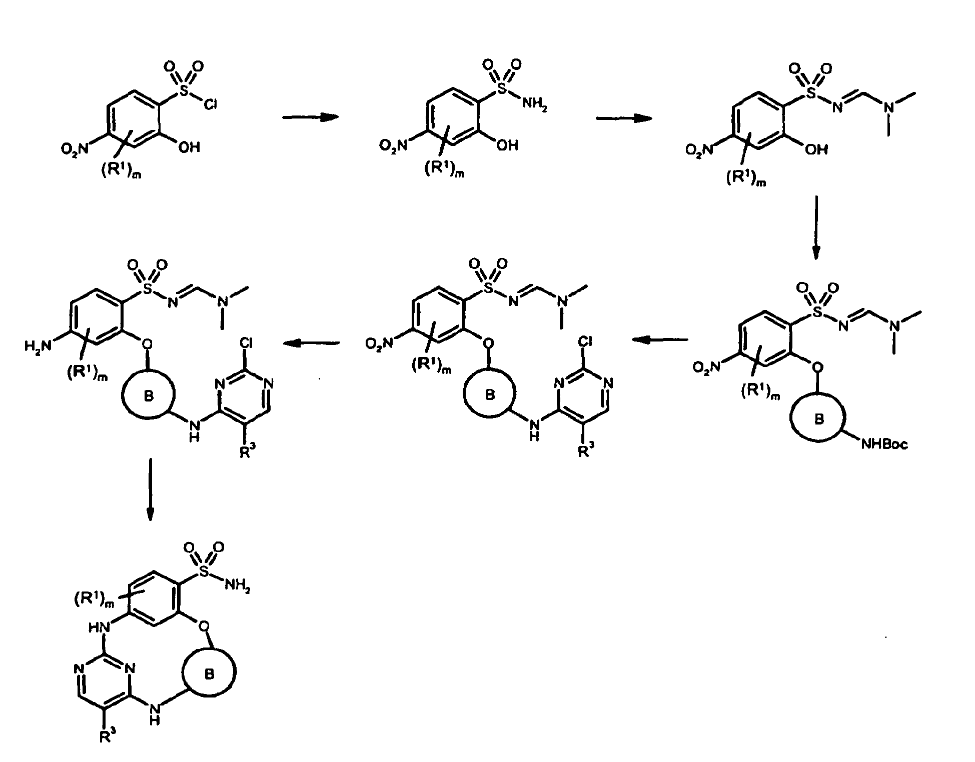

In den Formeln haben R¹, R³, B und m die in den Verbindungen der Ansprüche 1 bis 3 verwirklichten Bedeutungen.

### Beispiel 8.2

### Herstellung von (S)-1⁵-Bromo-8-(hydroxymethyl)-4-oxa-2,8-diaza-1(2,4)-pyrimidina-3(1,3)-benzenacyclononaphane-3(4)-sulfonamide

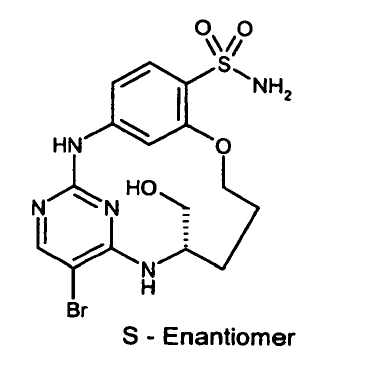

Eine Lösung von 90 mg (0,17 mmol) (S)-4-Amino-2-[4-(5-brom-2-chlor-pyrimidin-4-ylamino)-5-hydroxy-pentyloxy]-*N*-dimethylaminomethylen-benzolsulfonamid in Acetonitril / MeOH / Wasser (8 ml / 2 ml / 1 ml) wird mittels Spritzenpumpe innerhalb von 2,5 Stunden zu einer refluxierenden Lösung von Acetonitril / Wasser / 4 molare Lösung von Chlorwasserstoff in Dioxan (45 ml / 5 ml / 0,6 ml) gegeben. Nach weiteren 16 Stunden unter Rückfluss wird das Reaktionsgemisch am Rotationsverdampfer eingeengt. Der Ansatz wird mit gesättigter NaHCO₃ Lösung basisch gestellt und mit Essigester extrahiert (2x). Die vereinten organischen Phasen werden getrocknet (Na₂SO₄), filtriert und eingeengt. Der verbleibende Rückstand wird mit *tert*-Butylmethylether digeriert. Man erhält 62 mg (0,14 mmol, entsprechend 83% der Theorie) des S - Enantiomerproduktes.
¹H-NMR (DMSO): 9.79 (s, 1H), 8.53 (br, 1H), 8.11 (s, 1H), 7.52 (m, 1H), 6.83 (s, 2H), 6.68 (m, 1H), 6.43 (d, 1H), 4.98 (t, 1H), 4.60 (m, 1H), 4.12 (m, 1H), 3.81 (m, 1H), 3.62 (m, 2H), 2.18 (m, 1H), 2.02 (m, 1H), 1.64 (m, 2H).
MS : 444 (ES).

Das R-Enantiomers kann analog den obigen Vorschriften hergestellt werden, wobei man als Ausgangsprodukt (R)-4-Amino-2-[4-(5-brom-2-chlor-pyrimidin-4-ylamino)-5-hydroxy-pentyloxy]-*N*-dimethylaminomethylen-benzolsulfonamid verwenden sollte.

### Herstellung der Zwischenprodukte nach Verfahrensvariante 8b)

### 8c) Herstellung von N-tert-Butyl-2-methoxy-4-nitro-benzolsulfonamid

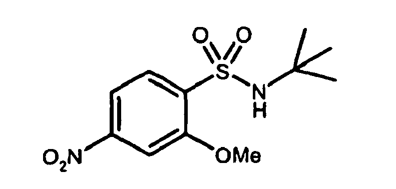

Eine Lösung von 5.0 g( 19.9 mmol) 2-Methoxy-4-nitro-benzolsulfonylchlorid in Aceton / Wasser (60 ml / 15 ml) wird bei Raumtemperatur mit 2.9 ml Triethylamin und 2.2 ml *tert*.-Butylamin versetzt. Nach 5 h wird das Aceton abgezogen und der Rückstand gegen Essigester extrahiert. Die vereinten organischen Phasen werden mit verdünnter HCl Lösung und gesättigter NaCl Lösung gewaschen und anschließend über einem Whatman Filter filtriert und eingeengt. Das erhaltene Rohprodukt wird aus Essigester umkristallisiert. Man erhält 4.2 g (14.6 mmol, entsprechend 73 % der Theorie) des Produktes.
¹H-NMR (DMSO): 8.03 (m, 1H), 7.95 (m, 2H), 7.48 (s, 1H), 4.05 (s, 3H), 1.08 (s, 9H).

### 8d) Herstellung von 2-Methoxy-4-nitro-benzolsulfonamid

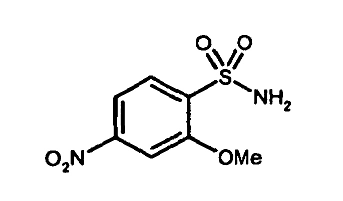

8.1 g (28.1 mmol) *N*-*tert*-Butyl-2-methoxy-4-nitro-benzolsulfonamid werden mit 350 ml Trifluoressigsäure versetzt und 20 h bei Raumtemperatur gerüht. Die Trifluoressigsäure wird abgezogen und der verbliebene Rückstand anschließend mit Essigester digeriert. Man erhält 5,0 g (21.6 mmol, entsprechend 77 % der Theorie) des Produktes. Die Essigesterphase wird eingeengt und der erhaltene Rückstand chromatographisch (DCM / EtOH 95 : 5, Flashmaster II) gereinigt. Man erhält weitere 0.84 g (3.6 mmol, entsprechend 13 % der Theorie) des Produktes.
¹H-NMR (DMSO): 7.95 (m, 3H), 7.45 (s, 2H), 4.03 (s, 3H). MS :233 (ES).

### 8e) Herstellung von N-Dimethylaminomethylen-2-methoxy-4-nitro-benzolsulfonamid

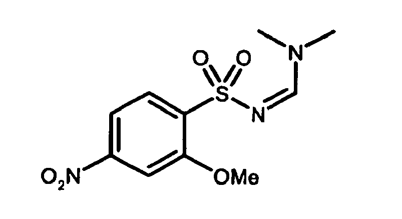

Eine Lösung von 5.0 g (21.5 mmol) 2-Methoxy-4-nitro-benzolsulfonamid in 15 ml DMF wird bei Raumtemperatur mit 3.5 ml N,N-Dimethylformamiddimethylacetal versetzt und 2.5 h gerührt. Der Ansatz wird auf eine 5%ige KHSO₄ Lösung in Eiswasser gegeben und anschließend gegen Essigester extrahiert. Die vereinten organischen Phasen werden getrocknet (MgSO₄), filtriert und eingeengt. Das erhaltene Rohprodukt wird mit Essigester digeriert. Man erhält 5.6 g (19.4 mmol, entsprechend 90 % der Theorie) des Produktes.
¹H-NMR (DMSO): 8.29 (s, 1H), 8.04 (m, 1H), 7.90 (m, 2H), 3.99 (s, 3H), 3.24 (s, 3H), 2.93 (s, 3H). MS :288 (ES).

### 8f) Herstellung von N-Dimethylaminomethylen-2-hydroxy-4-nitro-benzolsulfonamid

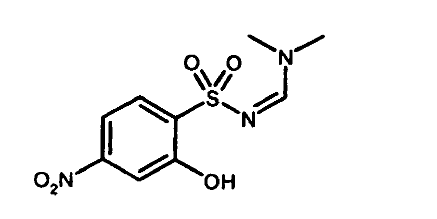

Eine Lösung von 2.82 g (9.8 mmol) *N*-Dimethylaminomethylen-2-methoxy-4-nitro-benzolsulfonamid in 70 ml DCM wird bei Raumtemperatur langsam mit 13 ml einer 1 molaren Lösung von Bortribromid in DCM versetzt. Nach 5 h werden erneut 3 ml der 1 molaren Lösung von Bortribromid in DCM zugegeben und weitere 16 h gerührt. Der Ansatz wird mit MeOH und Diisopropylether versetzt. Der gebildete Niederschlag wird abgesaugt, mit EtOH und Diisopropylether gewaschen und getrocknet. Man erhält 1.94 g (7.1 mmol, entsprechend 72 % der Theorie) des Produktes.
¹H-NMR (DMSO): 11.55 (s, 1H), 8.21 (s, 1H), 7.95 (m, 1H), 7.70 (m, 2H), 3.19 (s, 3H), 2.91 (s, 3H). MS : 274 (ES).

### 8g) Herstellung von (S)- {4-[2-(Dimethylaminomethylen-sulfamoyl)-5-nitro-phenoxy]-1-hydroxymethyl-butyl}-carbaminsäure-tert-butylester

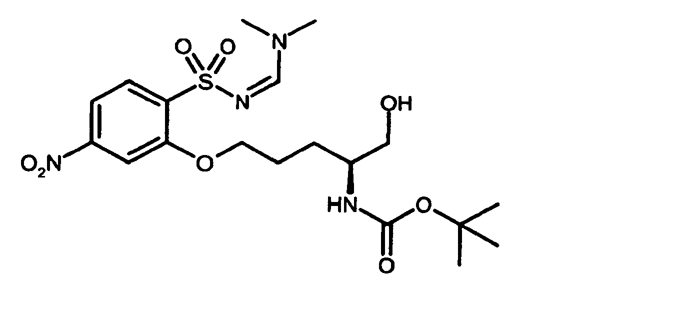

Zu einer Reaktionsmischung von 1.57 g (5.75 mmol) *N*-Dimethylaminomethylen-2-hydroxy-4-nitro-benzolsulfonamid, 1.26 g (5.75 mmol) (S)-2-Boc-amino-pentan-diol und 1.80 g (6.9 mmol) Triphenylphosphin in 30 ml THF wird bei 0 °C eine Lösung von 1.20 g (6.9 mmol) DEAD in 10 ml THF getropft. Nach 24 h werden zunächst weitere 0.20 g (1.1 mmol) DEAD zugegeben. Nach 5 h wird auch erneut mit 0.28 g (1.1 mmol) Triphenylphosphin versetzt und 68 h gerührt. Abschließend werden 0.2 g (1.1 mmol) DEAD zugegeben und weitere 22 h gerührt. Der Ansatz wird eingeengt und der Rückstand chromatographisch (DCM / EtOH 95 : 5, Flashmaster II) gereinigt. Man erhält 0.71 g (1.50 mmol, entsprechend 26 % der Theorie) des Produktes.
¹H-NMR (DMSO): 8.21 (s, 1H), 8.03 (m, 1H), 7.88 (m, 2H), 6.60 (d, 1H), 4.65 (t, 1H), 4.19 (m, 2H), 3.42 (m, 3H), 3.22 (s, 3H), 2.93 (s, 3H), 1.73 (m, 4H), 1.39 (s, 9H).
MS : 475 (ES).

### 8h) Herstellung von 2-[4-(5-Brom-2-chlor-pyrimidin-4-ylamino)-5-hydroxy-pentyloxy]-N-dimethylaminomethylen-4-nitro-benzolsulfonamid

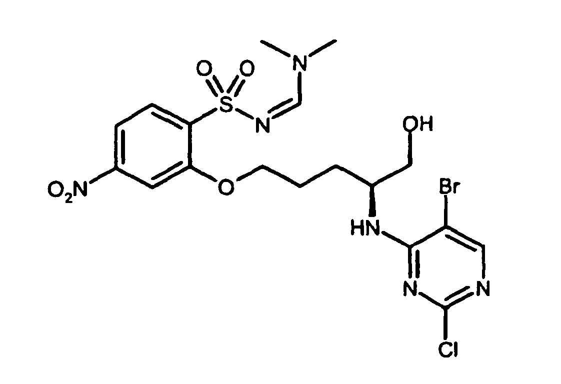

Eine Lösung von 212 mg (0.45 mmol) (S)- {4-[2-(Dimethylaminomethylen-sulfamoyl)-5-nitro-phenoxy]-1-hydroxymethyl-butyl}-carbaminsäure-*tert*-butylester in 5 ml Acetonitril wird bei Raumtemperatur mit 0.75 ml einer 4 molaren Lösung von Chlorwasserstoff in Dioxan versetzt: Nach 4 h wird der Ansatz eingeengt und man erhält 2-(4-Amino-5-hydroxy-pentyloxy)-*N*-dimethylaminomethylen-4-nitro-benzolsulfonamid in Form des Hydrochlorides.
Eine Lösung des erhaltenen Produktes in 4 ml Acetonitril wird anschließend bei Raumtemperatur mit 110 mg (0.48 mmol) 5-Brom-2,4-dichlor-pyrimidin in 4 ml Acetonitril versetzt. Man gibt 0.13 ml Triethylamin hinzu und rührt über Nacht. Der Ansatz wird eingeengt und der Rückstand chromatographisch (DCM / EtOH 95 : 5, Flashmaster II) gereinigt. Man erhält 152 mg (0.27 mmol, entsprechend 60 % der Theorie) des Produktes.
¹H-NMR (DMSO): 8.28 (s, 1H), 8.19 (s, 1H), 8.04 (m, 1H), 7.89 (m, 2H), 7.18 (d, 1H), 4.89 (t, 1H), 4.21 (m, 3H), 3.51 (m, 2H), 3.19 (s, 3H), 2.89 (s, 3H), 1.78 (m, 4H). MS : 565 (ES).

### 8i) Herstellung von (S)-4-Amino-2-[4-(5-brom-2-chlor-pyrimidin-4-ylamino)-5-hydroxy-pentyloxy]-N-dimethylaminomethylen-benzolsulfonamid

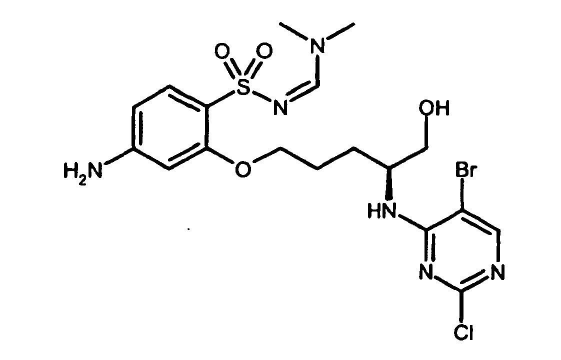

Eine Lösung von 145 mg (0,30 mmol) 2-[4-(5-Brom-2-chlor-pyrimidin-4-ylamoni)-5-hydroxy-pentyloxy]-*N*-dimethylaminomethylen-4-nitro-benzolsulfonamid in 20 ml THF wird unter Argon bei Raumtemperatur mit 2,0 ml einer ca. 10%igen Lösung von Ti(III)Cl in 20-30%iger Salzsäure versetzt. Nach 2 Stunden wird die Reaktionslösung erneut mit 0,3 ml der Ti(III)Cl-Lösung versetzt und weitere 18 Stunden gerührt. Der Ansatz wird mit Essigester verdünnt und mit 1 N NaOH Lösung basisch gestellt. Die Phasen werden getrennt und die wässrige Phase wird erneut gegen Essigester extrahiert. Die vereinten organischen Phasen werden getrocknet (Na₂SO₄), filtriert und eingeengt. Der verbleibende Rückstand wird chromatographisch gereinigt (DCM / Methanol 9 : 1; Flashmaster II). Man erhält 90 mg (0,17 mmol, entsprechend 56 % der Theorie) des Produktes.
¹H-NMR (DMSO): 8.28 (s, 1H), 8.06 (s, 1H), 7.39 (m, 1H), 7.17 (d, 1H), 6.11 (m, 2H), 5.79 (s, 2H), 4.89 (t, 1H), 4.20 (m, 1H), 3.88 (m, 2H), 3.51 (m, 2H), 3.11 (s, 3H), 2.85 (s, 3H), 1.75 (m, 4H).
MS : 535 (ES).

### Verfahrensvariante 8c

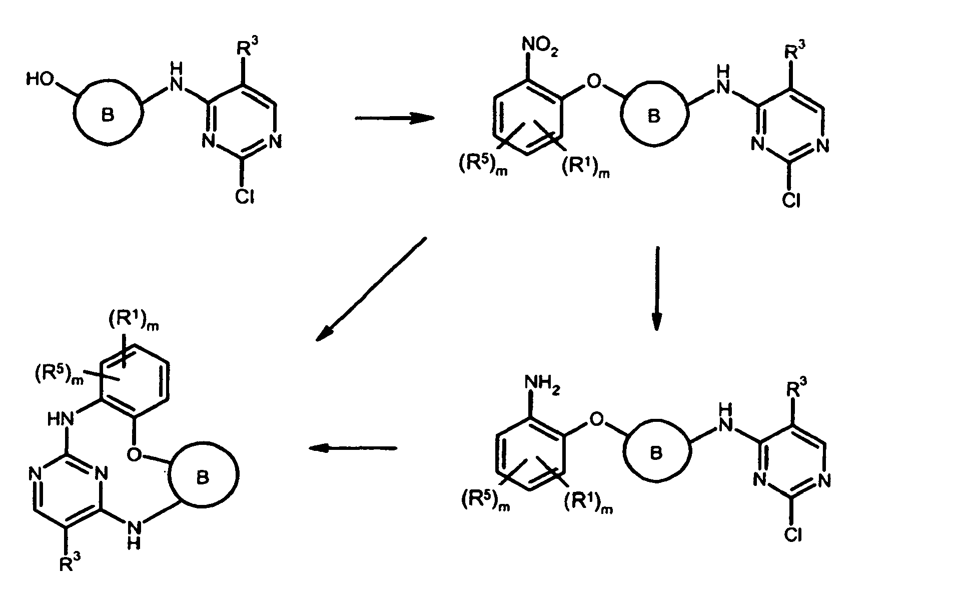

In den Formeln haben R¹, R³, R⁵, B und m die in den Verbindungen der Ansprüche 1 bis 3 verwirklichten Bedeutungen .

### Beispiel 8.3

### Herstellung von 1⁵-Bromo-4-oxa-2,8-diaza-1(2,4)-pyrimidina-3(1,2)-benzenacyclooctaphane

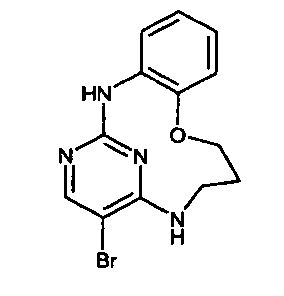

Eine Lösung von 145 mg (0,41 mmol) [3-(2-Amino-phenoxy)-propyl]-(5-brom-2-chlor-pyrimidin-4-yl)-amin in Acetonitril (10 ml) wird mittels Spritzenpumpe innerhalb von 3 Stunden zu einer refluxierenden Lösung von Acetonitril / Wasser / 4 molare Lösung von Chlorwasserstoff in Dioxan (45 ml / 5 ml / 0.6 ml) gegeben. Nach Beendigung der Zugabe wird die Reaktionslösung weitere 16 Stunden unter Rückfluss gerührt.

Der Ansatz wird eingeengt und der Rückstand chromatographisch (DCM / EtOH 95 : 5; Flashmaster II) gereinigt. Man erhält 81 mg (0,25 mmol, entsprechend 61 % der Theorie) des Produktes.
¹H-NMR (DMSO): 9.21 (s, 1H), 8.02 (s, 1H), 7.78 (br, 1H), 7.07 (m, 3H), 6.86 (m, 1H), 4.22 (m, 2H), 3.30 (m, 2H), 1.79 (m, 2H).
MS: 321 (ES).

### Herstellung der Zwischenprodukte nach Verfahrensvariante 8c

### 8j) Herstellung von (5-Brom-2-chlor-pyrimidin-4-yl)-[3-(2-nitro-phenoxy)-propyl]-amin

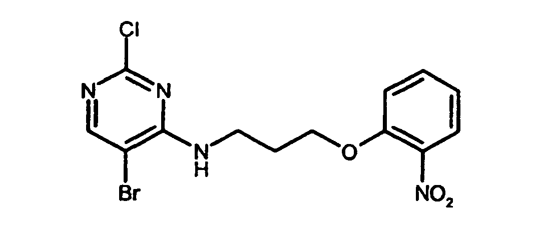

Eine Lösung von 1.06 g (4,0 mmol) 3-(5-Brom-2-chlor-pyrimidin-4-ylamino)-propan-1-ol, 0.65 g (4.8 mmol) 2-Nitro-phenol und 1.25 g (4.8 mmol) Triphenylphosphin in 30 ml THF wird unter Argon bei 0 °C mit 0.8 ml DEAD versetzt. Das Reaktionsgemisch wird unter Rühren auf Raumtemperatur erwärmt. Nach 20 h wird der Ansatz einrotiert und der Rückstand chromatographisch (Hexan / Essigester 3 : 1, Flashmaster II) gereinigt. Man erhält 1.15 g (3,0 mmol, entsprechend 74 % der Theorie) des Produktes.
¹H-NMR (DMSO): 8.22 (s, 1H), 7.88 (m, 2H), 7.63 (m, 1H), 7.33 (m, 1H), 7.09 (m, 1H), 4.21 (m, 2H), 3.54 (m, 2H), 2.03 (m, 2H).
MS: 387 (ES).

### 8k) Herstellung von [3-(2-Amino-phenoxy)-propyl]-(5-brom-2-chlor-pyrimidin-4-yl)-amin

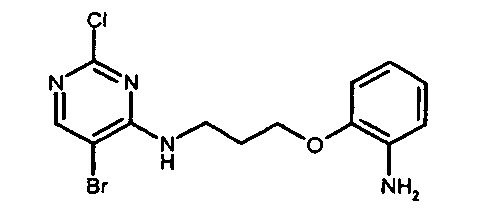

Eine Lösung von 500 mg (1.29 mmol) (5-Brom-2-chlor-pyrimidin-4-yl)-[3-(2-nitro-phenoxy)-propyl]-amin in 20 ml THF wird bei Raumtemperatur mit 6.0 ml einer ca. 10 %igen Lösung von Ti(III)Cl in 20-30 %iger Salzsäure versetzt. Nach 21 Stunden werden weitere 2,0 ml der Ti(III)Cl Lösung zugegeben. Erneute Zugaben der Ti(III)Cl Lösung erfolgen nach 4 Stunden (3,0 ml) bzw. 16 Stunden (4,0 ml). Nach weiteren 6 Stunden wird der Ansatz mit Essigester verdünnt und mit 1 N NaOH Lösung basisch gestellt. Man filtriert über Celite und wäscht den Filterkuchen mit Essigester. Die organische Phase wird abgetrennt, getrocknet (Na₂SO₄), filtriert und eingeengt. Der erhaltene Rückstand wird chromatographisch (Hexan / Essigester 7 : 3) gereinigt. Man erhält 290 mg (0.81 mmol, entsprechend 62 % der Theorie) des Produktes.
¹H-NMR (DMSO): 8.25 (s, 1H), 7.83 (t, 1H), 6.78 (m, 1H), 6.63 (m, 2H), 6.49 (m, 1H), 4.69 (s, 2H), 3.96 (m, 2H), 3.58 (m, 2H), 2.03 (m, 2H).
MS: 357 (ES).

### Beispiel 8.4

### Herstellung von 1⁵-Bromo-4-oxa-2,8-diaza-1(2,4)-pyrimidina-3(1,2)-benzenacyclooctaphane-3⁴-sulfonamide

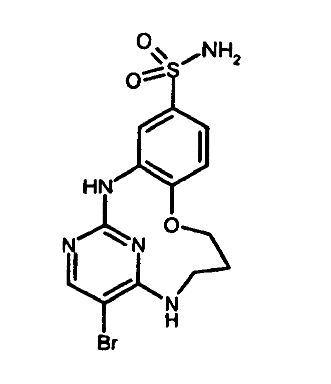

Eine Lösung von 200 mg (0.42 mmol) 4-[3-(5-Brom-2-chlor-pyrimidin-4-ylamino)-propoxy]-3-nitro-benzolsulfonamid in 14 ml THF wird mit 3 ml einer ca. 10 %igen Lösung von Ti(III)Cl in 20-30 %iger Salzsäure versetzt und 19 Stunden bei Raumtemperatur gerührt. Nach DC Kontrolle werden im Verlauf von 184 Stunden insgesamt weitere 9 ml der Ti(III)Cl Lösung portionsweise zugegeben. Der Ansatz wird mit 2 N NaOH Lösung basisch gestellt und gegen Essigester extrahiert. Die vereinten organischen Phasen werden getrocknet (Na₂SO₄), filtriert und eingeengt. Der erhaltene Rückstand wird chromatographisch (DCM / EtOH 95:5) gereinigt. Man erhält 62 mg (0.14 mmol, entsprechend 33 % der Theorie) des Produktes.
Das erhaltene Produkt wird in 5 ml Acetonitril gelöst und mittels Spritzenpumpe innerhalb von 2 Stunden zu einer refluxierenden Lösung von Acetonitril / Wasser / 4 molare Lösung von Chlorwasserstoff in Dioxan (30 ml / 3 ml / 0.4 ml) gegeben. Anschließend wird der Ansatz weitere 16 Stunden unter Rückfluss gerührt. Nach dem Erkalten wird der gebildete Niederschlag abgesaugt und mit Acetonitril und Wasser gewaschen. Man erhält 13 mg (0.03 mmol, entsprechend 8 % der Theorie) des Produktes.
¹H-NMR (DMSO): 10.53 (s, 1H), 8.97 (m, 1H), 8.25 (s, 1H), 7.67 (m, 2H), 7.30 (m, 3H), 4.31 (m, 2H), 3.35 (m, 2H), 1.88 (m, 2H).
MS: 400 (ES).

### Herstellung der Zwischenprodukte

### 8l) 4-[3-(5-Brom-2-chlor-pyrimidin-4-ylamino)-propoxy]-3-nitro-benzolsulfonamid

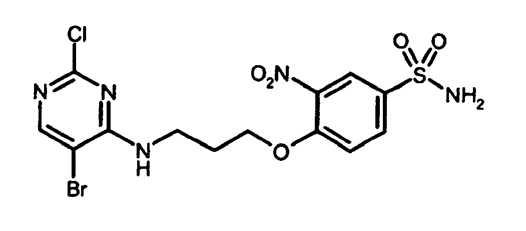

398 mg (1.02 mmol) (5-Brom-2-chlor-pyrimidin-4-yl)-[3-(2-nitro-phenoxy)-propyl]-amin werden portionsweise auf 4 ml eisgekühlte Chlorsulfonsäure gegeben. Der Ansatz wird 2.5 Stunden gerührt und das Reaktionsgemisch anschließend vorsichtig auf Eis getropft. Der gebildete Feststoff wird abgesaugt, mit Wasser gewaschen und getrocknet. Das erhaltene Rohprodukt wird in 20 ml Aceton gelöst, mit 3 ml Ammoniak (33%) versetzt und eine Stunde bei Raumtemperatur gerührt. Der Ansatz wird eingeengt, mit Essigester versetzt und mit Wasser gewaschen. Die vereinten organischen Phasen werden über einem Whatman Filter filtriert und eingeengt. Man erhält 223 mg (0.48 mmol, entsprechend 47 % der Theorie) des Produktes.
¹H-NMR (DMSO): 8.29 (m, 1H), 8.24 (s, 1H), 8.02 (m, 1H), 7.85 (t, 1H), 7.47 (m, 3H), 4.29 (t, 2H), 3.55 (m, 2H), 2.04 (m, 2H).
MS: 466 (ES).

### Herstellung der Amidderivate

### Verfahrensvariante 9

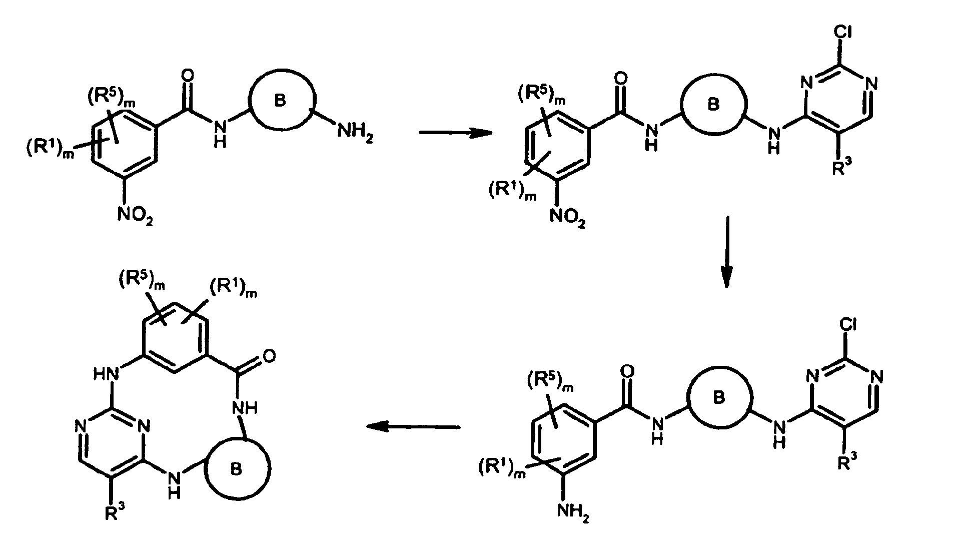

In den Formeln haben R¹, R³, R⁵, B und m die in den Verbindungen der Ansprüche 1 bis 3 verwirklichten Bedeutungen.

### Beispiel 9.0

### Herstellung von 1⁵-Bromo-2,5,10-triaza-1(2,4)-pyrimidina-3(1,3)-benzenacyclodecaphan-4-one

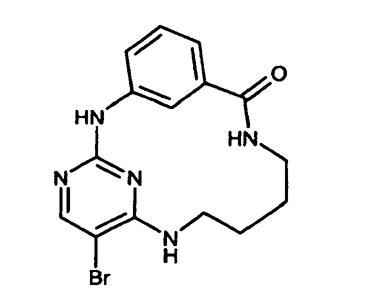

Eine Lösung von 440 mg (1.1 mmol) 3-Amino-*N*-[4-(5-brom-2-chlor-pyrimidin-4-ylamino)-butyl]-benzamid in Acetonitril / DMF / Wasser (25 ml / 5 ml / 5 ml) wird über einen Tropftrichter innerhalb von 2 Stunden zu einer refluxierenden Lösung von Acetonitril / Wasser / 4 molare Lösung von Chlorwasserstoff in Dioxan (150 ml / 10 ml / 2 ml) gegeben. Nach weiteren 3 Stunden unter Rückfluss wird der Ansatz aus dem Ölbad genommen. Der nach dem Erkalten gebildete Niederschlag wird abgesaugt und mit Wasser und Diisopropylether gewaschen. Man erhält 38 mg (0,10 mmol, entsprechend 9% der Theorie) des Produktes
¹H-NMR (DMSO):10.45 (s, 1H), 8.40 (m, 3H), 8.25 (s, 1H), 7.40 (m, 3H), 3.35 (m, 2H), 3.10 (m, 2H), 1.48 (m, 4H).
MS: 363 (ES).

### Herstellung der Zwischenprodukte nach Verfahrensvariante 9

### 9a) Herstellung von N-(4-Amino-butyl)-3-nitro-benzamid

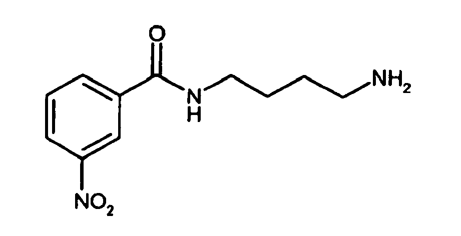

2.27 g (6.72 mmol) [4-(3-Nitro-benzoylamino)-butyl]-carbaminsäure-*tert*-butylester werden mit 9 ml Trifluoressigsäure versetzt und 3 h bei Raumtemperatur gerührt. Der Ansatz wird vorsichtig auf 2N NaOH Lösung gegeben und anschließend gegen Essigester extrahiert. Die vereinten organischen Phasen werden mittels eines Whatman Filters filtriert und eingeengt. Das erhaltene Rohprodukt wird aus Ethanol / Diisopropylether umkristallisiert. Man erhält 519 mg (2.19 mmol, entsprechend 33% der Theorie) des Produktes.
¹H-NMR (DMSO): 8.95 (m, 1H), 8.65 (s, 1H), 8.86 (m, 1H), 8.78 (m, 1H), 7.72 (m, 1H), 3.30 (m, 2H), 3.04 (m, 2H), 1.55 (m, 2H), 1.40 (m, 2H). MS: 238 (ES).

### 9b) Herstellung von N-[4-(5-Brom-2-chlor-pyrimidin-4-ylamino)-butyl]-3-nitro-benzamid

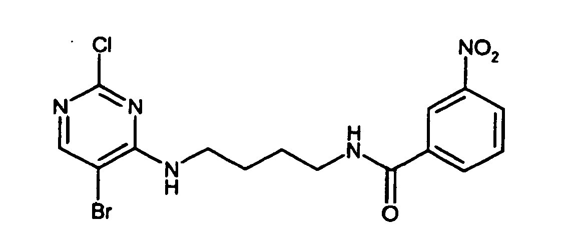

Eine Lösung 502 mg (2.2 mmol) 5-Brom-2,4-dichlor-pyrimidin und 0,4 ml Triethylamin (2.3 mmol) in 2.5 ml Acetonitril wird bei 0 °C unter Rühren mit einer 547 mg (2.3 mmol) *N*-(4-Amino-butyl)-3-nitro-benzamid versetzt. Der Ansatz wird über Nacht bei Raumtemperatur gerührt. Der gebildete Niederschlag wird abgesaugt, mit Wasser gewaschen und getrocknet. Man erhält 574 mg (1.3 mmol, entsprechend 61 % der Theorie) des Produktes
¹H-NMR (DMSO): 8.87 (t, 1H), 8.65 (m, 1H), 8.38 (m, 1H), 8.28 (m, 1H), 8.18 (s, 1H), 7.76 (m, 2H), 3.35 (m, 4H), 1.55 (m, 4H). MS: 427 (EI).

### 9c) Herstellung von 3-Amino-N-[4-(5-brom-2-chlor-pyrimidin-4-ylamino)-butyl]-benzamid

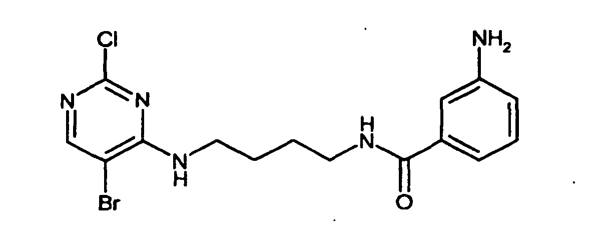

Eine Lösung von 568 mg (1.32 mmol) *N*-[4-(5-Brom-2-chlor-pyrimidin-4-ylamino)-butyl]-3-nitro-benzamid in 15 ml THF wird bei Raumtemperatur mit 9 ml einer 15%igen Lösung von Ti(III)CI in etwa 10%iger Salzsäure versetzt. Nach 21 Stunden wird der Ansatz wird mit 2N NaOH Lösung basisch gestellt, mit Essigester versetzt und über Celite filtriert. Der Filterkuchen mit Essigester nachgewaschen. Die organische Phase des Filtrats wird mittels Whatman Filter filtriert und eingeengt. Man erhält 447 mg (1.12 mmol, entsprechend 85 % der Theorie) des Produktes.
MS: 398 (ES).

### Herstellung der Harnstoffderivate

### Verfahrensvariante 10

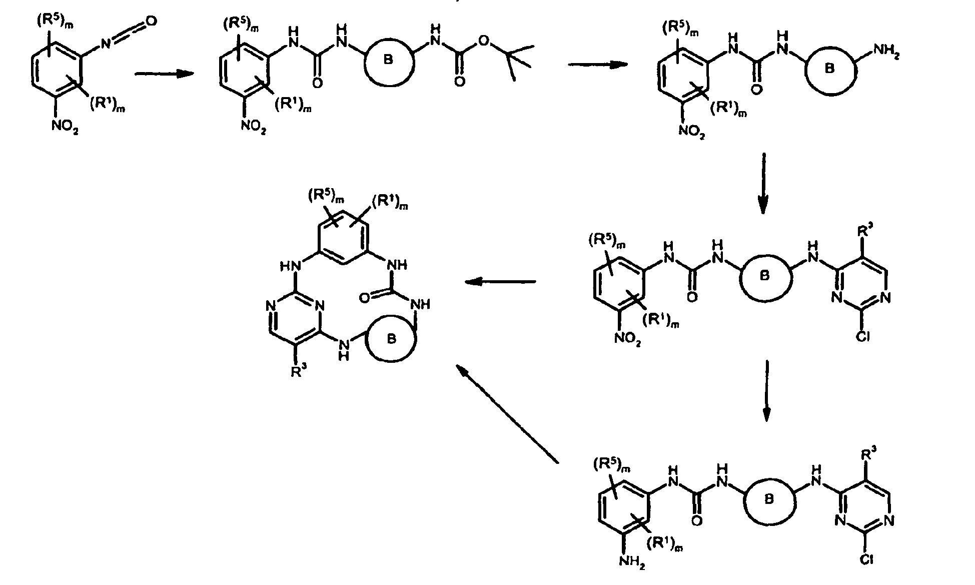

In den Formeln haben R¹, R³, R⁵, B und m die in den Verbindungen der Ansprüche 1 bis 3 verwirklichten Bedeutungen .

### Beispiel 10.0

### Herstellung von 1⁵-Bromo-2,4,6,10-tetraaza-1(2,4)-pyrimidina-3(1,3)-benzenacyclodecaphan-5-one

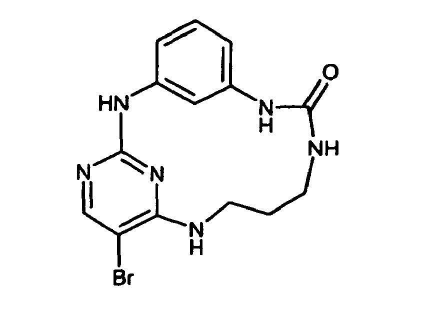

Eine Lösung von 265 mg (0.66 mmol) 1-(3-Amino-phenyl)-3-[3-(5-brom-2-chlor-pyrimidin-4-ylamino)-propyl]-hamstoff in Acetonitril / Dioxan / Wasser (8 ml / 1 ml / 1 ml) wird mittels einer Spritzenpumpe innerhalb von 2 Stunden zu einer refluxierenden Lösung von Acetonitril / Wasser / 4 molare Lösung von Chlorwasserstoff in Dioxan (70 ml / 5 ml / 1 ml) gegeben. Nach weiteren 18 Stunden unter Rückfluss wird der Ansatz nach dem Erkalten mit 2N NaOH basisch gestellt und gegen Essigester extrahiert. Die vereinten organischen Phasen werden über einem Whatman Filter filtriert und eingeengt. Der erhaltene Rückstand wird chromatographisch (DCM / EtOH 9 : 1) gereinigt. Das erhaltene Rohprodukt wird anschließend aus MeOH umkristallisiert. Man erhält 7 mg (0.02 mmol, entsprechend 3% der Theorie) des Produktes.
¹H-NMR (DMSO): 9.31 (s, 1H), 8.79 (m, 1H), 8.17 (s, 1H), 7.98 (s, 1H), 7.27 (t, 1H), 7.05 (t, 1H), 6.72 (m, 1H), 651 (m, 1H), 6.43 (m, 1H), 3.48 (m, 2H), 3.14 /m, 2H), 1.65 (m, 2H).
¹³C-NMR (DMSO): 158.6s, 157.8d, 156.1 s, 155.5s, 141.6s, 140.9s, 129.0d, 112.7d, 112.2d, 110.3d, 92.1s, 38.3t, 36.3t, 30.3t.
MS: 363 (ES).

### Herstellung der Zwischenprodukte nach Verfahrensvariante 10

### 10a) Herstellung von {3-[3-(3-Nitro-phenyl)-ureido]-Propyl}-carbaminsäure-tert-butylester

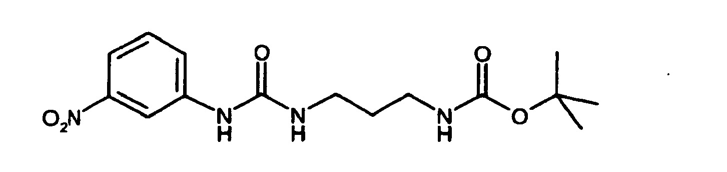

Eine Lösung von 3.35 g (19.2 mmol) *N*-Boc-1,3-diaminopropan in 50 ml EtOH wird bei 0°C Portionsweise mit 3.15 g (19.2 mmol) 3-Nitrophenylisocyanat versetzt. Der Ansatz wird bei Raumtemperatur über Nacht gerührt und anschließend am Rotationsverdampfer eingeengt. Man versetzt mit DCM und wäscht mit Wasser. Die organische Phase wird mittels Whatman Filter filtriert und eingeengt. Man erhält 6.4 g (18.9 mmol, entsprechend 98 % der Theorie) des Produktes.
¹H-NMR (DMSO): 9.11 (s, 1H), 8.48 (m, 1H), 7.71 (m, 1H), 7.65 (m, 1H), 7.48 (t, 1H), 6.82 (t, 1H), 6.32 (t, 1H), 3.12 (m, 2H), 2.95 (m, 2H), 1.55 (m, 2H), 1.38 (s, 9H).
MS: 339 (Cl).

### 10b) Herstellung von 1-(3-Amino-propyl)-3-(3-nitro-phenyl)-harnstoff

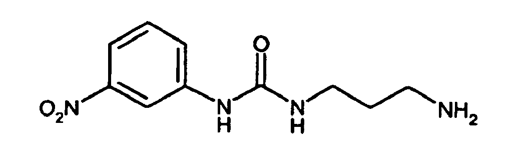

6.4 g (18,9 mmol) {3-[3-(3-Nitro-phenyl)-ureido]-propyl}-carbaminsäure-*tert*-butylester werden mit 22 ml Trifluoressigsäure versetzt und 2 h bei Raumtemperatur gerührt. Das Lösungsmittel wird abgezogen, der Ansatz mit NaHCO₃ Lösung versetzt und gegen Essigester extrahiert. Die vereinten organischen Phasen werden über einem Whatman Filter filtriert und eingeengt. Man erhält 4.4 g (18.5 mmol, entsprechend 97 % der Theorie) des Produktes.
¹H-NMR (DMSO):9.46 (s, 1H), 8.55 (m, 1H), 7.75 (m, 1H), 7.65 (m, 1H), 7.48 (m, 1H), 6.88 (t, 1H), 3.30 (m, 2H), 2.75 (m, 2H), 1.72 (m, 2H).
MS: 239 (ES).

### 10c) Herstellung von 1-[3-(5-Brom-2-chlor-pyrimidin-4-ylamino)-propyl]-3-(3-nitro-phenyl)-harnstoff

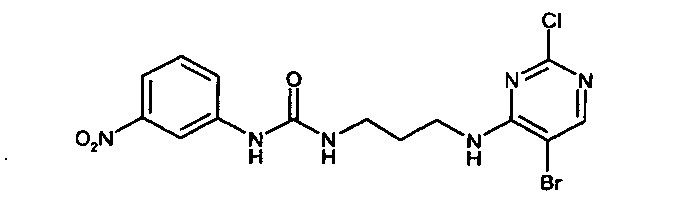

Eine Lösung von 1,6 g (6,7 mmol) 1-(3-Amino-propyl)-3-(3-nitro-phenyl)-hamstoff in 30 ml Acetonitril wird mit einer Lösung von 1,6 g (7,0 mmol) 5-Brom-2,4-dichlorpyrimidin in 10 ml Acetonitril versetzt. Man gibt 2,0 ml Triethylamin hinzu und rührt für 90 min bei Raumtemperatur. Es wird mit Essigester verdünnt (150 ml) und mit Zitronensäure (10%), gesättigter NaHCO₃ Lösung sowie gesättigter NaCl Lösung gewaschen, getrocknet (Na₂SO₄), filtriert und eingeengt. Der verbleibende Rückstand wird chromatographisch (Hexan / Essigester 1 : 1) gereinigt. Man erhält 1,7 g (4,0 mmol, entsprechend 60 % der Theorie) des Produktes.
¹H-NMR (DMSO): 9.08 (s, 1H), 8.52 (m, 1H), 8.21 (s, 1H), 7.70 (m, 3H), 7.46 (t, 1H), 6.38 (t, 1H), 3.45 (m, 2H), 3.15 (m, 2H), 1.72 (m, 2H).

### 10d) Herstellung von 1-(3-Amino-phenyl)-3-[3-(5-brom-2-chlor-pyrimidin-4-ylamino)-propyl]-harnstoff

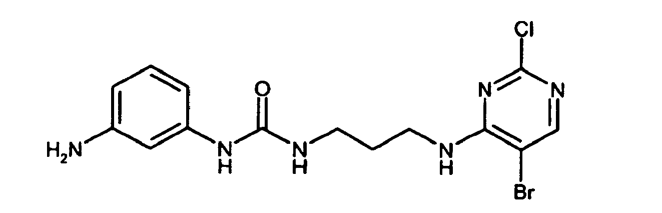

Eine Lösung von 1.71 g (3.98 mmol) 1-[3-(5-Brom-2-chlor-pyrimidin-4-ylamino)-propyl]-3-(3-nitro-phenyl)-harnstoff in 50 ml THF wird bei Raumtemperatur mit 30 ml einer 15%igen Lösung von Ti(III)Cl in etwa 10%iger Salzsäure versetzt. Nach 24 h werden weitere 5 ml der 15%igen Lösung von Ti(III)Cl in etwa 10%iger Salzsäure zugegeben. Nach weiteren 6 h wird der Ansatz wird mit 2N NaOH Lösung basisch gestellt und gegen Essigester extrahiert. Die vereinten organischen Phasen werden über einem Whatman Filter filtriert und eingeengt. Der erhaltene Rückstand wird chromatographisch (DCM / EtOH 9 : 1) gereinigt. Man erhält 850 mg (2.13 mmol, entsprechend 54% der Theorie) des Produktes.
¹H-NMR (DMSO): 8.25 (s, 1H), 8.14 (s, 1H), 7.78 (t, 1H), 6.82 (t, 1H), 6.68 (m, 1H), 6.50 (m, 1H), 6.06 (m, 2H), 4.94 (s, 1H), 3.30 (m, 2H), 3.31 (m, 2H), 1.67 (m, 2H).
MS: 399 (ES).

### Ringschluß von bifunktionellen acyclischen Vorläufern

### Verfahrensvariante 11

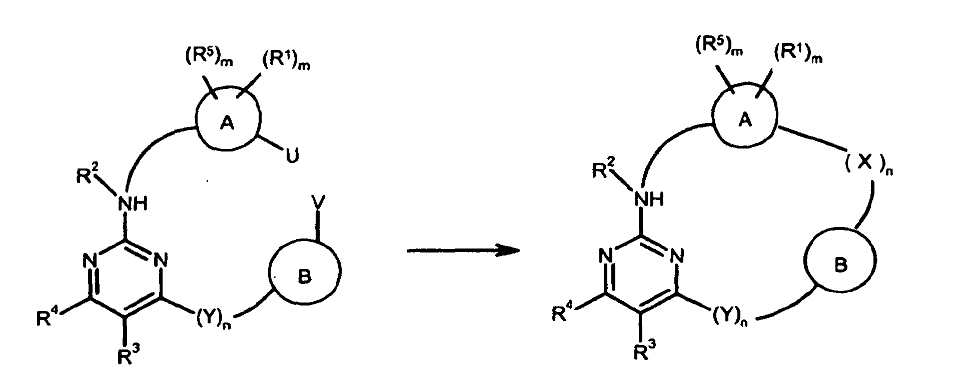

A, B, R¹, R², R³, R⁴, R⁵, X, Y, m und n haben die in den Verbindungen der Ansprüche 1 bis 3 verwirklichten Bedeutungen. U und V stehen für Gruppen wie -OH, -CO₂H, - CO₂-C1-C₆-Alkyl, -SO₂Cl, -SO₂F, -SO₃H, etc.

Der Ringschluss bzw. die Synthese der Makrozyklen kann auch analog bekannter Methoden durchgeführt werden ((a) Roxburgh, C.J. *Tetrahedron* 1995, *51*, 9767. (b) Meng, Q. *Top. Curr. Chem.* 1991, *161*, 107. (c) Paterson, I. *Tetrahedron* 1985, *41*, 3569. (d) Masamune, S. *Angew. Chem.* 1977, 89, 602. (e) Nicolaou, K.C. *Tetrahedron* 1977, 33, 683. (f) Ruggli, P. Liebigs Ann. Chem. 1912, 92.)

### Ringschluss durch Mitsunobu-Reaktion

### Verfahrensvariante 12

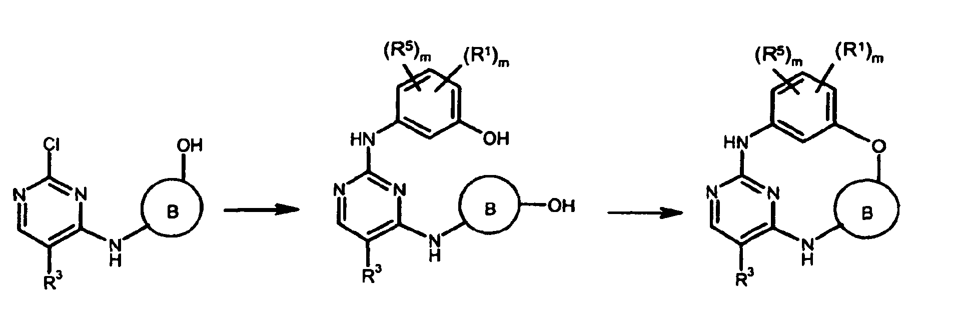

In den Formeln haben R¹, R³, R⁵, B und m die in den Verbindungen der Ansprüche 1 bis 3 verwirklichten Bedeutungen.

Synthese von Makrozyklen unter Verwendung der Mitsunobu-Reaktion ist allgemein bekannt und kann in (a) Xue, C.-B. *J*. *Med. Chem.* 2001, *44*, 2636. (b) Steglich, W. *Tet. Lett.* 1991, 32, 5781. (c) Mitsunobu, O. *Synthesis* 1981, 1 nachgelesen werden.

### Beispiel 12.0

### Herstellung von 1⁵-Bromo-4-oxa-2,9-diaza-1(2,4)-pyrimidina-3(1,3)-benzen-acyclononaphane

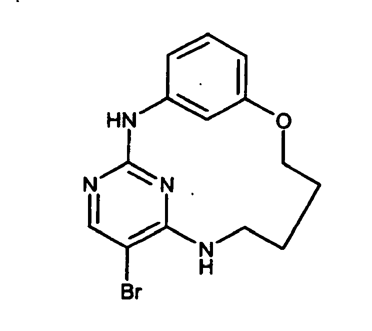

Eine Lösung von 108 mg (0,31 mmol) 3-[5-Brom-4-(4-hydroxy-butylamino)-pyrimidin-2-ylamino]-phenol in THF / *N*-Methylmorpholin (9 ml / 1ml) wird unter Rühren innerhalb von 3 Stunden zu einem Gemisch von 710 mg (2,7 mmol) Triphenylphosphin und 481 mg (2,8 mmol) DEAD in 100 ml THF bei 40°C gegeben. Nach weiteren 30 min wird das Reaktionsgemisch am Rotationsverdampfer eingeengt. Nach der Zugabe von Wasser wird mit Essigester extrahiert (2x). Die vereinten organischen Phasen werden getrocknet (Na₂SO₄), filtriert und eingeengt. Der gebildete Rückstand wird chromatographisch gereinigt (Dichlormethan / Methanol 9:1) und das erhaltene Rohprodukt anschließend mit Diisopropylether digeriert. Man erhält 17 mg (0,05 mmol, entsprechend 17% der Theorie) des Produktes.
¹H-NMR (DMSO): 9.18 (s, 1H), 9.08 (s, 1H), 8.04 (s, 1H), 7.20 (s, 1H), 7.09 (dd, 1H), 6.96 (t, 1H), 6.31 (dd, 1H), 3.30 (m, 4H), 1.90 (m, 4H).
MS: 334 (EI).

### Herstellung der Zwischenprodukte nach Verfahrensvariante 12

### 12a) Herstellung von 4-(5-Brom-2-chlor-pyrimidin-4-ylamino)-butan-1-ol

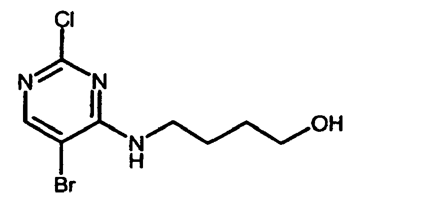

Eine Lösung von 2,28 g (10,0 mmol) 5-Brom-2,4-dichlor-pyrimidin und 1,7 ml (12,0 mmol) Triethylamin in 10 ml Acetonitril wird bei 0°C mit 1,1 ml (12,0 mmol) 4-Aminobutanol versetzt. Das Reaktionsgemisch wird durch Entfernen des Eisbades langsam unter Rühren auf Raumtemperatur erwärmt. Nach 16 Stunden wird der gebildete Niederschlag abfiltriert. Das Filtrat wird vollständig eingeengt und mit Diisopropylether digeriert. Man erhält 2,74 g (9,8 mmol, entsprechend 98% der Theorie) des Produktes.
¹H-NMR (DMSO): 8.19 (s, 1H), 7.72 (t, 1H), 4.45 (br, 1H), 3.38 (m, 4H), 1.56 (m, 2H), 1.45 (m, 2H).
MS: 279 (EI).

### 12b) 3-[5-Brom-4-(4-hydroxy-butylamino)-pyrimidin-2-ylamino]-phenol

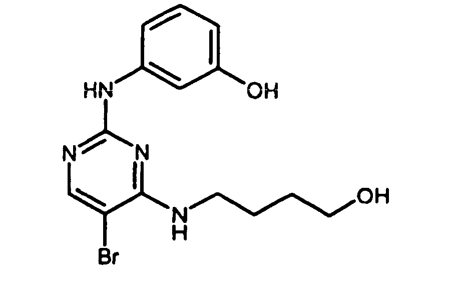

Ein Reaktionsgemisch von 327 mg (3,0 mmol) 3-Aminophenol und 864 mg (3,1 mmol) 4-(5-Brom-2-chlor-pyrimidin-4-ylamino)-butan-1-ol in 9 ml Acetonitril wird mit 0,75 ml einer 4M Lösung von Chlorwasserstoff in Dioxan versetzt und über Nacht unter Rückfluss gerührt. Nach dem Erkalten wird das Reaktionsgemisch filtriert und das Filtrat vollständig eingeengt. Das erhaltene Öl wird aus Essigester / Ethanol umkristallisiert. Der Feststoff wird abfiltriert und anschließend in Wasser gelöst. Durch Zugabe von Triethylamin wird die Lösung basisch gestellt und mit Essigester extrahiert (2x). Die vereinten organischen Phasen werden getrocknet (Na₂SO₄), filtriert und eingeengt. Man erhält 444 mg (1,2 mmol, entsprechend 40% der Theorie) des Produktes.
¹H-NMR (DMSO): 9.18 (s, 1H), 9.06 (s, 1H), 7.97 (s, 1H), 7.19 (m, 2H), 6.99 (m, 2H), 6.32 (m, 1H), 4.45 (t, 1H), 3.40 (m, 4H), 1.60 (m, 2H), 1.47 (m, 2H).
MS: 352 (ES).

### Ringschluss durch Makrolactamisierung

### Verfahrensvariante 13

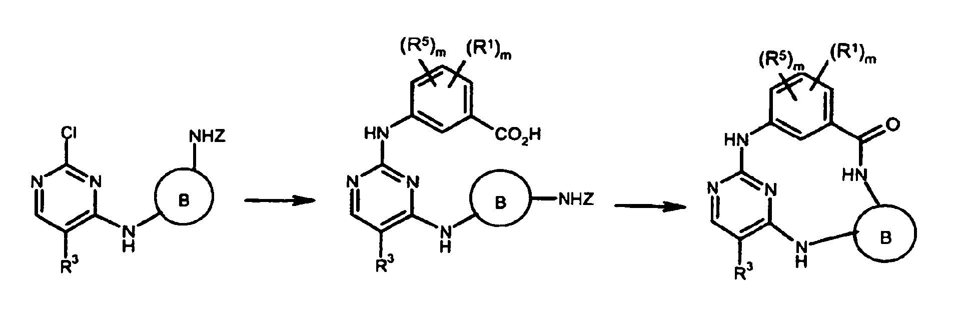

In den Formeln haben R¹, R³, R⁵, B und m die in den Verbindungen der Ansprüche 1 bis 3 verwirklichten Bedeutungen und NHZ steht für die

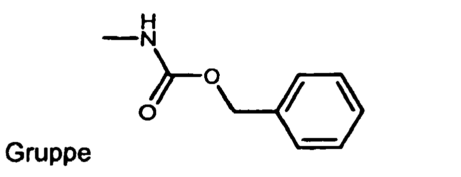

Synthese von Makrolactamen erfolgt nach gängigen Verfahren ((a) Xue, C.-B. *J. Med. Chem.* 2001, *44*, 2636. (b) Jackson, F.W. *J. Org. Chem.* 2002, *67*, 4882).

### Beispiel 13.0

### Herstellung von 1⁵-Bromo-2,5,11-triaza-1(2,4)-pyrimidina-3(1,3)-benzenacycloundecaphane-4-one (A) und 2,5,11-Triaza-1(2,4)-pyrimidina-3(1,3)-benzenacycloundecaphane-4-one (B)

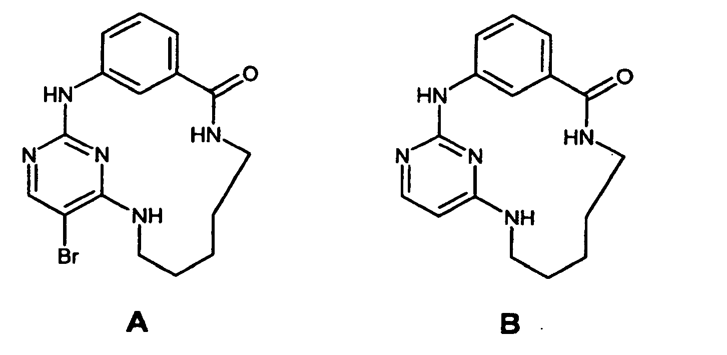

Eine Lösung von 300 mg (0,57 mmol) 3-[4-(5-Benzyloxycarbonylamino-pentylamino)-5-brom-pyrimidin-2-ylamino]-benzoesäure in Methanol / Dichlormethan (40ml / 5 ml) wird mit 350 mg Pd / C (10%) versetzt und 150 min in einer Niederdruck-Apparatur hydriert. Das Reaktionsgemisch wird über Celite filtriert und vollständig eingeengt. Der verbleibende Rückstand wird in DMF / Methanol / Wasser (10,0 ml / 1,0 ml / 0,2 ml) gelöst und mittels Spritzenpumpe über 2 Stunden zu einer Lösung von 410 mg (2,2 mmol) EDC, 330 mg (2,2 mmol) HOBt und 0,25 ml *N*-Methylmorpholin in 200 ml DMF gegeben. Nach 72 Stunden wird das Reaktionsgemisch eingeengt, mit Wasser versetzt und anschließend mit Essigester extrahiert (2x). Die vereinten organischen Phasen werden mit Wasser gewaschen, getrocknet (Na₂SO₄), filtriert und eingeengt.

Der gebildete Rückstand wird chromatographisch gereinigt (Dichlormethan / Methanol 1:1). Man erhält 35 mg (0,09 mmol, entsprechend 16% der Theorie) an 1⁵⁻Bromo-2,5,11-triaza-1(2,4)-pyrimidina-3(1,3)-benzenacycloundecaphane-4-one (**A**) und 13 mg (0,04 mmol, entsprechend 7% der Theorie) an 2,5,11-Triaza-1(2,4)-pyrimi-dina-3(1,3)-benzenacycloundecaphane-4-one (**B**).

1⁵-Brom-2,5,11-triaza-1(2,4)-pyrimidina-3(1,3)-benzenacycloundecaphane-4-one (**A**):
¹H-NMR (DMSO): 9.45 (s, 1H), 8.81 (s, 1H), 8.12 (t, 1H), 7.98 (s, 1H), 7.20 (m, 4H), 3.30 (m, 4H), 1.78 (m, 2H), 1.60 (m, 2H), 1.30 (m, 2H).
MS: 376 (ES).

2,5,11-Triaza-1(2,4)-pyrimidina-3(1,3)-benzenacycloundecaphane-4-one (**B**):
¹H-NMR (DMSO): 9.21 (s, 1H), 8.97 (s, 1H), 8.10 (t, 1H), 7.72 (d, 1H), 7.38 (t, 1H), 7.25 (t, 1H), 7.18 (dd, 1H), 7.08 (dd, 1H), 5.84 (d, 1H), 3.30 (m, 2H), 3.17 (m, 2H), 1.75 (m, 2H), 1.53 (m, 2H), 1.30 (m, 2H).MS: 298 (ES).

### Herstellung der Zwischenprodukte nach Verfahrensvariante 13

### 13a) Herstellung von [5-(5-Brom-2-chlor-pyrimidin-4-ylamino)-pentyl]-carbaminsäure-benzylester

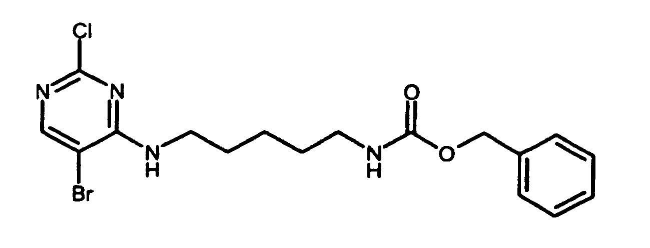

Eine Lösung von 860 mg (3,8 mmol) 5-Brom-2,4-dichlor-pyrimidin und 1,2 ml (8,5 mmol) Triethylamin in 6 ml Acetonitril wird bei 0°C mit 1,0 g (3,7 mmol) (5-Amino-pentyl)-carbaminsäure-benzylester versetzt. Das Reaktionsgemisch wird durch Entfernen des Eisbades langsam unter Rühren auf Raumtemperatur erwärmt. Nach 60 Stunden wird mit Wasser versetzt und anschließend mit Essigester extrahiert (2x). Die vereinten organischen Phasen werden getrocknet (Na₂SO₄), filtriert und eingeengt. Der gebildete Rückstand wird chromatographisch gereinigt (Hexan / Essigester 1:1). Man erhält 1,2 g (2,8 mmol, entsprechend 77% der Theorie) des Produktes.
¹H-NMR (DMSO): 8.21 (s, 1H), 7.72 (t, 1H), 7.35 (m, 5H), 7.23 (t, 1H), 4.99 (s, 2H), 3.30 (m, 2H), 2.97 (m, 2H), 1.47 (m, 4H), 1.27 (m, 2H). MS: 427 (ES).

### 13b) Herstellung von 3-[4-(5-Benzyloxycarbonylamino-pentylamino)-5-brom-pyrimidin-2-ylamino]-benzoesäure

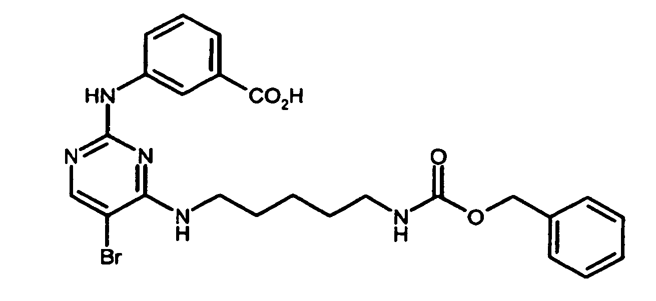

Ein Reaktionsgemisch von 1,20 g (2,8 mmol) [5-(5-Brom-2-chlor-pyrimidin-4-ylamino)-pentyl]-carbaminsäure-benzylester und 0,37 g (2,7 mmol) 3-Aminobenzoesäure in Acetonitril / Wasser (8 ml / 1,5 ml) wird 20 Stunden unter Rückfluss gerührt. Das Reaktionsgemisch wird einrotiert und der verbleibende Rückstand chromatographisch (Dichlormethan / Methanol 9:1, Flashmaster II) gereinigt. Man erhält 1,27 g (2,4 mmol, entsprechend 86% der Theorie) des Produktes.
¹H-NMR (DMSO): 10.03 (s, 1H), 8.38 (s, 1H), 8.15 (s, 1H), 7.81 (m, 2H), 7.56 (d, 1H), 7.30 (m, 7H), 4.98 (s, 2H), 3.35 (m, 2H), 2.98 (m, 2H), 1.54 (m, 2H), 1.32 (m, 4H).
MS: 528 (CI).

### Herstellung von Aza-phanderivaten

### Verfahrensvariante 14

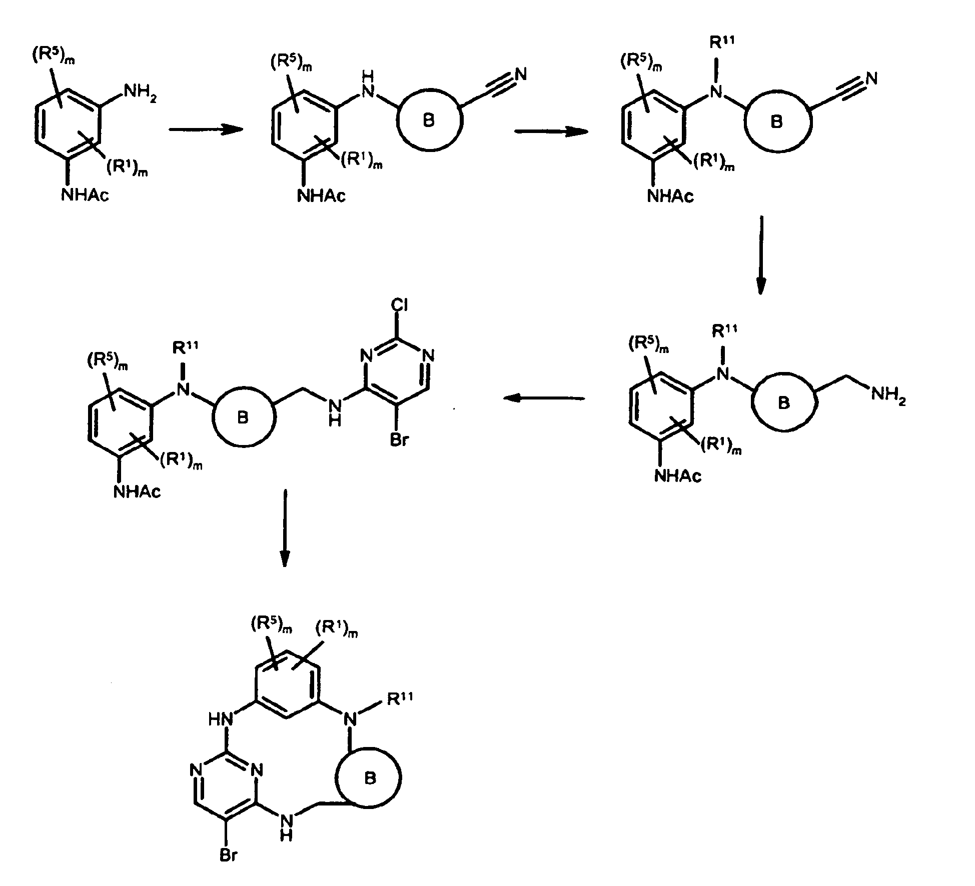

In den allgemeinene Formeln haben R¹, R⁵, R¹¹, B und m die in den Verbindungen der Ansprüche 1 bis 3 verwirklichten Bedeutungen .

### Beispiel 14.0

### Herstellung von 1⁵ -Bromo-4-mesyl-2,4,9-triaza-1(2,4)-pyrimidina-3(1,3)-benzenacyclononaphane

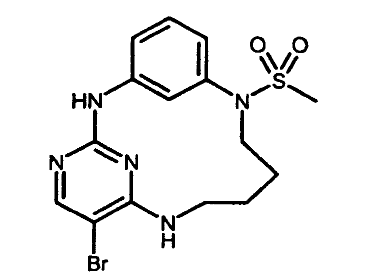

Eine Lösung von 160 mg (0.33 mmol) *N*-(3-{[4-(5-Brom-2-chlor-pyrimidin-4-ylamino)-butyl]-methansulfonyl-amino}-phenyl)-acetamid in 10 ml Acetonitril wird mittels Spritzenpumpe innerhalb von 3 Stunden zu einer refluxierenden Lösung von Acetonnitril / Wasser / 4 molare Lösung von Chlorwasserstoff in Dioxan (40 ml / 10 ml / 1 ml) gegeben. Nach beendeter Zugabe wird der Ansatz weitere 16 Stunden unter Rückfluss gerührt und anschließend das organische Lösungsmittel abgezogen. Man versetzt mit Essigester und wäscht mit verdünnter NaHCO₃ Lösung. Die vereinten organischen Phasen werden eingeengt und der erhaltene Rückstand mit Essigester, MeOH und Wasser gewaschen. Nach dem Trocknen erhält man 81 mg (0.20 mmol, entsprechend 63 % der Theorie) des Produktes.
¹H-NMR (DMSO): 9.38 (s, 1H), 8.16 (m, 1H), 7.97 (s, 1H), 7.23 (m, 2H), 6.99 (m, 1H), 6.92 (m, 1H), 3.64 (m, 2H), 3.16 (m, 2H), 3.06 (s, 3H), 1.77 (m, 2H), 1.60 (m, 2H).
MS: 412 (ES).

### Herstellung der Zwischenprodukte

### 14a) N-(3-{[4-(5-Brom-2-chlor-pyrimidin-4-ylamino)-butyl]-methansulfonyl-amino}-phenyl)-acetamid

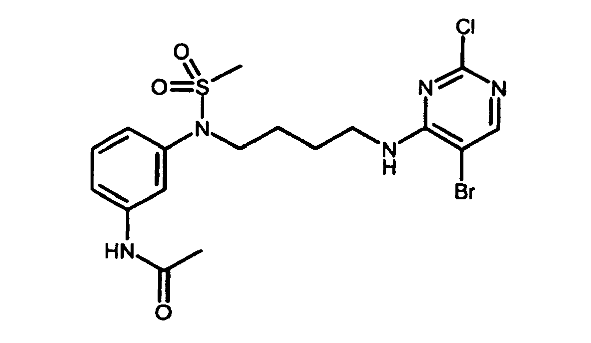

460 mg (1.56 mmol) *N*-{3-[(3-Cyano-propyl)-methansulfonyl-amino]-phenyl}-acetamid in 25 ml Ethanol und 0.5 ml konz. HCl werden unter Verwendung von 60 mg (0.26 mmol) Platin(IV)oxid 5 Stunden bei Raumtemperatur unter Normaldruck hydriert. Der Ansatz wird filtriert und eingeengt. Der erhaltene Rückstand wird mit einer Lösung von 355 mg (1.56 mmol) 5-Brom-2,4-dichlor-pyrimidin in 15 ml Acetonitril versetzt. Man gibt 0.45 ml Triethylamin tropfenweise hinzu und rührt 16 Stunden bei Raumtemperatur. Der Ansatz wird eingeengt und der erhaltene Rückstand chromatographisch (DCM / EtOH 95:5) gereinigt. Man erhält 330 mg (0.67 mmol, entsprechend 43 % der Theorie) des Produktes.
¹H-NMR (DMSO): 10.03 (s, 1H), 8.21 (s, 1H), 7.69 (t, 1H), 7.55 (m, 2H), 7.29 (m, 1 H), 7.03 (m, 1H), 3.60 (t, 2H), 3.30 (m, 2H), 2.97 (s, 3H), 2.03 (s, 3H), 1.57 (m, 2H), 1.36 (m, 2H).
MS: 490 (ES).

### 14b) N-{3-[(3-Cyano-propyl)-methansulfonyl-amino]-phenyl}-acetamid

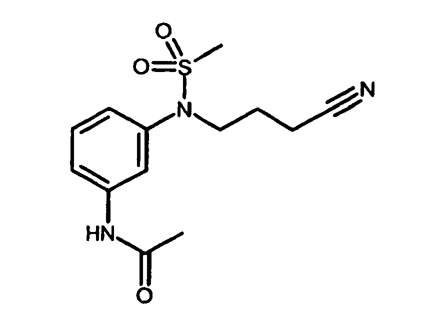

Eine Lösung von 510 mg (2.35 mmol) N-[3-(3-Cyano-propylamin)-phenyl]-acetamid in 10 ml Pyridin wird bei 0 °C tropfenweise mit 0.21 ml Methansulfonylchlorid versetzt und anschließend 24 h bei Raumtemperatur gerührt. Nach DC Kontrolle wird erneut mit 0.1 ml Methansulfonylchlorid versetzt und weitere 3 Tage gerührt. Der Ansatz wird mit Essigester verdünnt und mit Zitronensäure (10%), gesättigter NaHCO₃ Lösung sowie gesättigter NaCl Lösung gewaschen. Die organische Phase wird getrocknet (Na₂SO₄), filtriert und eingeengt. Man erhält 469 mg (1.60 mmol, entsprechend 68 % der Theorie) des Produktes.
¹H-NMR (DMSO): 10.11 (s, 1H), 7.59 (m, 2H), 7.37 (m, 1H), 7.11 (m, 1H), 3.68 (t, 2H), 3.02 (s, 3H), 2.51 (m, 2H), 2.04 (s, 3H), 1.67 (p, 2H).
MS: 321 (ES).

### 14c) N-[3-(3-Cyano-propylamin)-phenyl]-acetamid

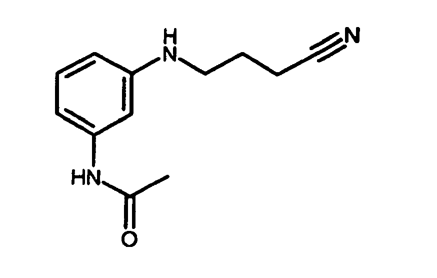

Eine Lösung von 3.18 g (21.2 mmol) *N*-(3-Amino-phenyl)-acetamid in 100 ml Acetonitril wird bei Raumtemperatur mit 1.9 ml (19.0 mmol) 4-Brombuttersäurenitril und 2.6 ml Triethylamin versetzt und anschließend über Nacht unter Rückfluss gerührt. Nach dem Erkalten wird mit Essigester verdünnt und mit Zitronensäure (10%), gesättigter NaHCO₃ Lösung sowie gesättigter NaCl Lösung gewaschen. Die organische Phase wird getrocknet (Na₂SO₄), filtriert und eingeengt. Der erhaltene Rückstand wird chromatographisch (DCM / EtOH 95:5) gereinigt. Man erhält 0.56 g (2.35 mmol, entsprechend 12 % der Theorie) des Produktes.
¹H-NMR (DMSO): 9.66 (s, 1H), 6.98 (m, 2H), 6.72 (m, 1H), 6.26 (m, 1H), 5.69 (t, 1H), 3.04 (m, 2H), 2.62 (t, 2H), 2.02 (s, 3H),1.82 (p, 2H).
MS: 218 (ES).

In analoger Verfahrensweise zu den oben jeweils beschriebenen Verfahrensvarianten werden auch die nachfolgenden Verbindungen hergestellt:

| | | | |
|---|---|---|---|
| | 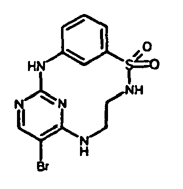 | 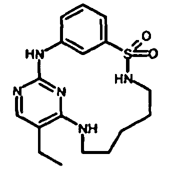 | 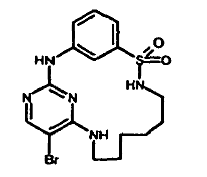 |
| Beispiel-Nr. | 14.1 | 14.2 | 14.3 |
| Masse | 370 (ES) | 362 (ES) | 426 (ES) |
| Verf.var. | 1 | 1 | 1 |
| | 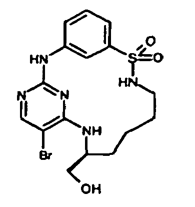 | 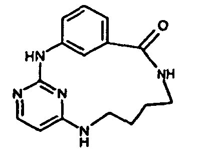 | 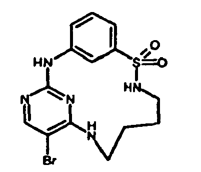 |
| Beispiel-Nr. | 14.4 | 14.5 | 14.6 |
| Masse | 442 (ES) | 284 (Cl) | 398 (ES) |
| Verf.var. | 1 | 13 | 1 |
| | 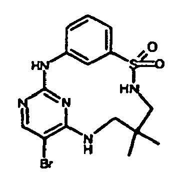 | 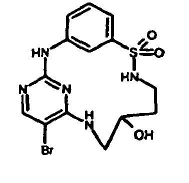 | 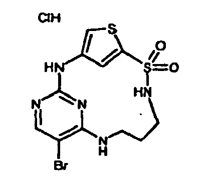 |
| Beispiel-Nr. | 14.7 | 14.8 | 14.9 |
| Masse | 412 (ES) | 414 (ES) | 390 (ES) |
| Verf.var. | 1 | 1 | 6 |
| | 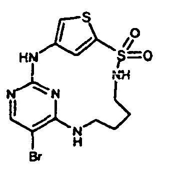 | 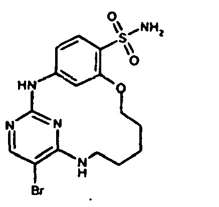 | 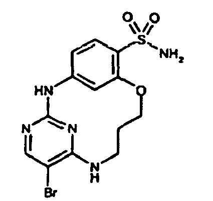 |
| Beispiel-Nr | 14.10 | 14.11 | 14.12 |
| Masse | 404 (ES) | 428 (ES) | 400 (ES) |
| Verf.var. | 6 | 8 | 8 |
| | 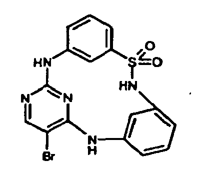 | 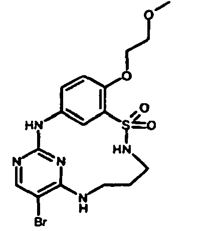 | 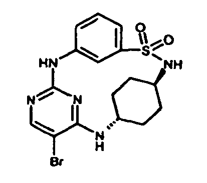 |
| Beispiel-Nr. | 14.13 | 14.14 | 14.15 |
| Masse | 418 (ES) | 458 (ES) | 424 (ES) |
| Verf.var. | 1 | 1 | 1 |
| | 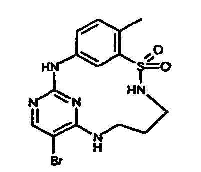 | 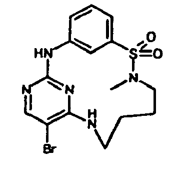 | 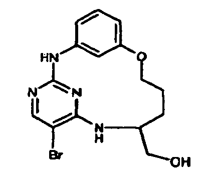 |
| Beispiel-Nr. | 14.16 | 14.17 | 14.18 |
| Masse | 398 (ES) | 412 (ES) | 365 (ES) |
| Verf.var. | 1 | 2 | 1 |
| | 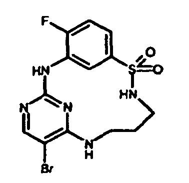 | 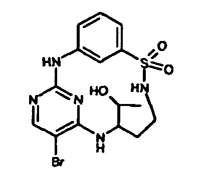 | 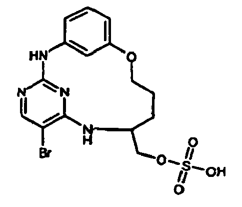 |
| Beispiel-Nr. | 14.19 | 14.20 | 14.21 |
| Masse | 402 (ES) | 442 (ES) | 445 (ES) |
| Verf.var. | 1 | 1 | 1 |
| | 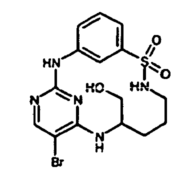 | 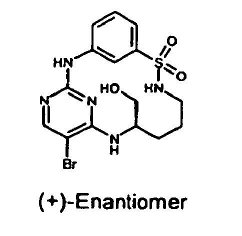 | 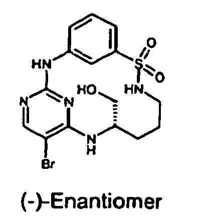 |
| Beispiel-Nr. | 14.22 | 14.23 | 14.24 |
| Masse | 428 (ES) | 428 (ES) | 428 (ES) |
| Verf.var. | 1 | 1 | 1 |
| | 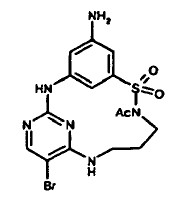 | 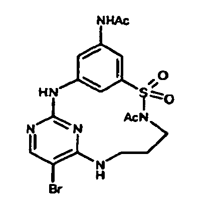 | 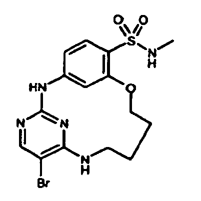 |
| Beispiel-Nr. | 14.25 | 14.26 | 14.27 |
| Masse | 441 (ES) | 483 (ES) | 430 (ES) |
| Verf.var. | 1 | 1 | 8 |
| | 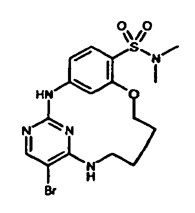 | | |
| Beispiel-Nr. | 14.28 | | |
| Masse | 442 (ES) | | |
| Verf.var. | 8 | | |

Wie durch den Fachmann angenommen werden kann, beschreiben die oben beschriebenen Verfahren nicht alle möglichen Herstellungsweisen der erfindungsgemäßen Produkte. Weiterführende Methoden werden einem Fachmann anhand seines Fachwissen naheliegend sein. Zusätzlich sind die Herstellungsverfahren nicht darauf beschränkt in dieser Reihenfolge durchgeführt zu werden. Die chemischen Transformationen und Schutzgruppen, die in dieser Anmeldung beschrieben sind und für die Synthesewege erforderlich sind, sind Stand der Technik und insbesondere in R. Larock, Comprehensive Organic Transformations, VCH Publishers (1989), T.W. Greene und P.G.M. Wurtz, Protective Groups in Organic Synthesis, John Wiley und Sons (1994) und L. Paquette, ed., Encyclopedia of Reagents for Organic Synthesis, John Wiley and Sons(1995), beschrieben.

Die nachfolgenden Beispiele können analog der vorbeschriebenen Verfahrensvarianten oder dem Fachmann naheliegender Varianten erhalten werden:

| | | | |
|---|---|---|---|
| | 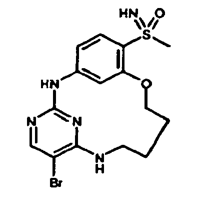 | 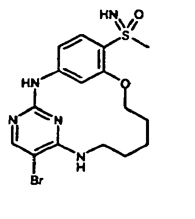 | 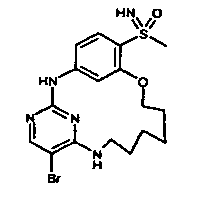 |
| Bsp | 15.0 | 15.1 | 15.2 |

Weiteren Syntheseerfordemisse bezüglich der Sulfoximinderivate sind in a) M. Regglin, C. Zur, Synthesis, 2000, 1, 1-64. b) S.L. Huang, D. Swem, Phosphorous and Sulfur, 1976, 1, 309-314. c) S. Oae, K. Harada, K. Tsujihara, N. Furukawa, lnt. J. Sulfur Chem., Part A, 2, 1, 49-61. d) S.G. Pyne, Sulfur Reports, 12, 1, 57-93 beschrieben.

| | | | |
|---|---|---|---|
| | 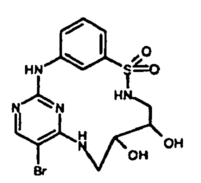 | 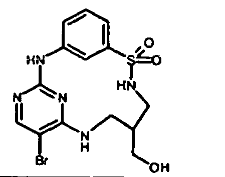 | 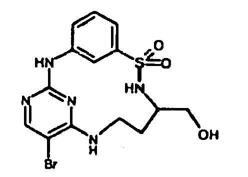 |
| Bsp.nr. | 15.4 | 15.5 | 15.6 |
| | 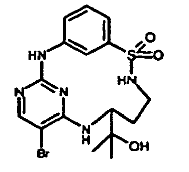 | 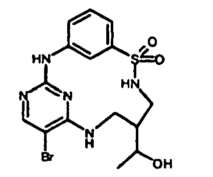 | 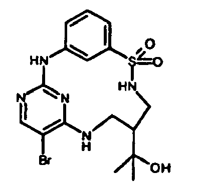 |
| Bsp.nr. | 15.7 | 15.8 | 15.9 |
| | 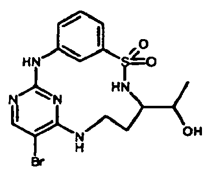 | 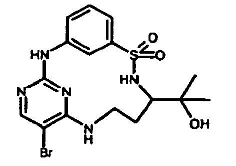 | 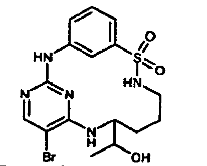 |
| Bsp.nr. | 15.10 | 15.11 | 15.12 |
| | 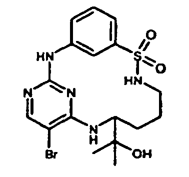 | 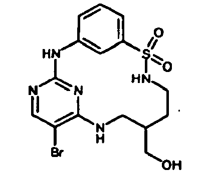 | 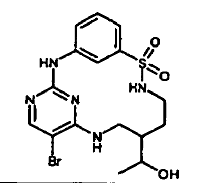 |
| Bsp.nr. | 15.13 | 15.14 | 15.15 |
| | 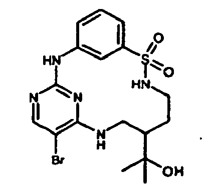 | 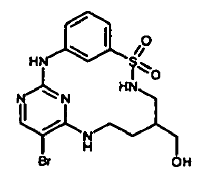 | 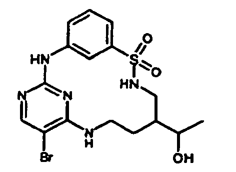 |
| Bsp.nr. | 15.16 | 15.17 | 15.18 |
| | 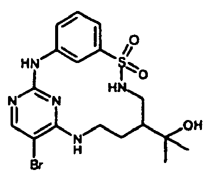 | 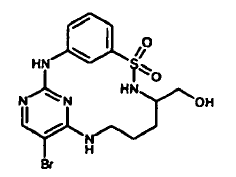 | 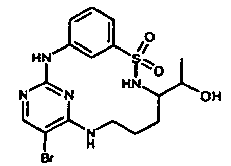 |
| Bsp.nr. | 15.19 | 15.20 | 15.21 |
| | 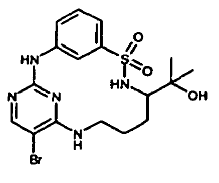 | | |
| Bsp.nr. | 15.22 | | |
| | 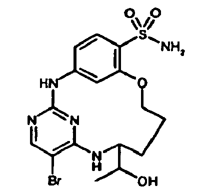 | 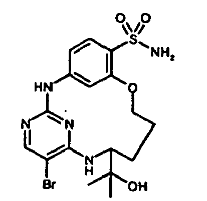 | 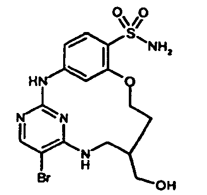 |
| Bsp.nr. | 15.23 | 15.24 | 15.25 |
| | 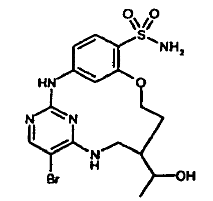 | 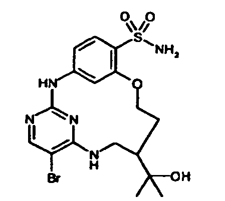 | 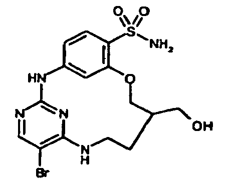 |
| Bsp.nr. | 15.26 | 15.27 | 15.28 |
| | 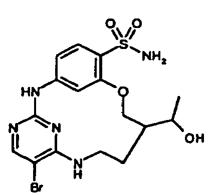 | 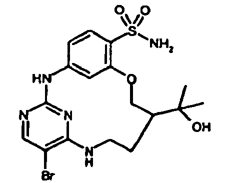 | 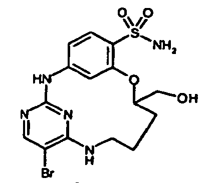 |
| Bsp.nr. | 15.29 | 15.30 | 15.31 |
| | 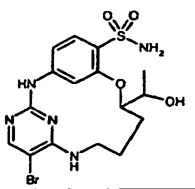 | 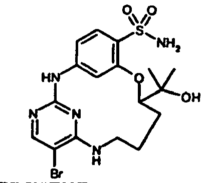 | |
| Bsp.nr. | 15.32 | 15.33 | |
| | 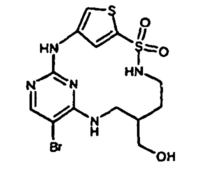 | 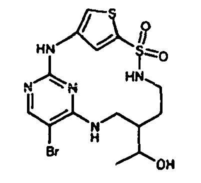 | 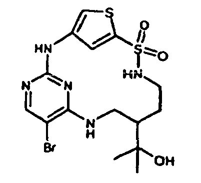 |
| Bsp.nr. | 15.34 | 15.35 | 15.36 |
| | 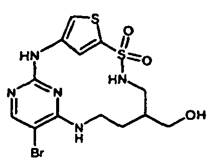 | 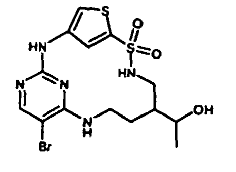 | 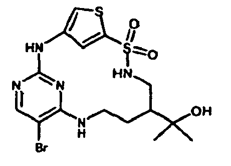 |
| Bsp.nr. | 15.37 | 15.38 | 15.39 |
| | 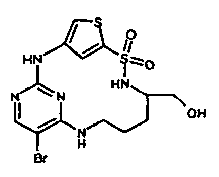 | 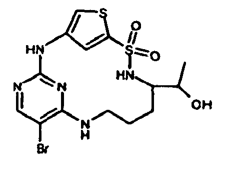 | 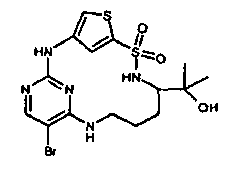 |
| Bsp.nr. | 15.40 | 15.41 | 15.42 |
| | 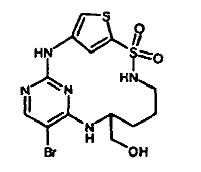 | 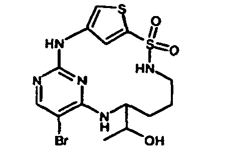 | 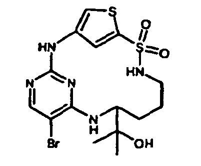 |
| Bsp.nr. | 15.43 | 15.44 | 15.45 |
| | 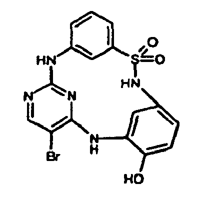 | 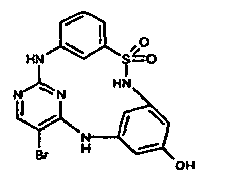 | 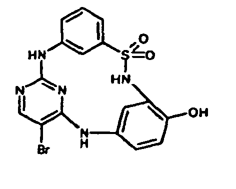 |
| Bsp.nr. | 15.46 | 15.47 | 15.48 |
| | 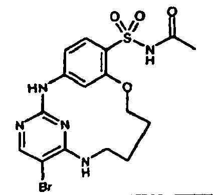 | 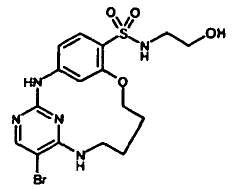 | 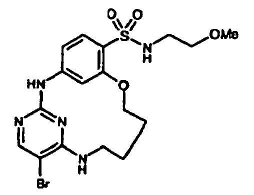 |
| Bsp.nr. | 15.49 | 15.50 | 15.51 |
| | 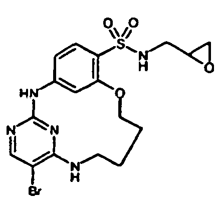 | 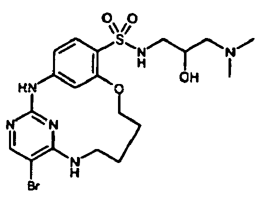 | 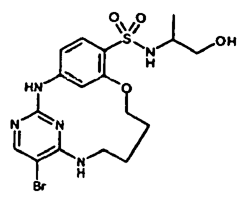 |
| Bsp.nr. | 15.52 | 15.53 | 15.54 |
| | 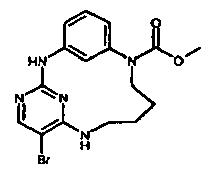 | 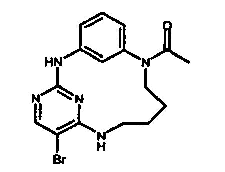 | 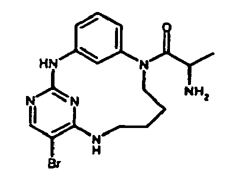 |
| Bsp.nr. | 15.55 | 15.56 | 15.57 |
| | 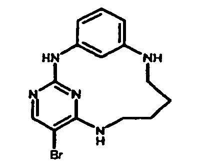 | 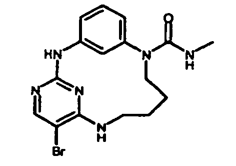 | 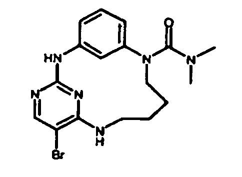 |
| Bsp.nr. | 15.58 | 15.59 | 15.60 |
| | 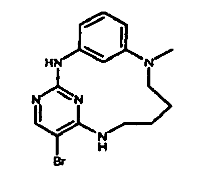 | 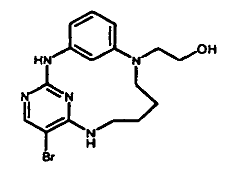 | 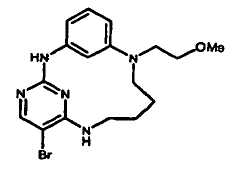 |
| Bsp.nr. | 15.61 | 15.62 | 15.63 |
| | 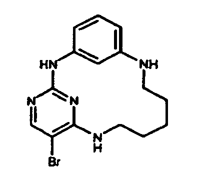 | 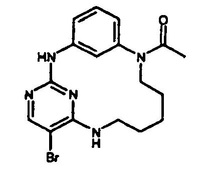 | 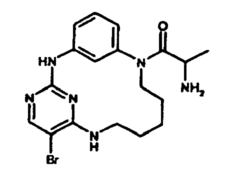 |
| Bsp.nr | 15.64 | 15.65 | 15.66 |
| | 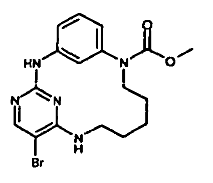 | 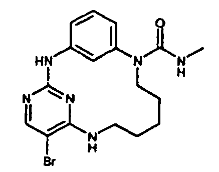 | 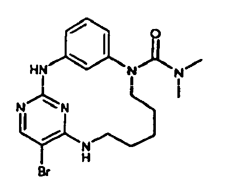 |
| Bsp.nr. | 15.67 | 15.68 | 15.69 |
| | 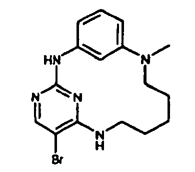 | 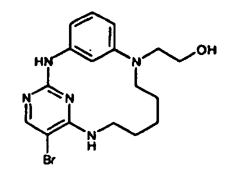 | 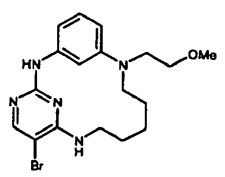 |
| Bsp.nr. | 15.70 | 15.71 | 15.72 |
| | 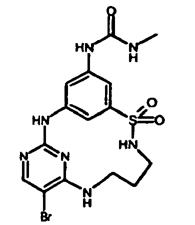 | 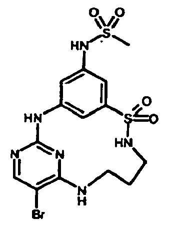 | |
| Bsp.nr. | 15.73 | 15.74 | |
| | 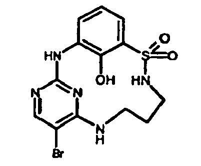 | 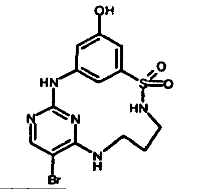 | 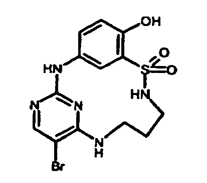 |
| Bsp.nr. | 15.75 | 15.76 | 15.77 |
| Weiterführende Literatur | W.G. Watson, Pestic. Sci., 1996, 46, 131-138 | Yoshida, Bull. Soc.Sci. Photogr. Jpn., 1969, 19, 41 | R. Cremlyn, Phosphorus sulfur, 1981, 12, 197-204. |
| | 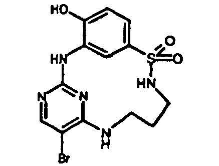 | | |
| Bsp.nr. | 15.78 | | |
| Weiterführende Literatur | Katrizky, Synth. Synth. Commun., 1993, 23, 3, 405-417 | | |
| | 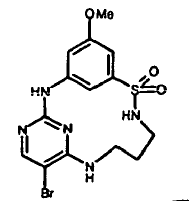 | 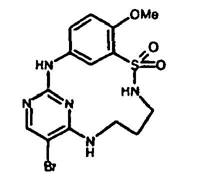 | 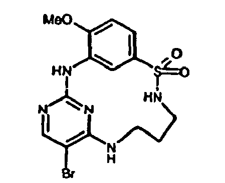 |
| Bsp.nr. | 15.79 | 15.80 | 15.81 |
| | 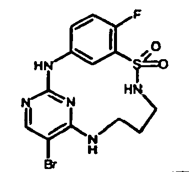 | 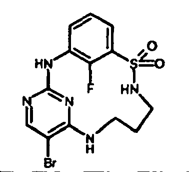 | |
| Bsp.nr. | 15.82 | 15.83 | |
| Weiterführende Literatur | A. Courtin, Helv. Chim. Acta, 1982, 65, 2, 546-550. | A. Courtin, Helv. Chim. Acta, 1983, 66, 1, 68-75 | |
| | 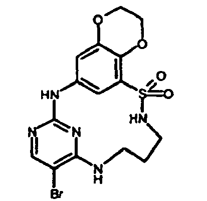 | 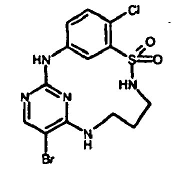 | 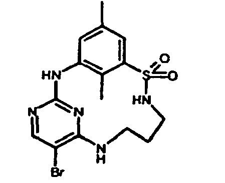 |
| Bsp.nr. | 15.84 | 15.85 | 15.86 |
| Weiterführende Literatur | Heertjes, Red. Trav. Chim. Pays-Bas, 1950, 69, 262 | Fischer, Chem. Ber., 1891, 3188 | Karslake, J. Am. Chem. Soc., 1914, 36, 1247 |
| | 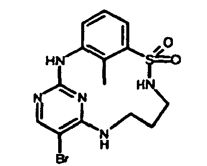 | 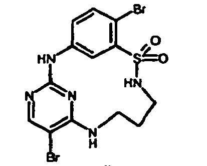 | |
| Bsp.nr. | 15.87 | 15.88 | |
| Weiterführende Literatur | Courtin, Chimica, 1975, 29, 168 | Petrov, J. Pharm. Pharmacol., 1960, 12 | |
| | 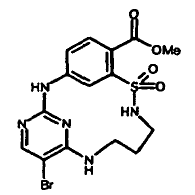 | 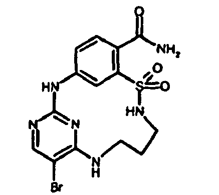 | 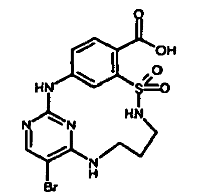 |
| Bsp.nr. | 15.89 | 15.90 | 15.91 |
| Weiterführende Literatur | G. Remsen, Am. Chem. J., 1897, 1897, 19, 496. | G. Remsen, Am. Chem. J., 1897, 1897, 19, 496. | G. Remsen, Am. Chem. J., 1897, 1897, 19, 496. |
| | 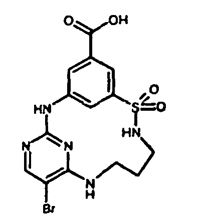 | 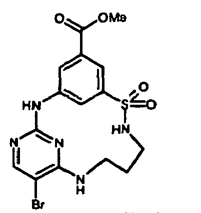 | 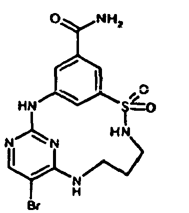 |
| Bsp.nr. | 15.92 | 15.93 | 15.94 |
| Weiterführende Literatur | Shah, J. Chem. Soc.; 1933, 1373 | Shah, J. Chem. Soc.; 1933, 1373 | Shah, J. Chem. Soc.; 1933, 1373 |
| | 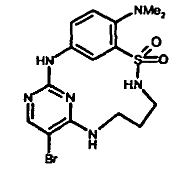 | 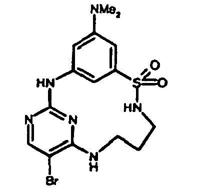 | 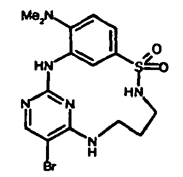 |
| Bsp.nr. | 15.95 | 15.96 | 15.97 |
| Weiterführende Literatur | Abramovitch, J. Org. Chem. 1977, 42,2920 | | Wilson, J. Am. Chem. Soc., 1944,66,835 |
| | 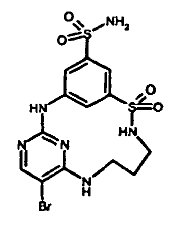 | 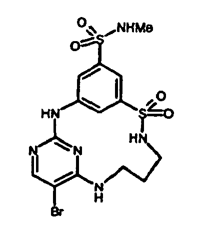 | 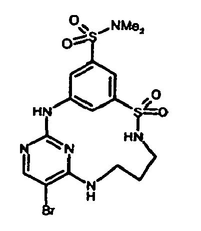 |
| Bsp.nr. | 15.98 | 15.99 | 16.0 |
| Weiterführende Literatur | Bennett, J. Chem. Soc., 1929, 267 | Bennett, J. Chem. Soc., 1929, 267 | Bennett, J. Chem. Soc., 1929, 267 |
| | 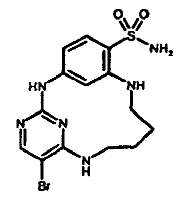 | 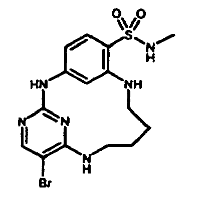 | 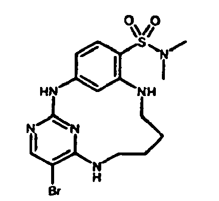 |
| Bsp.nr. | 16.1 | 16.2 | 16.3 |
| Weiterführende Literatur | Williams, Biochem. J., 1941, 35, 61 | Williams, Biochem. J., 1941,35,61 | Williams, Biochem. J., 1941, 35, 61 |
| | 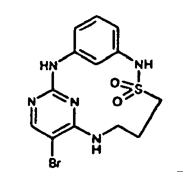 | 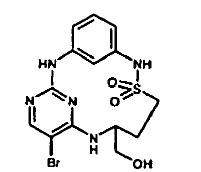 | |
| Bsp.nr. | 16.4 | 16.5 | |
| Weiterführende Literatur | Orus, Pharmazie, 2002, 57, 8, 515 | Orus, Pharmazie, 2002, 57,8,515 | |
| | 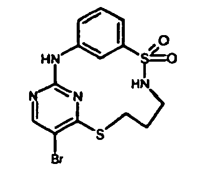 | 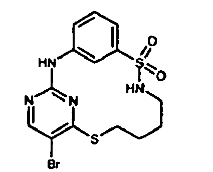 | |
| Bsp.nr. | 16.6 | 16.7 | |
| Weiterführende Literatur | Strekowski, Bull. Acad. Pol. Sci. Ser. Sci. Chim, 1976, 24, 29 | Strekowski, Bull. Acad. Pol. Sci. Ser. Sci. Chim, 1976, 24, 29 | |

Sekundäre oder tertiäre Alkoholderivate können aus primären Alkoholen über Oxidation / Grignard Reaktion z.B. analog der Methoden aus WO 02/096888, Seite 186-191 hergestellt werden. Zur Oxidation eignet sich unter anderem die TPAP Oxidation (siehe S.V. Ley, Synthesis, 1994, 639). Eine Übersicht zur Grignard Reaktion gibt z. B. H. Gilman in Methoden der Org. Chem. (Houben-Weyl), 1973, Bd. 13/2a, S.49.

Zusätzliche Literatur, die weitere Angaben zur Herstellung von den jeweiligen Derivaten gibt, ist als weiterführende Literatur zu den bestimmten Beispielen aufgelistet.

Die nachfolgenden Beispiele beschreiben die biologische Wirkung der erfindungsgemäßen Verbindungen ohne die Erfindung auf diese Beispiele zu beschränken.

### Beispiel 1

### CDK1/CycB Kinase Assay

Rekombinante CDK1- und CycB-GST-Fusionsproteine, gereinigt aus Bakulovirus-infizierten Insektenzellen (Sf9), wurden von ProQinase GmbH, Freiburg, gekauft. Das als Kinase-Substrat verwendet Histon IIIS ist über die Fa. Sigma käuflich zu erwerben.
CDK1/CycB (200 ng/Meßpunkt) wurde für 15 min bei 22°C in Anwesenheit verschiedener Konzentrationen an Testsubstanzen (0 µM, sowie innerhalb des Bereiches 0,01 - 100 µM) in Assaypuffer [50 mM Tris/HCl pH8,0, 10 mM MgCl2, 0,1 mM Na ortho-Vanadat, 1,0 mM Dithiothreitol, 0,5 µM Adenosintrisphosphat (ATP), 10 µg/Meßpunkt Histon IIIS, 0,2 µCi/Messpunkt 33P-gamma ATP, 0,05% NP40, 12,5% Dimethylsulfoxid] inkubiert. Die Reaktion wurde durch Zugabe von EDTA-Lösung (250 mM, pH8,0, 14 µl/Meßpunkt) gestoppt.
Von jedem Reaktionsansatz wurden 10 µl auf P30 Filterstreifen (Fa. Wallac) aufgetragen, und nicht-eingebautes 33P-ATP wurde durch dreimaliges Waschen der Filterstreifen für je 10 min in 0,5%iger Phosphorsäure entfernt. Nach dem Trocknen der Filterstreifen für 1 Stunde bei 70°C wurden die Filterstreifen mit Szintillator-Streifen (MeltiLexTM A, Fa. Wallac) bedeckt und für 1 Stunde bei 90°C eingebrannt. Die Menge an eingebautem 33P (Substratphosphorylierung) wurde durch Szintillationsmessung in einem Gamma-Strahlungsmessgerät (Wallac) bestimmt.

### Beispiel 2

### CDK2/CycE Kinase Assay

Rekombinante CDK2- und CycE-GST-Fusionsproteine, gereinigt aus Bakulovirus-infizierten Insektenzellen (Sf9), wurden von ProQinase GmbH, Freiburg, gekauft. Histon IIIS, das als Kinase-Substrat verwendet wurde, wurde bei der Fa. Sigma gekauft.
CDK2/CycE (50 ng/Meßpunkt) wurde für 15 min bei 22°C in Anwesenheit verschiedener Konzentrationen an Testsubstanzen (0 µM, sowie innerhalb des Bereiches 0,01 - 100 µM) in Assaypuffer [50 mM Tris/HCl pH8,0, 10 mM MgCl₂, 0,1 mM Na ortho-Vanadat, 1,0 mM Dithiothreitol, 0,5 µM Adenosintrisphosphat (ATP), 10 µg/Messpunkt Histon IIIS, 0,2 µCi/Messpunkt ³³P-gamma ATP, 0,05% NP40, 12,5% Dimethylsulfoxid] inkubiert. Die Reaktion wurde durch Zugabe von EDTA-Lösung (250 mM, pH8,0, 14 µl/Meßpunkt) gestoppt.
Von jedem Reaktionsansatz wurden 10 µl auf P30 Filterstreifen (Fa. Wallac) aufgetragen, und nicht-eingebautes ³³P-ATP wurde durch dreimaliges Waschen der Filterstreifen für je 10 min in 0,5%iger Phosphorsäure entfernt. Nach dem Trocknen der Filterstreifen für 1 Stunde bei 70°C wurden die Filterstreifen mit Szintillator-Streifen (MeltiLex™ A, Fa. Wallac) bedeckt und für 1 Stunde bei 90°C eingebrannt. Die Menge an eingebautem ³³P (Substratphosphorylierung) wurde durch Szintillationsmessung in einem Gamma-Strahlungsmessgerät (Wallac) bestimmt.

### Beispiel 3

### VEGF Rezeptor-2 Kinase Assay

Rekombinante VEGF Rezeptortyrosinkinase-2 wurde als GST-Fusionsprotein aus Bakulovirus-infizierten Insektenzellen (Sf9) gereinigt. Poly-(Glu4Tyr), das als Kinase-Substrat verwendet wurde, wurde bei der Fa. Sigma gekauft.
VEGF Rezeptortyrosinkinase (90 ng/Meßpunkt) wurde für 10 min bei 22°C in Anwesenheit verschiedener Konzentrationen an Testsubstanzen (0 µM, sowie innerhalb des Bereiches 0,001 - 30 µM) in 30 µl Assaypuffer [40 mM Tris/HCl pH5,5, 10 mM MgCl2, 1 mM MnCl2, 3 µM Na ortho-Vanadat, 1,0 mM Dithiothreitol, 8 µM Adenosintrisphosphat (ATP), 27 µg/Meßpunkt poly-(Glu4Tyr), 0,2 µCi/Meßpunkt 33P-gamma ATP, 1% Dimethylsulfoxid] inkubiert. Die Reaktion wurde durch Zugabe von EDTA-Lösung (250 mM, pH7,0, 10 µl/Meßpunkt) gestoppt.
Von jedem Reaktionsansatz wurden 10 µl auf P30 Filterstreifen (Fa. Wallac) aufgetragen, und nicht-eingebautes 33P-ATP wurde durch dreimaliges Waschen der Filterstreifen für je 10 min in 0,5%iger Phosphorsäure entfernt. Nach dem Trocknen der Filterstreifen für 1 Stunde bei 70°C wurden die Filterstreifen mit Szintillator-Streifen (MeltiLexTM A, Fa. Wallac) bedeckt und für 1 Stunde bei 90°C eingebrannt. Die Menge an eingebautem 33P (Substratphosphorylierung) wurde durch Szintillationsmessung in einem gamma-Strahlungsmeßgerät (Wallac) bestimmt. Die IC50-Werte bestimmen sich aus der Inhibitorkonzentration, die notwendig ist, um den Phosphateinbau auf 50% des ungehemmten Einbaus nach Abzug des Leerwertes (EDTA-gestoppte Reaktion) zu hemmen.

### Beispiel 4

### Proliferationsassay

Kultivierte humane Tumorzellen (MCF7, hormonunabhängige menschliche Mammakarzinomazellen, bezogen von ATCC HTB22; NCI-H460, menschliche nicht kleinzellige Lungenkarzinomazellen, ATCC HTB-177, HCT 116, menschliche Kolonkarzinomazellen, ATCC CCL-247; DU 145, hormonunabhängige menschliche Prostatakarzinomzellen, ATCC HTB-81; MaTu-MDR, hormonunabhängige, multiple Arzneimittel resistente menschliche Mammakarzinomzellen, EPO-GmbH, Berlin) wurden in einer Dichte von ca. 5000 Zellen/Messpunkt, je nach Wachstumsgeschwindigkeit der jeweiligen Zellen in einer 96-Loch Multititerplatte in 200 µl des entsprechenden Wachstumsmediums ausplattiert. Nach 24 Stunden wurden die Zellen einer Platte (Nullpunkt-Platte) mit Kristallviolett gefärbt (s.u.), während das Medium der anderen Platten durch frisches Kulturmedium (200 µl), dem die Testsubstanzen in verschiedenen Konzentrationen (0 µM, sowie im Bereich 0,01 - 30 µM; die finale Konzentration des Lösungsmittels Dimethylsulfoxid betrug 0,5%) zugesetzt waren, ersetzt. Die Zellen wurden für 4 Tage in Anwesenheit der Testsubstanzen inkubiert. Die Zellproliferation wurde durch Färbung der Zellen mit Kristallviolett bestimmt: Die Zellen wurden durch Zugabe von 20 µl/Messpunkt einer 11 %igen Glutaraldehyd-Lösung 15 min bei Raumtemperatur fixiert. Nach dreimaligem Waschen der fixierten Zellen mit Wasser wurden die Platten bei Raumtemperatur getrocknet. Die Zellen wurden durch Zugabe von 100 µl/Messpunkt einer 0,1 %igen Kristallviolett-Lösung (pH durch Zugabe von Essigsäure auf pH3 eingestellt) gefärbt. Nach dreimaligem Waschen der gefärbten Zellen mit Wasser wurden die Platten bei Raumtemperatur getrocknet. Der Farbstoff wurde durch Zugabe von 100 µl/Messpunkt einer 10%igen Essigsäure-Lösung gelöst. Die Extinktion wurde photometrisch bei einer Wellenlänge von 595 nm bestimmt. Die prozentuale Änderung des Zellwachstums wurde durch Normalisierung der Messwerte auf die Extinktionwerte der Nullpunktplatte (=0%) und die Extinktion der unbehandelten (0 µM) Zellen (=100%) berechnet.

Die Ergebnisse aus den Beispielen sind in den nachfolgenden Tabellen angegeben.

**Tabelle I**

| **Beispiel-Nr.** | **CDK2/CycE IC**_{**50**} **[nM]** | **CDK1/CycB IC**_{**50**} **[nM]** | **VEGF-R2 IC**_{**50**} **[nM]** | **MCF7 IC**_{**50**} **[µM]** |
|---|---|---|---|---|
| 1.0 | 420 | 200 | | 1,1 |
| 1.1 | 140 | 20 | 40 | 0,2 |
| 1.2 | 510 | 40 | | 2,6 |
| 1.3 | 23 | 28 | <10 | 1,0 |
| 1.3 (+)Enantiomer | 23 | | 69 | 0,9 |
| 1.4 | | | 11 | 0.4 |
| 1.5 | | 28 | 69 | 0,9 |
| 3.0 | 160 | 50 | 89 | 1,6 |
| 5.0 | | 300 | | |
| 8.0 | 130 | 80 | 140 | 1.3 |
| 10.0 | 120 | 360 | | 6 |
| 14.4 | 250 | 1700 | | 4,0 |
| 14.15 | 130 | 1500 | | 1,3 |
| 14.6 | 320 | 90 | | 0,7 |
| 14.8 | | 200 | | |
| 14.10 | 65 | | 190 | 1,9 |
| 14.11 | 2400 | 200 | | 0,75 |
| 14.16 | 4400 | 300 | | >10 |
| 14.18 | 30 | 80 | 370 | 2,1 |

Aus den Ergebnissen der Tabelle ist deutlich ersichtlich, dass sich die erfindungsgemäßen makrozyklischen Pyrimidine als CDK- und VEGF-Rezeptor-Inhibitoren oder CDK1- oder CDK2-Inhibitoren oder als VEGF-Rezeptor-Inhibitoren auszeichnen. Die Aktivität gegenüber CDK1 und/oder CDK2 und/oder VEGF erklärt u.a. die zelluläre Wirkung der Substanzen. Damit sind die makrozyklischen Verbindungen den bisher bekannten Verbindungen deutlich überlegen.

**Tabelle II**

| **Beispiel-Nr.** | **Inhibition IC**_{**50**} **[nM]** | **Proliferation IC**_{**50**} **[µM]** | | | | |
|---|---|---|---|---|---|---|
| | **CDK2/CycE** | **MCF7** | **H460** | **HCT116** | **DU145** | **MaTu-ADR** |
| 1.0 | 420 | 1,1 | 1,8 | 1,3 | 2,0 | 0,7 |
| 1.1 | 140 | 0,2 | 0,3 | 0,2 | 2,2 | 0,12 |
| 1.3 | 23 | 1,0 | 1,7 | 0,9 | 2,9 | 2,8 |
| 1.4 | | 0,4 | 0,2 | <0,1 | 0,7 | 0,1 |
| 1.5 | | 0,9 | 0,6 | 0,4 | 1,3 | 0,6 |
| 7.1 | 2400 | 4 | | | | |
| 8.0 | 130 | 1,3 | 0,5 | 0,4 | 0,5 | 0,4 |
| 13.0 A | 7000 | 30 | | | | |
| 13.0 B | >10000 | | | | | |
| 14.2 | 5000 | | | | | |
| 14.4 | 2000 | | | | | |
| 14.6 | 320 | 0,7 | 0,7 | 0,5 | 1,7 | 0,3 |
| 14.10 | | 0,3 | 0,1 | 0,1 | 0,4 | |
| 14.11 | 2400 | 0,75 | 0,9 | 0,7 | 0,9 | 1,3 |
| 14.15 | 1500 | 1,3 | 1,2 | 1,0 | 1,2 | 1,3 |
| 1.3 (+)Enantiomer | 23 | 0,9 | 2,1 | 1,4 | 4 | 4 |

## Patentansprüche

1. Verbindungen
- 1⁵-Brom-4-thia-2,5,11-triaza-1(2,4)-pyrimidina-3(1,3)-benzenacycloundecaphane 4,4-dioxide
- 1⁵-Bromo-4-thia-2,5,9-triaza-1(2,4)-pyrimidina-3(1,3)-benzenacyclononaphane-4,4-dioxide
- *rac*-1⁵-Bromo-4-thia-2,5,9-triaza-1(2,4)-pyrimidina-3(1,3)-benzenacyclononaphan-7-ol-4,4-dioxide
- *rac*-1⁵- Bromo-4-thia-2,5,9-triaza-1(2,4)-pyrimidina-3(1,3)-benzenacyclonaphane-8-methanol-4,4-dioxide hydrochloride
- 1⁵-Bromo-4-thia-2,5,9-triaza-1(2,4)-pyrimidina-3(1,3)-benzenacyclononaphan-3⁵⁻amine- 4,4-dioxide
- 1⁵-Bromo-3⁵-nitro-4-thia-2,5,9-triaza-1(2,4)-pyrimidina-3(1,3)-benzenacyclononaphane- 4,4-dioxide hydrochloride
- 1⁵-Bromo-5-methyl-4-thia-2,5,9-triaza-1(2,4)-pyrimidina-3(1,3)-benzenacyclononaphane- 4,4-dioxide
- 1⁵-Bromo-9-oxa-4-thia-2,5-diaza-1(2,4)-pyrimidina-3(1,3)-benzenacyclononaphane-4,4-dioxide
- *N*-tert-Butyl-4-thia-2,5,9-triaza-1(2,4)-pyrimidina-3(1,3)-benzenacyclononaphan-1⁵-carboxamid-4,4-dioxid
- 1⁵-Cyano-4-thia-2,5,9-triaza-1(2,4)-pyrimidina-3(1,3)-benzenacyclononaphan 4,4-dioxide
- 1⁵-Bromo-4-thia-2,5,8-triaza-1(2,4)-pyrimidina-3(4,2)-thiophenacyclooctaphan-4,4-dioxide
- 1⁵-Bromo-N,N'-dimethyl-4-oxa-2,9-diaza-1(2,4)-pyrimidina-3(1,3)-benzenacyclononaphan-3⁴,3⁶-disulfonamide
- 1⁵-Bromo-4-oxa-2,9-diaza-1(2,4)-pyrimidina-3(1,3)-benzenacyclononaphane
- 1⁵-Bromo-4-oxa-2,9-diaza-1(2,4)-pyrimidina-3(1,3)-benzenacyclononaphane-3⁴⁻sulfonamide
- 1⁵-Bromo-*N*-(dimethylaminomethylen)-4-oxa-2,9-diaza-1(2,4)-pyrimidiria-3(1,3)-benzenacyclononaphane-3⁴-sulfonamide
- (S)-1⁵-Bromo-8-(hydroxymethyl)-4-oxa-2,9-diaza-1(2,4)-pyrimidina-3(1,3)-benzenacyclononaphane-3(4)-sulfonamide
- 1⁵-Bromo-4-oxa-2,8-diaza-1(2,4)-pyrimidina-3(1,2)-benzenacyclooctaphane
- 1⁵-Bromo-4-oxa-2,8-diaza-1(2,4)-pyrimidina-3(1,2)-benzenacyclooctaphane-3⁴⁻sulfonamide
- 1⁵-Bromo-2,5,10-triaza-1(2,4)-pyrimidina-3(1,3)-benzenacyclodecaphan-4-one
- 1⁵-Bromo-2,4,6,10-tetraaza-1(2,4)-pyrimidina-3(1,3)-benzenacyclodecaphan-5-one
- 1⁵-Bromo-4-oxa-2,9-diaza-1(2,4)-pyrimidina-3(1,3)-benzen-acyclononaphane
- 1⁵-Bromo-2,5,11-triaza-1(2,4)-pyrimidine-3(1,3)-benzenacycloundecaphane-4-one
- 2,5,11-Triaza-1(2,4)-pyrimidina-3(1,3)-benzenacycloundecaphane-4-one
- 1⁵ -Bromo-4-mesyl-2,4,9-triaza-1(2,4)-pyrimidina-3(1,3)-benzenacyclononaphane

2. Verbindungen

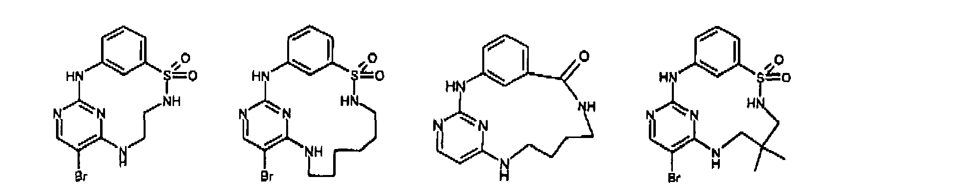

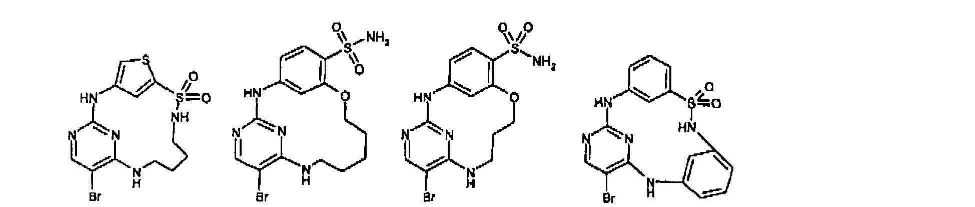

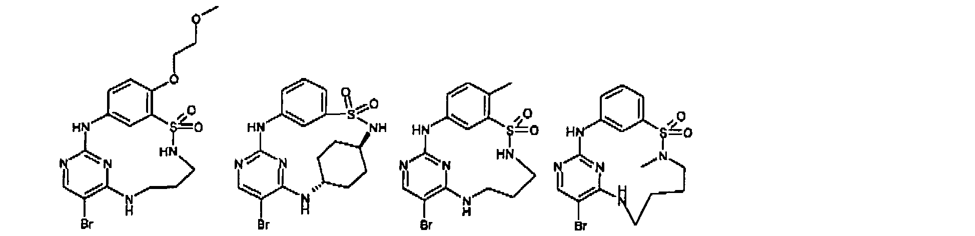

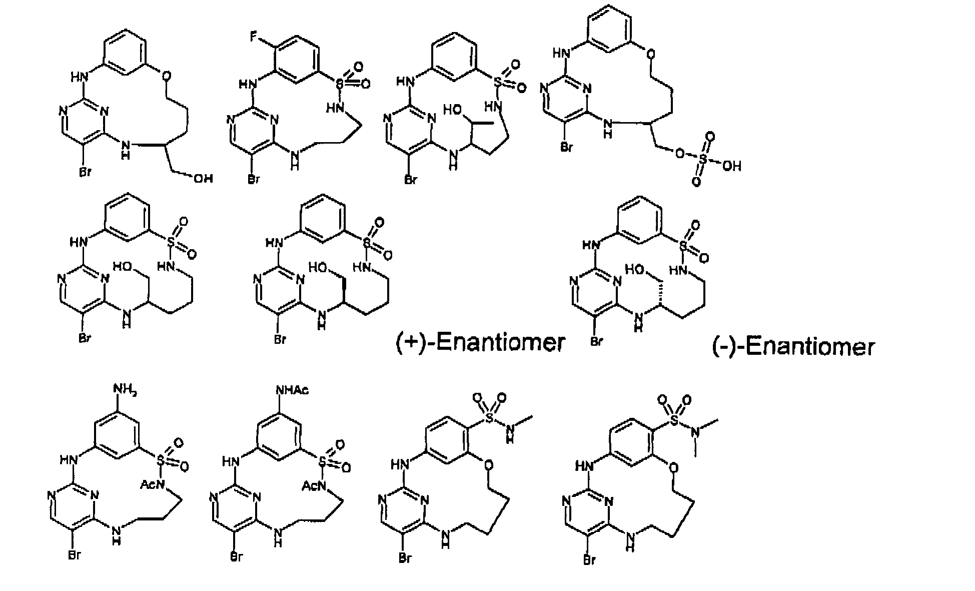

3. Verbindungen

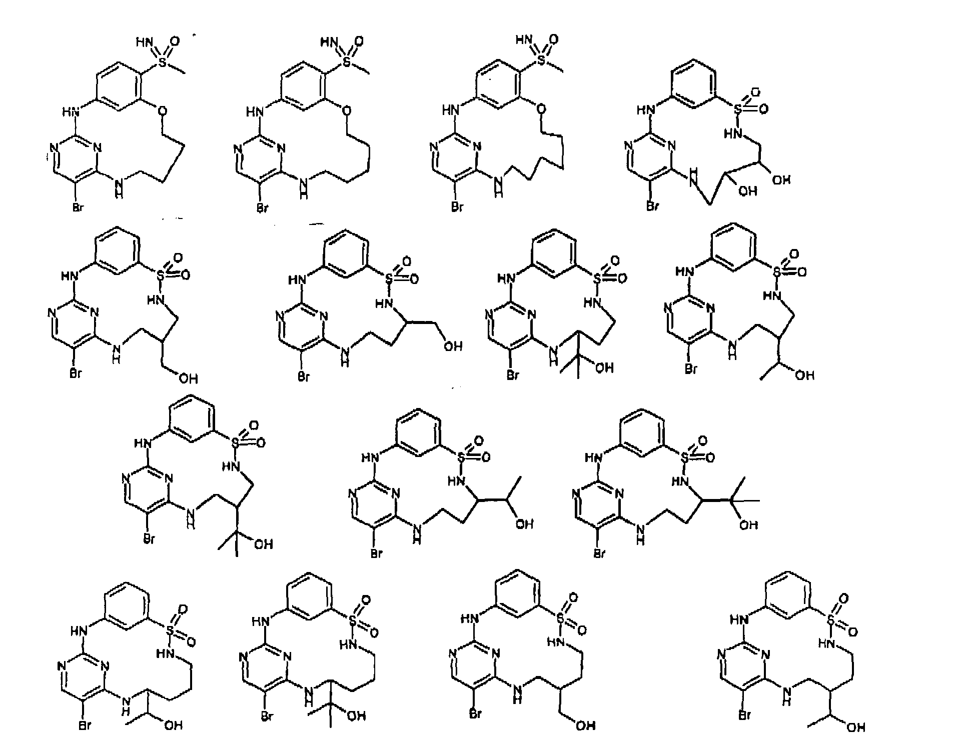

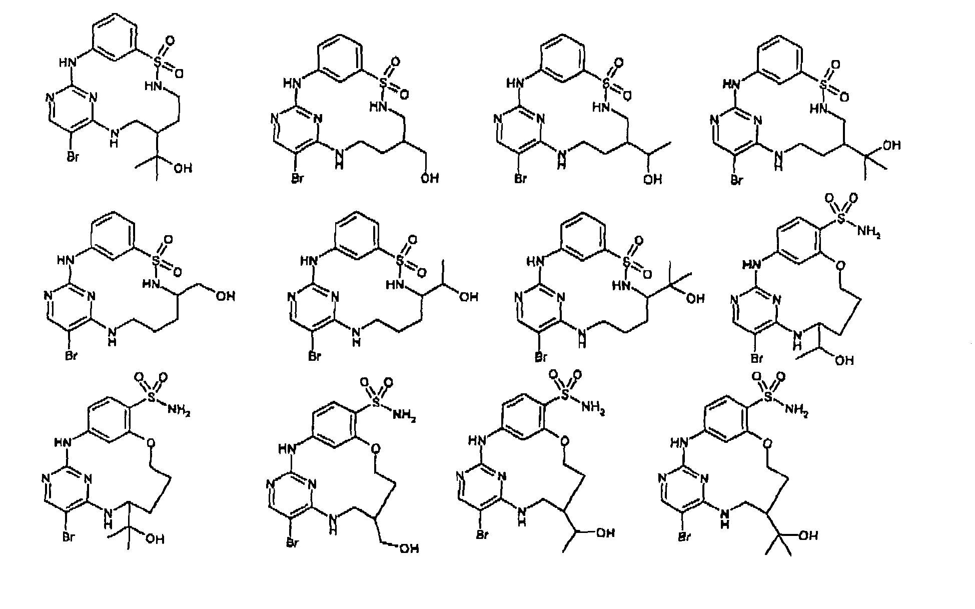

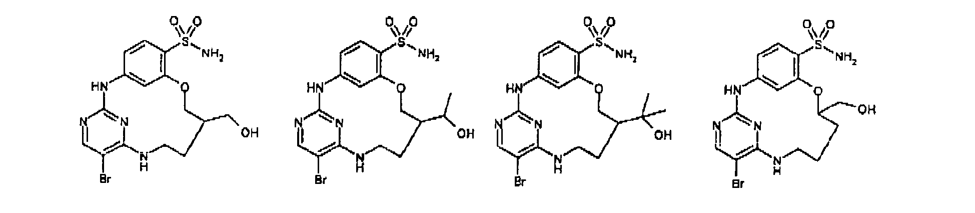

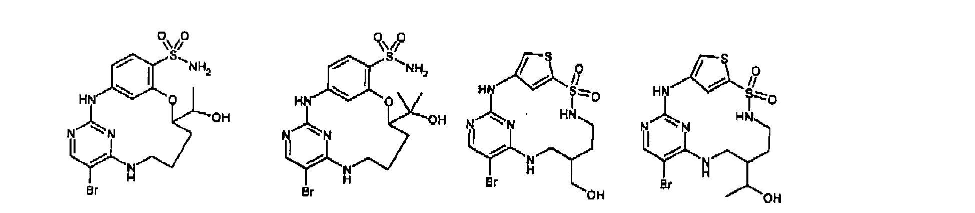

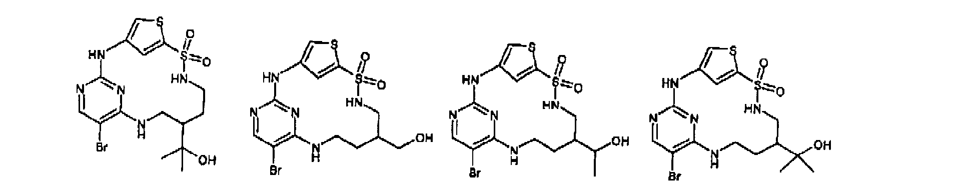

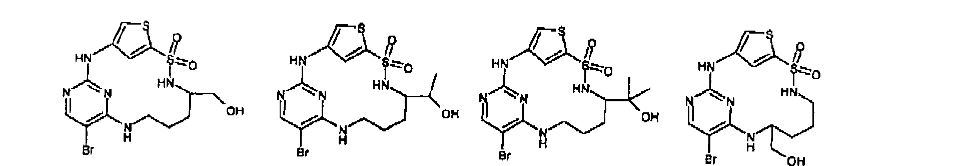

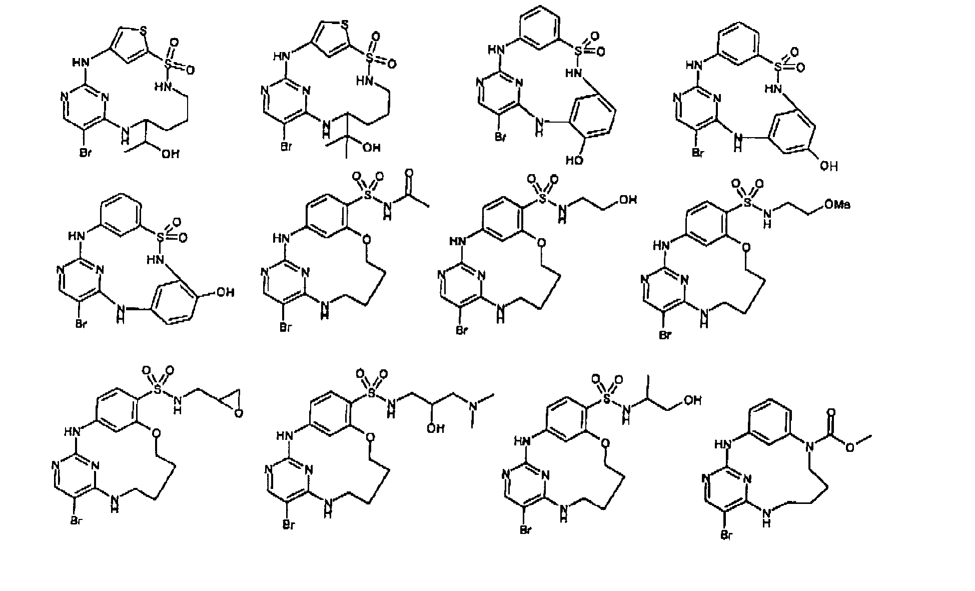

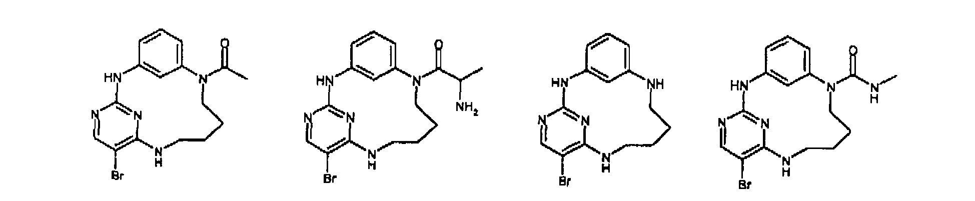

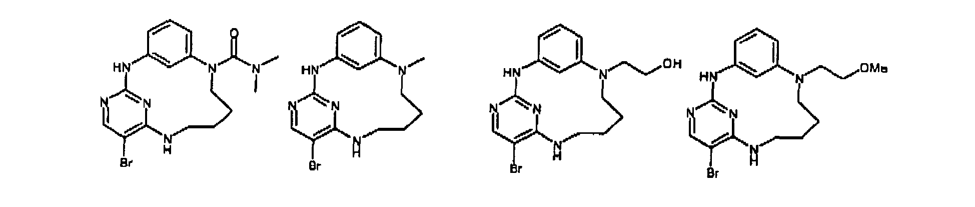

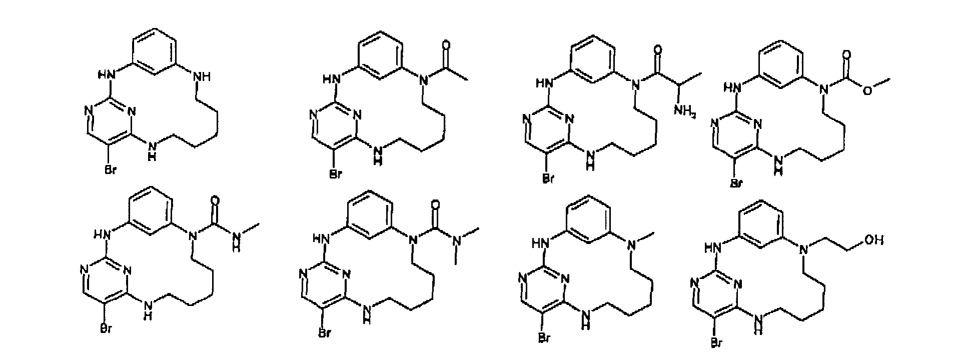

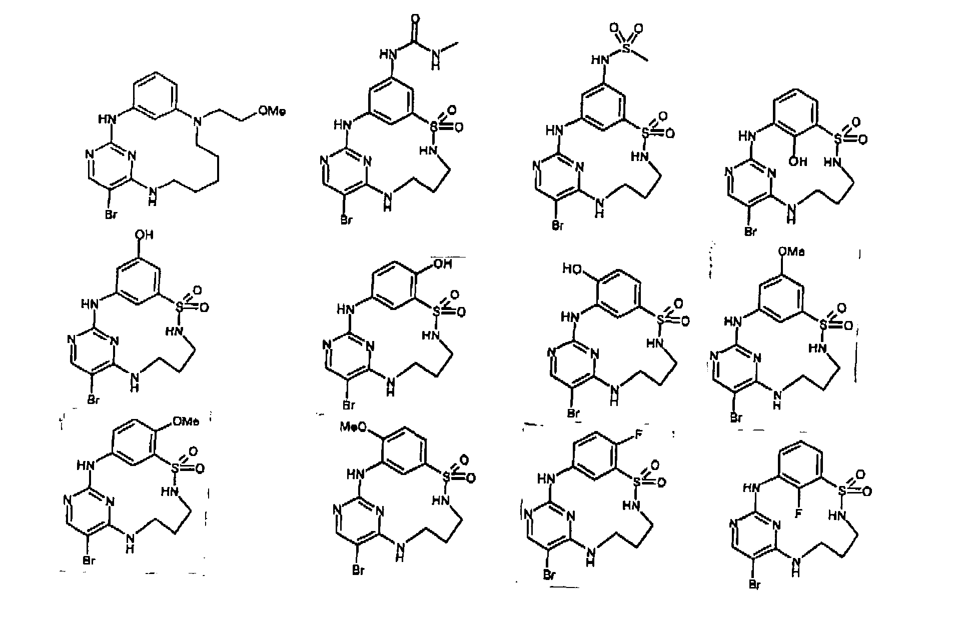

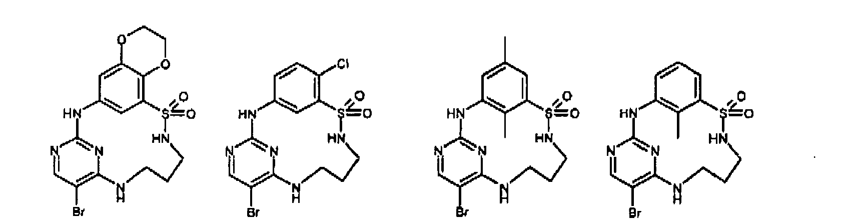

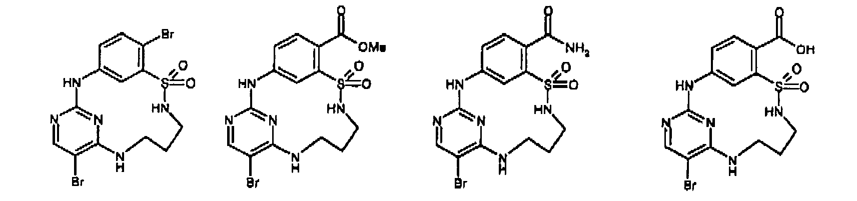

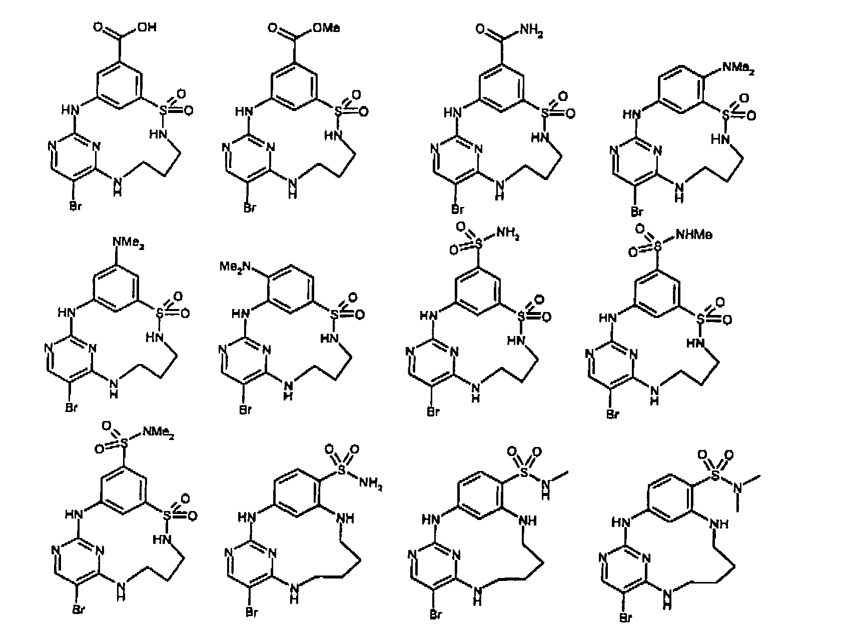

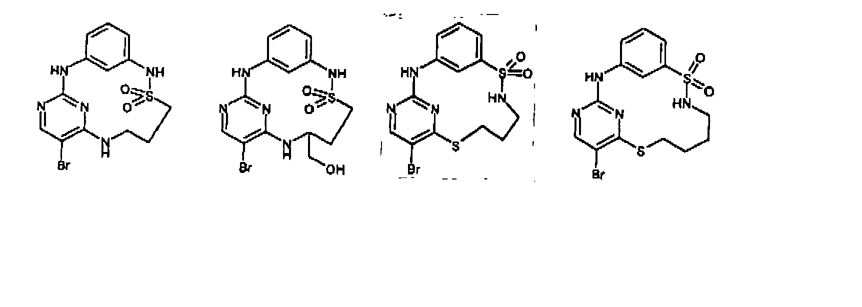

4. Verwendung einer Verbindungen gemäß einem der Ansprüche 1 bis 3 zur Herstellung eines Arzneimittels.

5. Verwendung einer Verbindungen gemäß einem der Ansprüche 1 bis 3 zur Herstellung eines Arzneimittels zur Behandlung von Krebs, Angiofribroma, Arthritis, Augenerkrankungen, Autoimmunerkrankungen, Chemotherapeutika-induzferter Alopezie und Mukositis, Crohn-Krankheit, Endometriose, fibrotische Erkrankungen, Hämangioma, kardiovaskulären Erkrankungen, infektiösen Erkrankungen, nephrologischen Erkrankungen, chronischen und akuten neurodegenerativen Erkrankungen, sowie von Verletzungen des Nervengewebes, viralen Infektionen, zur Hemmung der Reocclusion von Gefäßen nach Ballonkatheterbehandlung, bei der Gefäßprothetik oder nach dem Einsetzen von mechanischen Vorrichtungen zum Offenhalten von Gefäßen, wie z. B. Stents, als Immunsuppressiva, zur Unterstützung der narbenfreien Wundheilung, bei Altersflecken und bei Kontaktdermatitis.

6. Verwendung gemäß Anspruch 5, **dadurch gekennzeichnet, dass**
unter Krebs solide Tumoren, Tumor- oder Metastasenwachstum, Kaposis Sarkom, Morbus Hodgkin und Leukämie,
unter Arthritis, rheumatoide Arthritis, unter Augenerkrankungen, diabetische Retinopathie, Neovaskulares Glaukom,
unter Autoimmunerkrankungen Psoriasis, Alopezie und Multiple Sklerose,
unter fibrotische Erkrankungen, Leberzirrhose, mesangialzellproliferative Erkrankungen, Arteriosklerose,
unter infektiösen Erkrankungen durch unizelluläre Parasiten hervorgerufene Erkrankungen, unter kardiovaskulären Erkrankungen Stenosen, wie z. B. Stent-induzierte Restenose, Arteriosklerosen und Restenosen,
unter nephrologischen Erkrankungen Glomerulonephritis, diabetische Nephropatie, maligne Nephrosklerose, thrombische mikroangiopatische Syndrome, Transplantationsabstoßungen und Glomerulopathie,
unter chronisch neurodegenerativen Erkrankungen Huntington's Erkrankung, amyotrophe Lateralsklerose, Parkinsonsche Erkrankung, AIDS Dementia und Alzheimer'sche Erkrankung, unter akut neurodegenerativen Erkrankungen Ischämien des Gehirns und Neurotraumata, und unter viralen Infektionen Cytomegalus-Infektionen, Herpes, Hepatitis B oder C, und HIV Erkrankungen zu verstehen sind.

7. Arzneimittel, die mindestens eine Verbindung gemäß einem der Ansprüche 1 bis 3 enthalten.

8. Arzneimittel gemäß Anspruch 7, zur Behandlung Krebs, Angiofribroma, Arthritis, Augenerkrankungen, Autoimmunerkrankungen, Chemotherapeutika-induzierterAiopezie und Mukositis, Crohn-Krankheit, Endometriose, fibrotische Erkrankungen, Hämangioma, kardlovaskulären Erkrankungen, infektiösen Erkrankungen, nephrologischen Erkrankungen, chronischen und akuten neurodegenerativen Erkrankungen, sowie von Verletzungen des Nervengewebes, und viralen Infektionen und zur Hemmung der Reocclusion von Gefäßen nach Ballonkatheterbehandlung, bei der Gefäßprothetik oder nach dem Einsetzen von mechanischen Vorrichtungen zum Offenhalten von Gefäßen, wie z. B. Stents, und als immunsuppressiva, und zur Unterstützung der narbenfreien Wundheilung, und bei Altersflecken und bei Kontaktdermatitis.

9. Arzneimittel zur Verwendung gemäß Anspruch 8, wobei unter Krebs solide Tumoren, Tumor- oder Metastasenwachstum, Kaposis Sarkom, Morbus Hodgkin und Leukämie,
unter Arthritis, rheumatoide Arthritis, unter Augenerkrankungen, diabetische Retinopathie, Neovaskulares Glaukom,
unter Autoimmunerkrankungen Psoriasis, Alopezie und Multiple Sklerose,
unter fibrotische Erkrankungen, Leberzirrhose, mesangialzellproliferative Erkrankungen, Arteriosklerose,
unter infektiösen Erkrankungen durch unizelluläre Parasiten hervorgerufene Erkrankungen, unter kardiovaskulären Erkrankungen Stenosen, wie z. B. Stent-induzierte Restenose, Arteriosklerosen und Restenosen,
unter nephrologischen Erkrankungen Glomerulonephritis, diabetische Nephropatie, maligne Nephrosklerose, thrombische mikroangiopatische Syndrome, Transplantationsabstoßungen und Glomerulopathie,
unter chronisch neurodegenerativen Erkrankungen Huntington's Erkrankung, amyotrophe Lateralsklerose, Parkinsonsche Erkrankung, AIDS Dementia und Alzheimer'sche Erkrankung, unter akut neurodegenerativen Erkrankungen Ischämien des Gehirns und Neurotraumata, und unter viralen Infektionen Cytomegalus-Infektionen, Herpes, Hepatitis B oder C, und HIV Erkrankungen zu verstehen sind.

## Claims

1. Compounds
- 1⁵-bromo-4-thia-2,5,11-triaza-1(2,4)-pyrimidina-3(1,3)-benzenacycloundecaphane 4,4-dioxide
- 1⁵-bromo-4-thia-2,5,9-triaza-1(2,4)-pyrimidina-3(1,3)-benzenacyclononaphane 4,4-dioxide
- *rac*-1⁵-bromo-4-thia-2,5,9-triaza-1(2,4)-pyrimidina-3(1,3)-benzenacyclononaphan-7-ol 4,4-dioxide
- *rac*-1⁵-bromo-4-thia-2,5,9-triaza-1(2,4)-pyrimidina-3(1,3)-benzenacyclononaphane-8-methanol 4,4-dioxide hydrochloride
- 1⁵-bromo-4-thia-2,5,9-triaza-1(2,4)-pyrimidina-3(1,3)-benzenacyclononaphane-3⁵-amine 4,4-dioxide
- 1⁵-bromo-3⁵-nitro-4-thia-2,5,9-triaza-1(2,4)-pyrimidina-3(1,3)-benzenacyclononaphane-4,4-dioxide hydrochloride
- 1⁵-bromo-5-methyl-4-thia-2,5,9-triaza-1(2,4)-pyrimidina-3(1,3)-benzenacyclononaphane 4,4-dioxide
- 1⁵-bromo-9-oxa-4-thia-2,5-diaza-1(2,4)-pyrimidina-3(1,3)-benzenacyclononaphane 4,4-dioxide
- *N*-tert-butyl-4-thia-2,5,9-triaza-1(2,4)-pyrimidina-3(1,3)-benzenacyclononaphane-1⁵-carboxamide 4,4-dioxide
- 1⁵-cyano-4-thia-2,5,9-triaza-1(2,4)-pyrimidina-3(1,3)-benzenacyclononaphane 4,4-dioxide
- 1⁵-bromo-4-thia-2,5,8-triaza-1(2,4)-pyrimidina-3(4,2)-thiophenacyclooctaphane 4,4-dioxide
- 1⁵-bromo-N,N'-dimethyl-4-oxa-2,9-diaza-1(2,4)-pyrimidina-3(1,3)-benzenacyclononaphane-3⁴,3⁶-di-sulphonamide
- 1⁵-bromo-4-oxa-2,9-diaza-1(2,4)-pyrimidina-3(1,3)-benzenacyclononaphane
- 1⁵-bromo-4-oxa-2,9-diaza-1(2,4)-pyrimidina-3(1,3)-benzenacyclononaphane-3⁴-sulphonamide
- 1⁵-bromo-*N*-(dimethylaminomethylene)-4-oxa-2,9-diaza-1(2,4)-pyrimidina-3(1,3)-benzenacyclononaphane-3⁴⁻sulphonamide
- (S)-1⁵-bromo-8- (hydroxymethyl)-4-oxa-2,9-diaza-1(2,4)-pyrimidina-3(1,3)-benzenacyclononaphane-3(4)-sulphonamide
- 1⁵-bromo-4-oxa-2,8-diaza-1(2,4)-pyrimidina-3(1,2)-benzenacyclooctaphane
- 1⁵-bromo-4-oxa-2,8-diaza-1(2,4)-pyrimidina-3(1,2)-benzenacyclooctaphane-3⁴-sulphonamide
- 1⁵-bromo-2,5,10-triaza-1(2,4)-pyrimidina-3(1,3)-benzenacyclodecaphan-4-one
- 1⁵-bromo-2,4,6,10-tetraaza-1(2,4)-pyrimidina-3(1,3)-benzenacyclodecaphan-4-one
- 1⁵-bromo-4-oxa-2,9-diaza-1(2,4)-pyrimidina-3(1,3)-benzenacyclononaphane
- 1⁵-bromo-2,5,11-triaza-1(2,4)-pyrimidina-3(1,3)-benzenacycloundecaphan-4-one
- 2,5,11-triaza-1(2,4)-pyrimidina-3(1,3)-benzenacycloundecaphan-4-one
- 1⁵-bromo-4-mesyl-2,4,9-triaza-1(2,4)-pyrimidina-3(1,3)-benzenacyclononaphane
and their diastereomers, enantiomers and salts.

2. Compounds 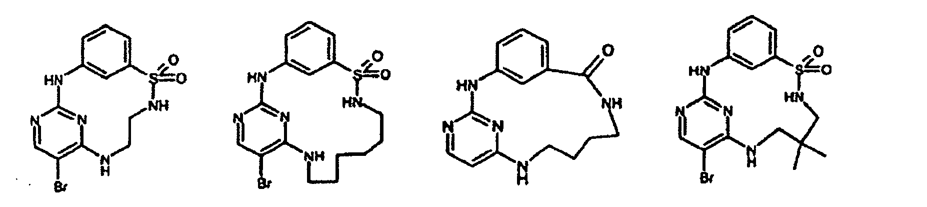 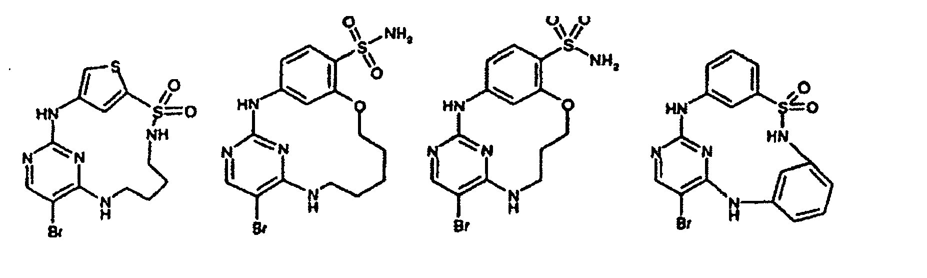 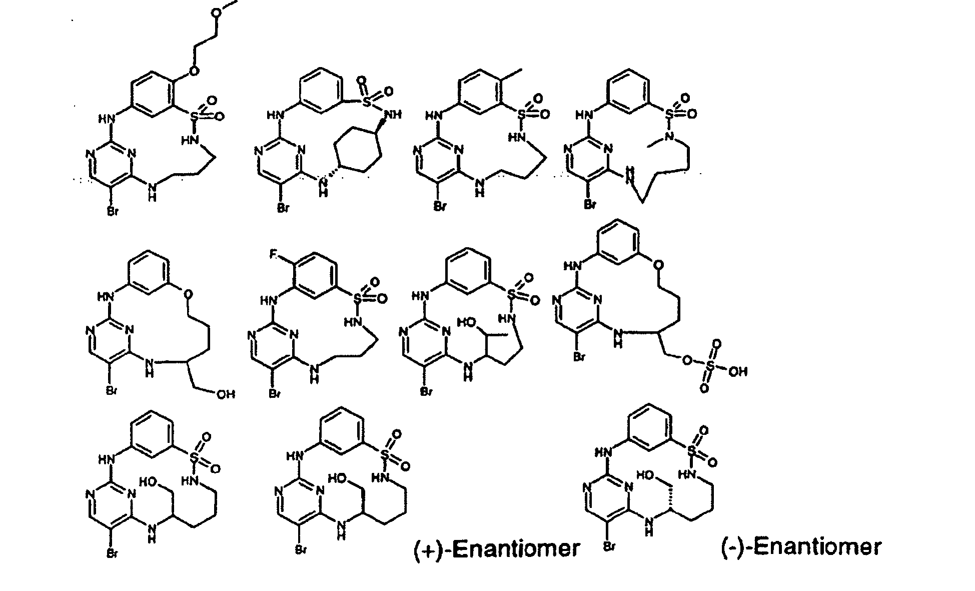 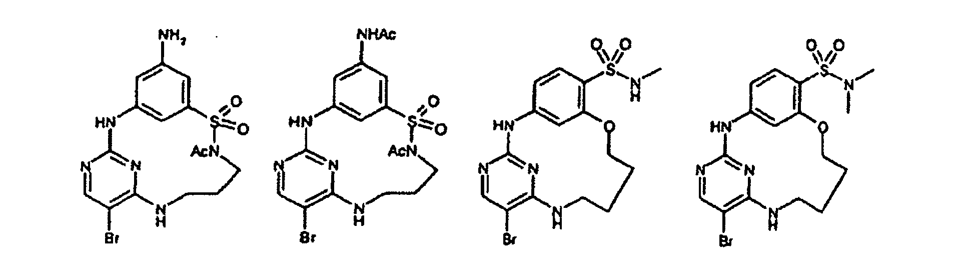 and their diastereomers, enantiomers and salts.

3. Compounds 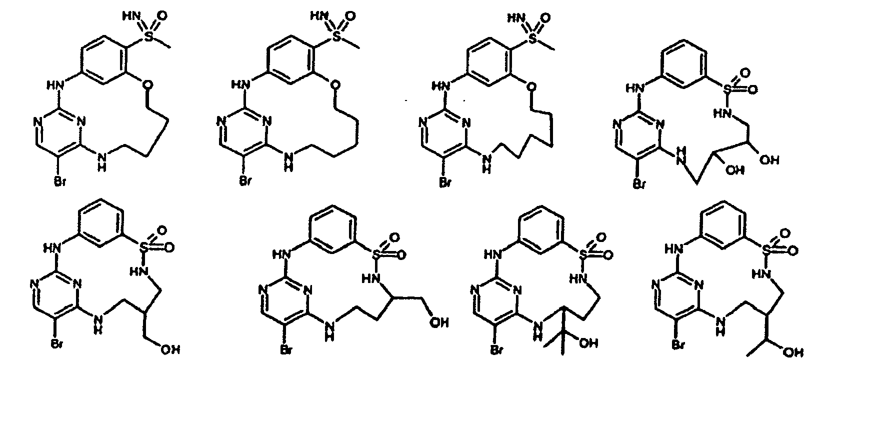 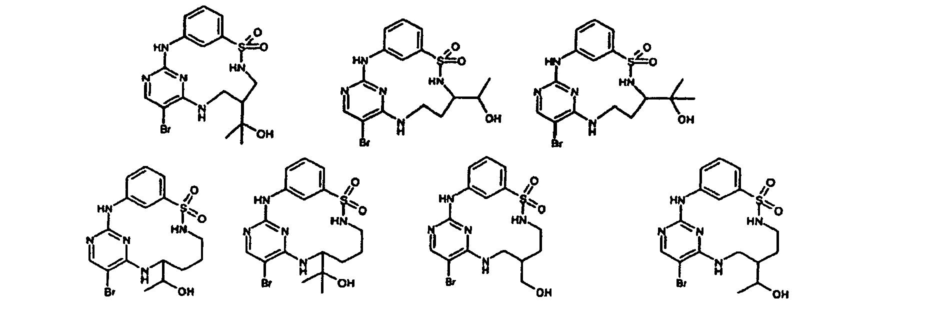 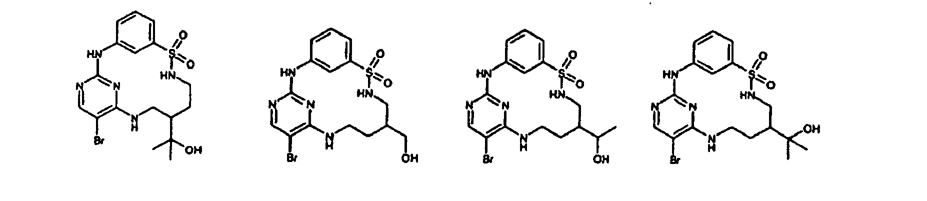 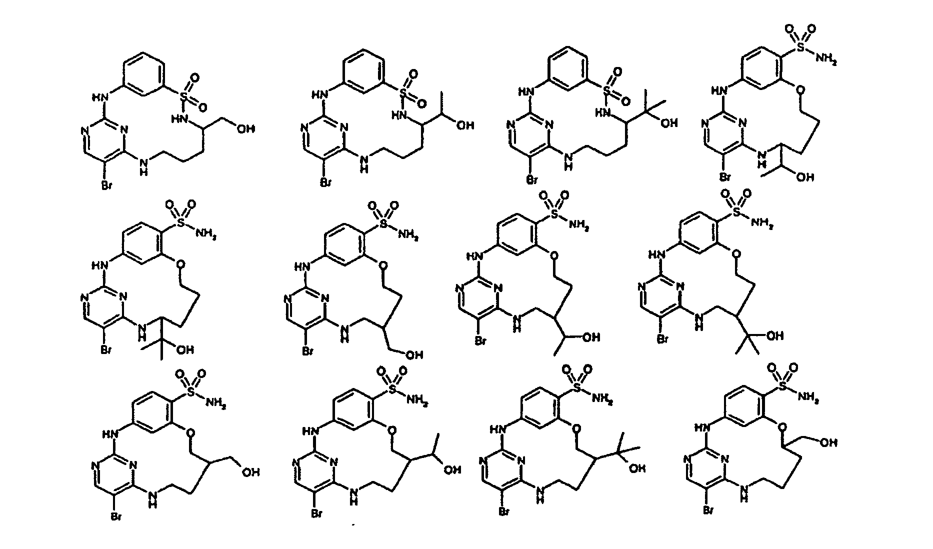 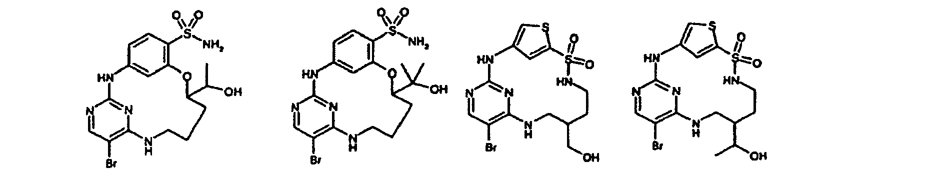 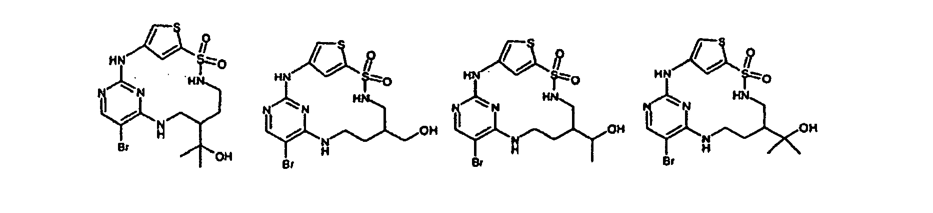 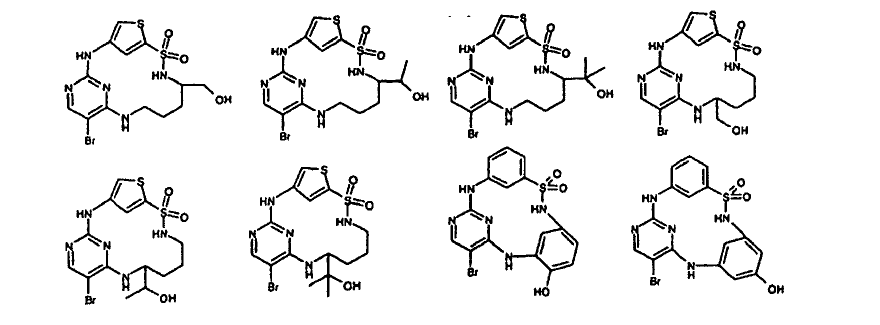 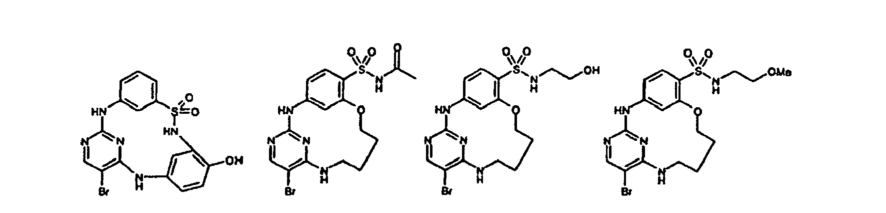 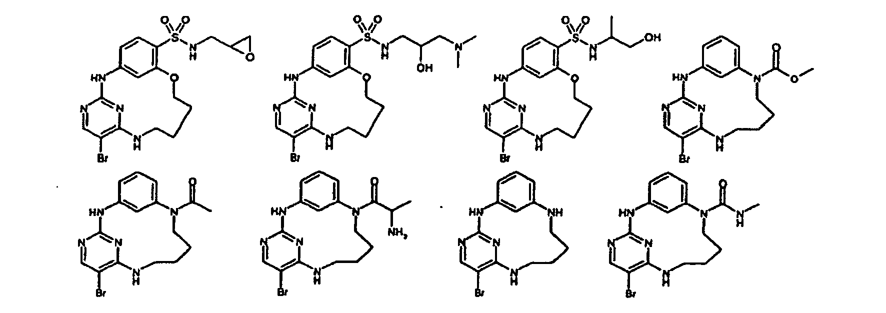 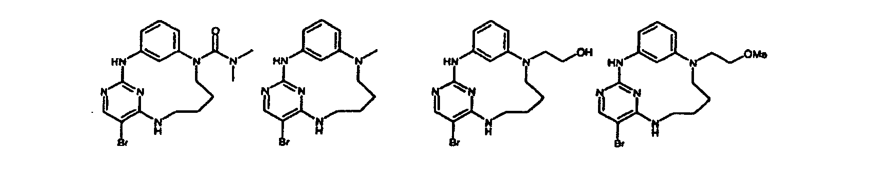 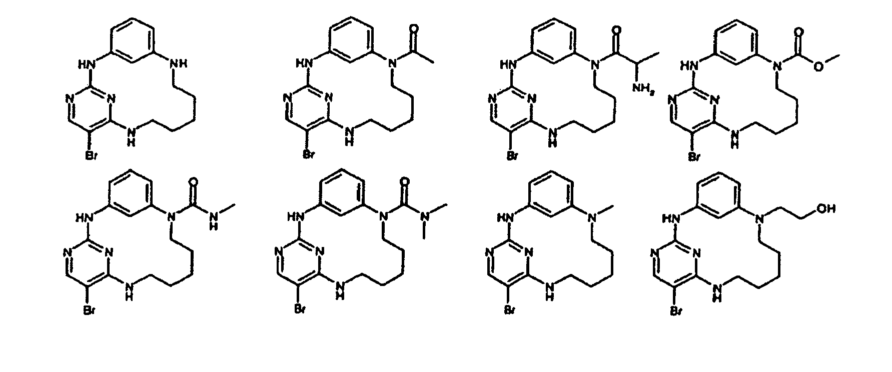 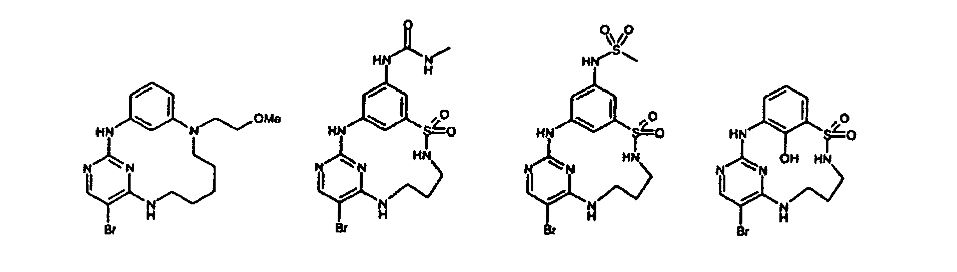 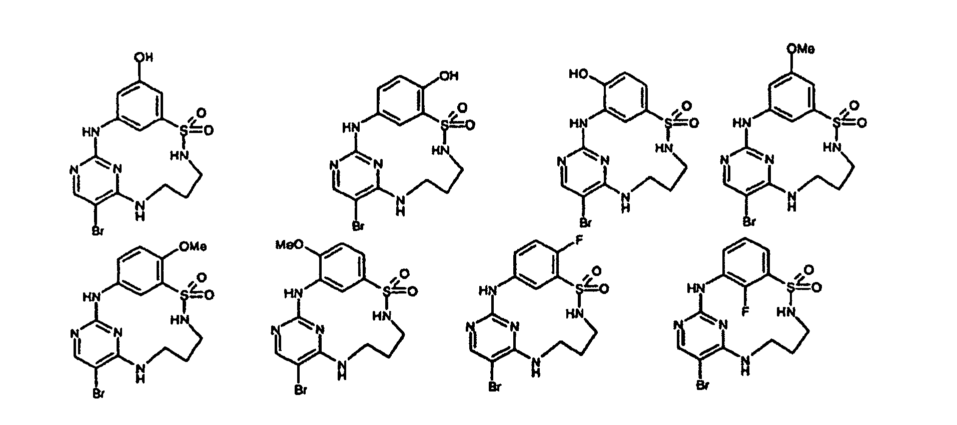 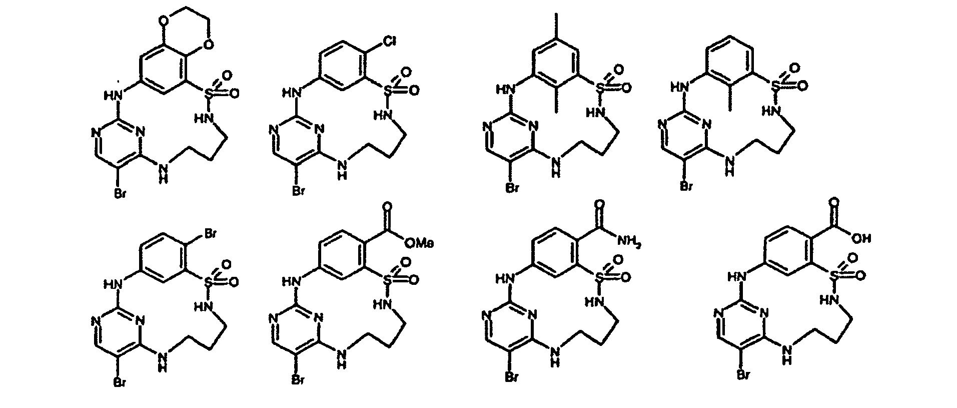 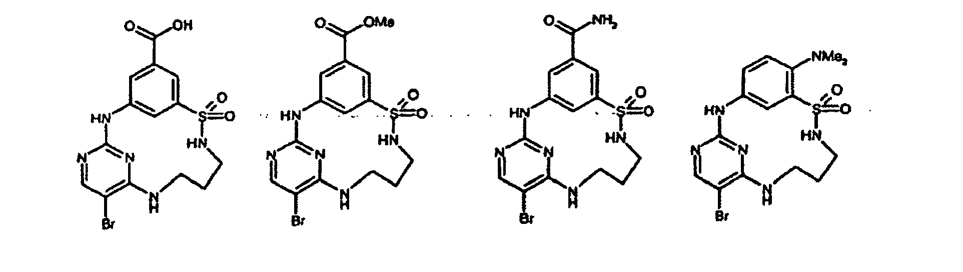 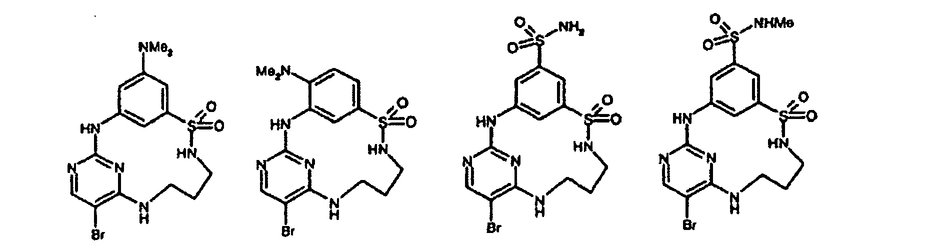 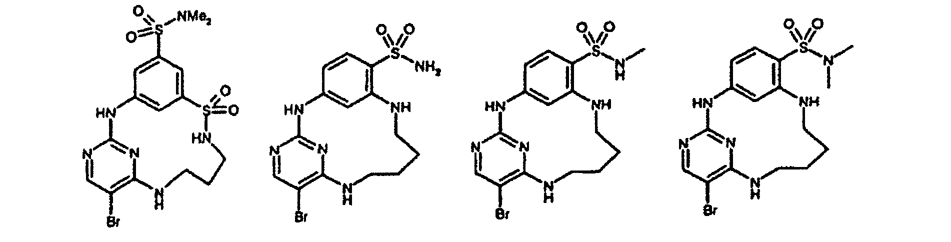 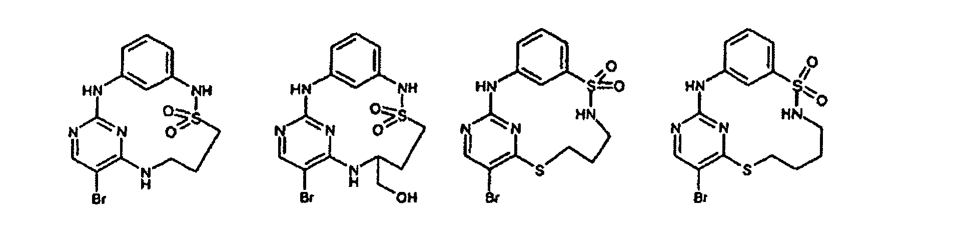 and their diastereomers, enantiomers and salts.

4. Use of a compound according to one of Claims 1 to 3 for the production of a medicament.

5. Use of a compound according to one of Claims 1 to 3 for the production of a medicament for the treatment of cancer, angiofibroma, arthritis, eye diseases, autoimmune diseases, chemotherapeutic-induced alopecia and mucositis, Crohn's disease, endometriosis, fibrotic diseases, haemangioma, cardiovascular diseases, infectious diseases, nephrological diseases, chronic and acute neurodegenerative diseases, and of injuries of the nervous tissue, viral infections, for the inhibition of the reocclusion of vessels after balloon catheter treatment, in vascular prosthetics or after the insertion of mechanical devices for keeping open vessels, such as, for example, stents, as immunosuppressives, for assisting scar-free wound healing, in age spots and in contact dermatitis.

6. Use according to Claim 5, **characterized in that** cancer is to be understood as meaning solid tumours, tumour or metastasis growth, Kaposi's sarcoma, Hodgkin's disease and leukaemia, arthritis is to be understood as meaning rheumatoid arthritis, eye diseases are to be understood as meaning diabetic retinopathy, neovascular glaucoma, autoimmune diseases are to be understood as meaning psoriasis, alopecia and multiple sclerosis,
fibrotic diseases are to be understood as meaning cirrhosis of the liver, mesangial cell proliferative diseases, arteriosclerosis, infectious diseases are to be understood as meaning diseases caused by unicellular parasites, cardiovascular diseases are to be understood as meaning stenoses, such as, for example, stent-induced restenosis, arteriosclerosis and restenoses,
nephrological diseases are to be understood as meaning glomerulonephritis, diabetic nephropathy, malignant nephrosclerosis, thrombic microangiopathic syndromes, transplantation rejections and glomerulopathy,
chronic neurodegenerative diseases are to be understood as meaning Huntington's disease, amyotropic lateral sclerosis, Parkinson's disease, AIDS dementia and Alzheimer's disease,
acute neurodegenerative diseases are to be understood as meaning ischaemias of the brain and neurotraumata,
and viral infections are to be understood as meaning cytomegalus infections, herpes, hepatitis B or C, and HIV diseases.

7. Medicaments which contain at least one compound according to one of Claims 1 to 3.

8. Medicaments according to Claim 7, for the treatment of cancer, angiofibroma, arthritis, eye diseases, autoimmune diseases, chemotherapeutic-induced alopecia and mucositis, Crohn's disease, endometriosis, fibrotic diseases, haemangioma, cardiovascular diseases, infectious diseases, nephrological diseases, chronic and acute neurodegenerative diseases, and of injuries of the nervous tissue, and viral infections and for the inhibition of the reocclusion of vessels after balloon catheter treatment, in vascular prosthetics or after the insertion of mechanical devices for keeping open vessels, such as, for example, stents, and as immunosuppressives, and for assisting scar-free wound healing, and in age spots and in contact dermatitis.

9. Medicaments for use according to Claim 8, where cancer is to be understood as meaning solid tumours, tumour or metastasis growth, Kaposi's sarcoma, Hodgkin's disease and leukaemia,
arthritis is to be understood as meaning rheumatoid arthritis, eye diseases are to be understood as meaning diabetic retinopathy, neovascular glaucoma,
autoimmune diseases are to be understood as meaning psoriasis, alopecia and multiple sclerosis,
fibrotic diseases are to be understood as meaning cirrhosis of the liver, mesangial cell proliferative diseases, arteriosclerosis, infectious diseases are to be understood as meaning diseases caused by unicellular parasites, cardiovascular diseases are to be understood as meaning stenoses, such as, for example, stent-induced restenosis, arteriosclerosis and restenoses,
nephrological diseases are to be understood as meaning glomerulonephritis, diabetic nephropathy, malignant nephrosclerosis, thrombic microangiopathic syndromes, transplantation rejections and glomerulopathy,
and chronic neurodegenerative diseases are to be understood as meaning Huntington's disease, amyotropic lateral sclerosis, Parkinson's disease, AIDS dementia and Alzheimer's disease,
acute neurodegenerative diseases are to be understood as meaning ischaemias of the brain and neurotraumata,
and viral infections are to be understood as meaning cytomegalus infections, herpes, hepatitis B or C, and HIV diseases.

## Revendications

1. Composés
- 1⁵-bromo-4-thia-2,5,11-triaza-1(2,4)-pyrimidina-3(1,3)-benzénacyclo-undécaphane-4,4-dioxyde
- 1⁵-bromo-4-thia-2,5,9-triaza-1(2,4)-pyrimidina-3(1,3)-benzénacyclonaphane-4,4-dioxyde
- *rac*-1⁵-bromo-4-thia-2,5,9-triaza-1(2,4)-pyrimidina-3(1,3)-benzénacyclononaphan-7-ol-4,4-dioxyde
- chlorhydrate de *rac*-1⁵-bromo-4-thia-2,5,9-triaza-1(2,4)-pyrimidina-3(1,3)-benzénacyclononaphan-8-méthanol-4,4-dioxyde
- 1⁵-bromo-4-thia-2,5,9-triaza-1(2,4)-pyrimidina-3(1,3)-benzénacyclononaphan-3⁵-amine-4,4-dioxyde
- chlorhydrate de 1⁵-bromo-3⁵-nitro-4-thia-2,5,9-triaza-1(2,4)-pyrimidina-3(1,3)-benzénacyclononaphane-4,4-dioxyde
- 1⁵-bromo-5-méthyl-4-thia-2,5,9-triaza-1(2,4)-pyrimidina-3(1,3)-benzénacyclononaphane-4,4-dioxyde
- 1⁵-bromo-9-oxa-4-thia-2,5-diaza-1(2,4)-pyrimidina-3(1,3)-benzénacyclononaphane-4,4-dioxyde
- N-tert-butyl-4-thia-2,5,9-triaza-1(2,4)-pyrimidina-3(1,3)-benzénacyclononaphane-15-carboxamide-4,4-dioxyde
- 1⁵-cyano-4-thia-2,5,9-triaza-1(2,4)-pyrimidina-3(1,3)-benzénacyclononaphan-4,4-dioxyde
- 1⁵-bromo-4-thia-2,5,8-triaza-1(2,4)-pyrimidina-3(4,2)-thiophénacyclooctaphane-4,4-dioxyde
- 1⁵-bromo-N,N'-diméthyl-4-oxa-2,9-diaza-1(2,4)-pyrimidina-3(1,3)-benzénacyclononaphane-3⁴,3⁶⁻disulfonamide
- 1⁵-bromo-4-oxa-2,9-diaza-1(2,4)-pyrimidina-3(1,3)-benzénacyclononaphane
- 1⁵-bromo-4-oxa-2,9-diaza-1(2,4)-pyrimidina-3(1,3)-benzénacyclononaphane-3⁴-sulfonamide
- 1⁵-bromo-*N*-(diméthylaminométhylène)-4-oxa-2,9-diaza-1(2,4)-pyrimidina-3(1,3)-benzénacyclononaphane-3⁴-sulfonamide
- (S)-1⁵-bromo-8-(hydroxyméthyl)-4-oxa-2,9-diaza-1(2,4)-pyrimidina-3(1,3)-benzénacyclononaphane-3(4)-sulfonamide
- 1⁵-bromo-4-oxa-2,8-diaza-1(2,4)-pyrimidina-3(1,2)-benzénacyclooctaphane
- 1⁵-bromo-4-oxa-2,8-diaza-1(2,4)-pyrimidina-3(1,2)-benzénacyclooctaphane-3⁴-sulfonamide
- 1⁵-bromo-2,5,10-triaza-1(2,4)-pyrimidina-3(1,3)-benzénacyclodécaphan-4-one
- 1⁵-bromo-2,4,6,10-tétraaza-1(2,4)-pyrimidina-3(1,3)-benzénacyclodécaphan-5-one
- 1⁵-bromo-4-oxa-2,9-diaza-1(2,4)-pyrimidina-3(1,3)-benzénacyclononaphane
- 1⁵-bromo-2,5,11-triaza-1(2,4)-pyrimidina-3(1,3)-benzénacyclo-undécaphan-4-one
- 2,5,11-triaza-1(2,4)-pyrimidina-3(1,3)-benzénacyclo-undécaphan-4-one
- 1⁵-bromo-4-mésyl-2,4,9-triaza-1(2,4)-pyrimidina-3(1,3)-benzénacyclononaphane
ainsi que leurs diastéréoisomères, leurs énantiomères et leurs sels.

2. Composés 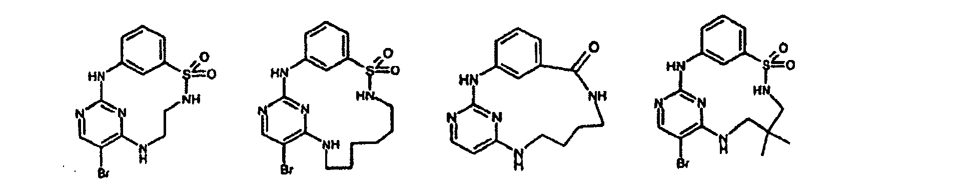 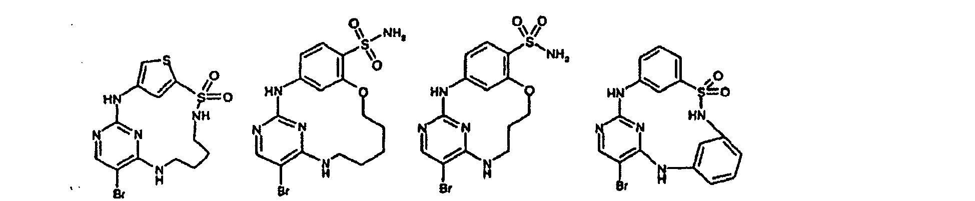 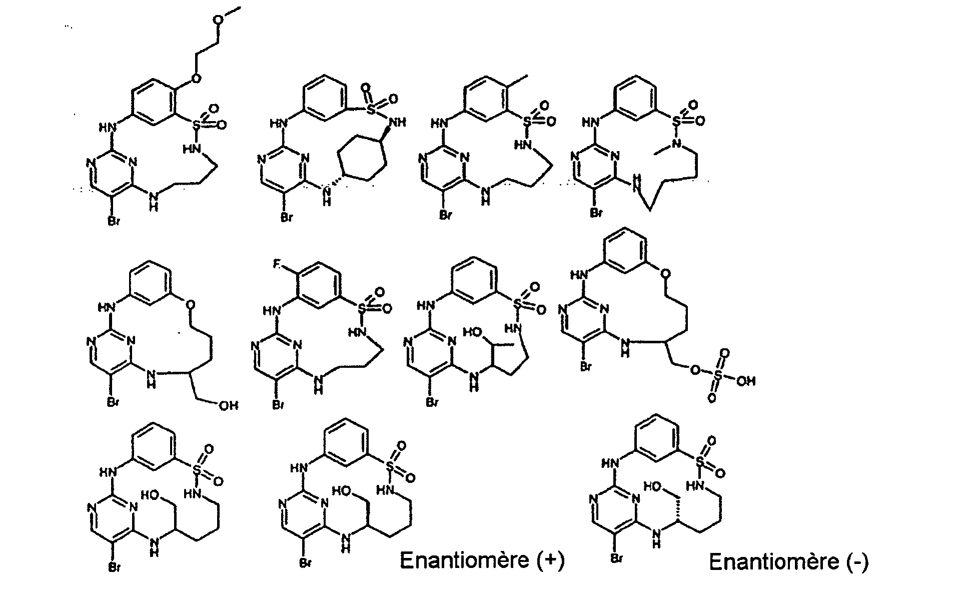 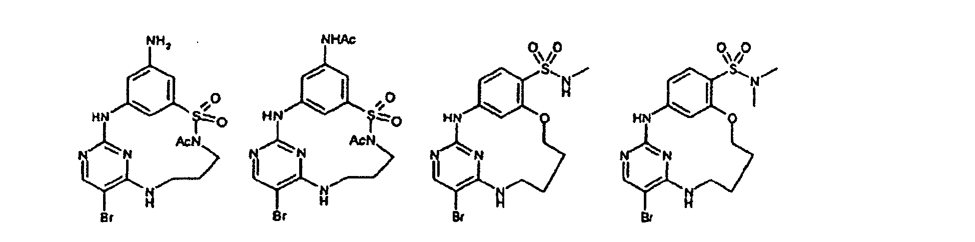 ainsi que leurs diastéréoisomères, leurs énantiomères et leurs sels.

3. Composés 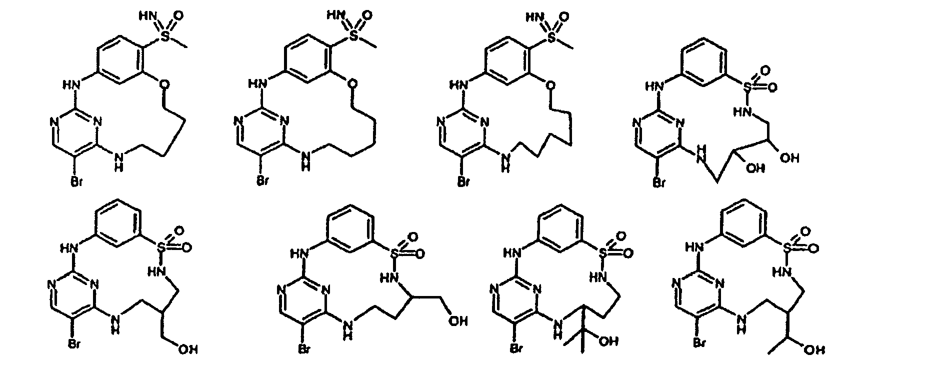 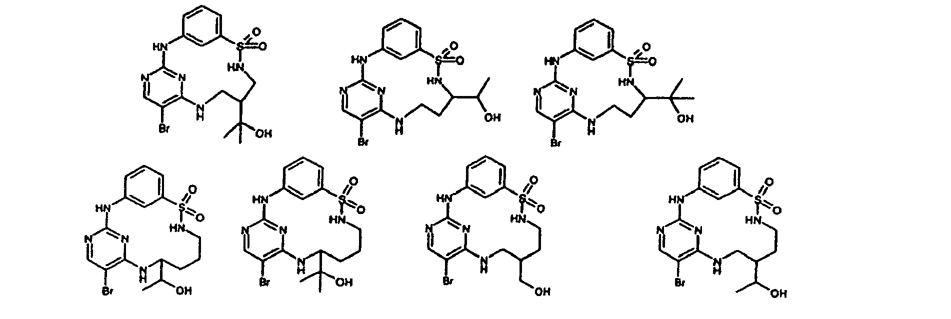 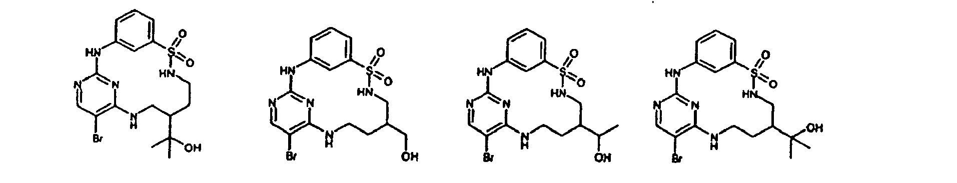 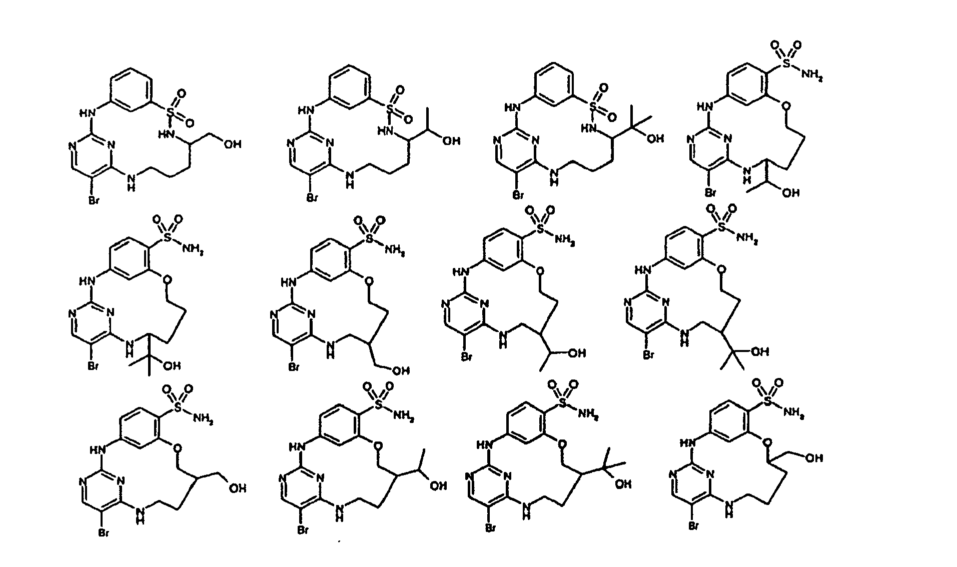 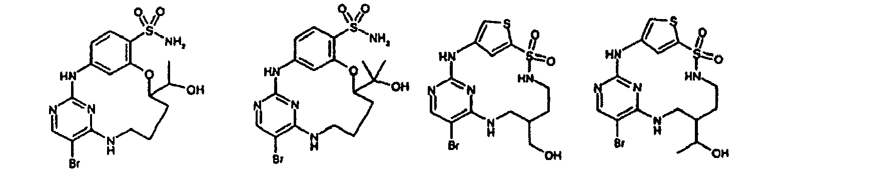 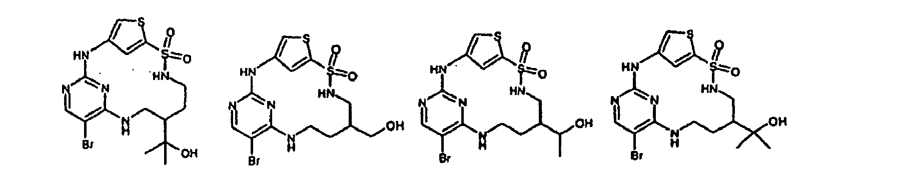 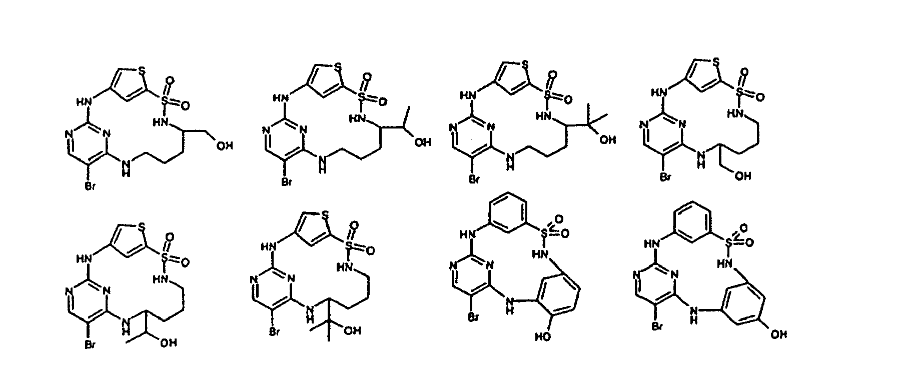 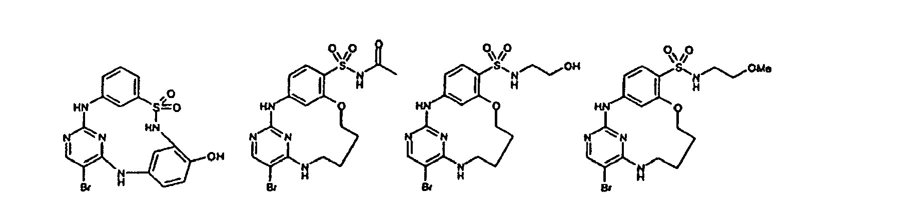 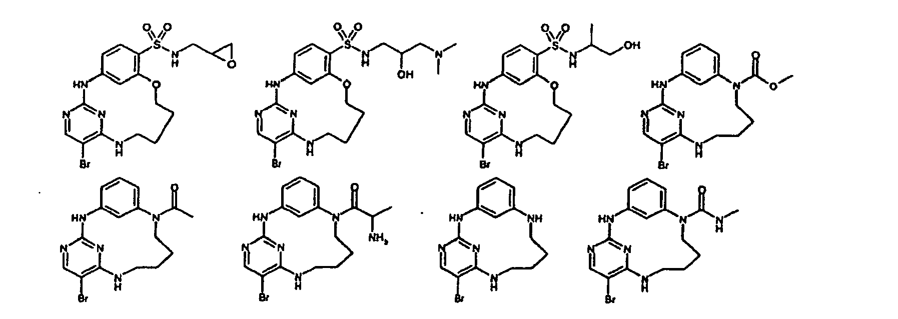 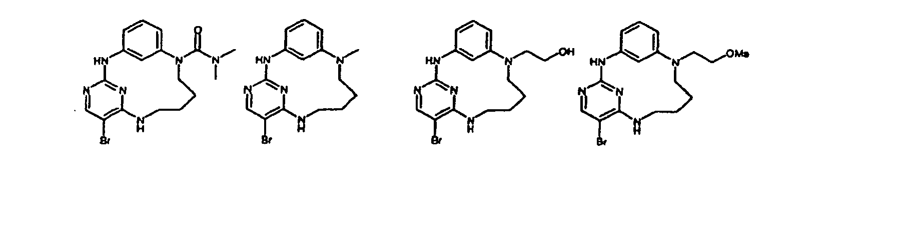 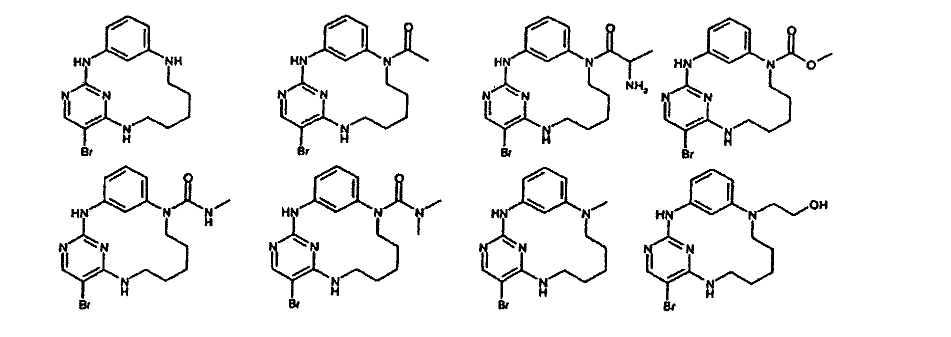 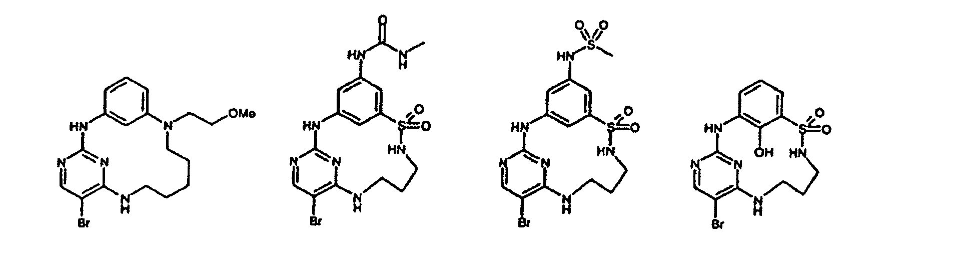 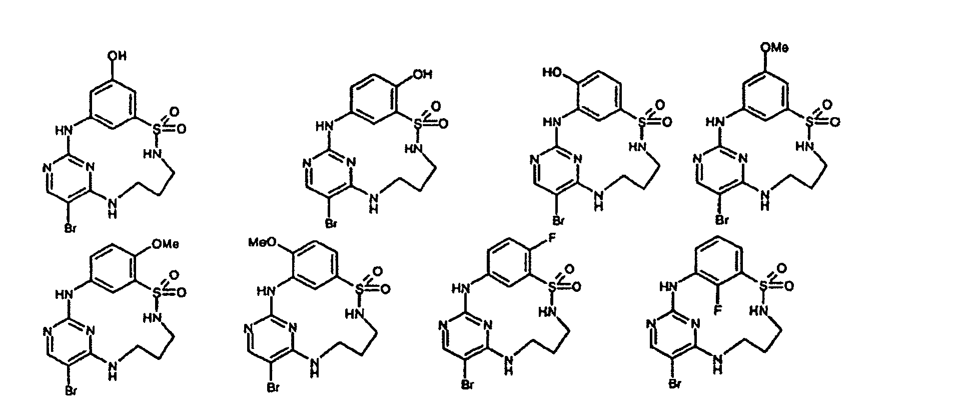 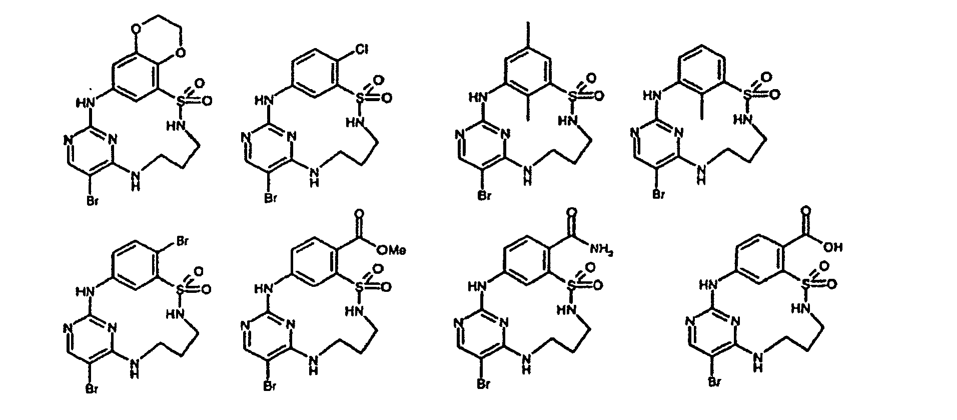 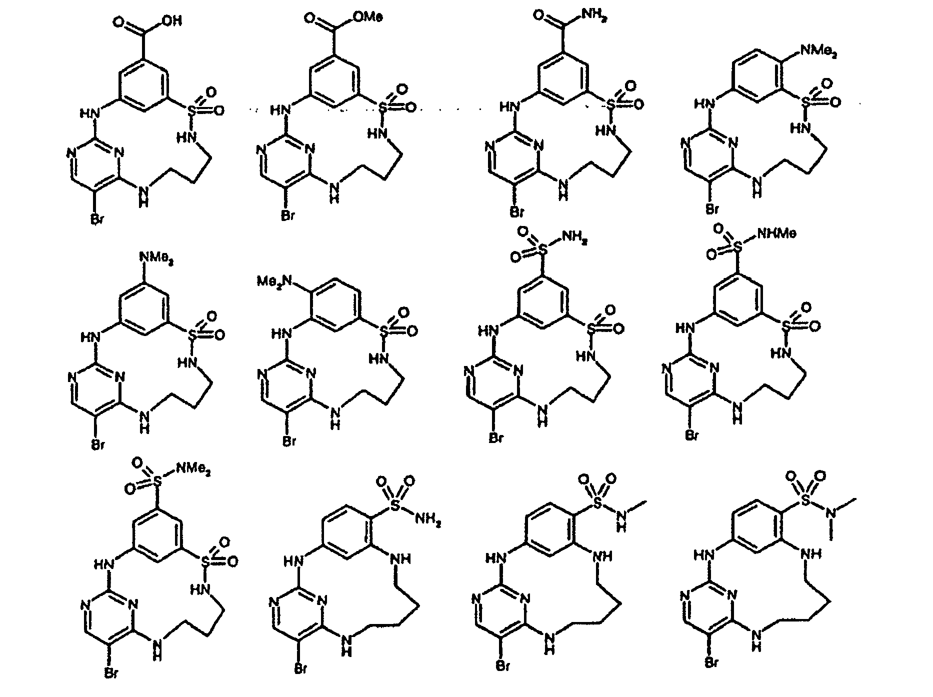 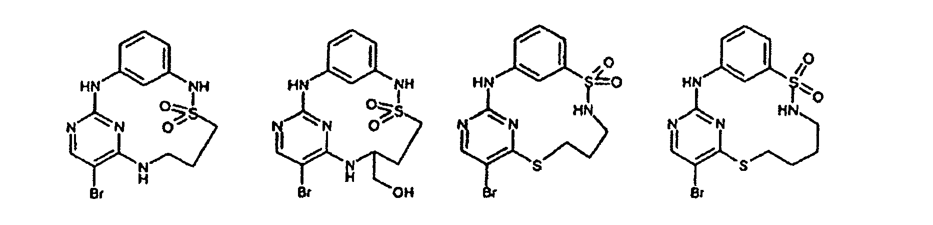 ainsi que leurs diastéréoisomères, leurs énantiomères et leurs sels.

4. Utilisation d'un composé selon l'une quelconque des revendications 1 à 3 pour la production d'un médicament.

5. Utilisation d'un composé selon l'une quelconque des revendications 1 à 3 pour la production d'un médicament destiné au traitement d'un cancer, d'un angiofibrome, de l'arthrite, de maladies oculaires, de maladies autoimmunes, d'une alopécie et d'une inflammation de muqueuse provoquées par voie chimiothérapeutique, de la maladie de Crohn, de l'endométriose, de maladies fibreuses, d'un hémangiome, de maladies cardiovasculaires, de maladies infectieuses, de maladies néphrologiques, de maladies neurodégénératives chroniques et aigues, ainsi que de lésions du tissu nerveux, d'infections virales, pour l'inhibition de la réocclusion de vaisseaux après un traitement par cathéters à ballonnet, dans le cas de prothèses vasculaires ou après l'insertion de dispositifs mécaniques pour maintenir des vaisseaux ouverts, tels que, par exemple, des stents, en tant qu'agents immunosuppressifs, pour favoriser la cicatrisation sans marques, dans le cas de tâches dues à la vieillesse et dans le cas de la dermatite de contact.

6. Utilisation selon la revendication 5, **caractérisée en ce que**
par cancer, on entend des tumeurs solides, une croissance de tumeur ou de métastases, le sarcome de Kaposi, la maladie de Hodgkin et la leucémie, par arthrite, on entend la polyarthrite rhumatoïde,
par maladies oculaires, on entend la rétinopathie diabétique, le glaucome néovasculaire,
par maladies autoimmunes, on entend le psoriasis, l'alopécie et la sclérose en plaques,
par maladies fibreuses, on entend la cirrhose du foie, des maladies prolifératives des cellules mésangiales, l'artériosclérose,
par maladies infectieuses, on entend des maladies causées par des parasites unicellulaires,
par maladies cardiovasculaires, on entend des sténoses, telles que, par exemple, une resténose provoquée par un stent, des artérioscléroses et des resténoses,
par maladies néphrologiques, on entend la glomérulonéphrite, la néphropathie diabétique, la néphrosclérose maligne, des syndromes microangiopathiques thrombiques, des rejets de greffes et la glomérulopathie,
par maladies neurodégénératives chroniques, on entend la maladie de Huntington, la sclérose latérale amyotrophique, la maladie de Parkinson, le SIDA, la démence et la maladie d'Alzheimer, par maladies neurodégénératives aigues, on entend des ischémies du cerveau et des neurotraumatismes, et par infections virales, on entend des infections par cytomégalovirus, l'herpès, l'hépatite B ou C, et des maladies dues au HIV.

7. Médicament contenant au moins un composé selon l'une quelconque des revendications 1 à 3.

8. Médicament selon la revendication 7, destiné au traitement d'un cancer, d'un angiofibrome, de l'arthrite, de maladies oculaires, de maladies autoimmunes, d'une alopécie et d'une inflammation de muqueuse provoquées par voie chimiothérapeutique, de la maladie de Crohn, de l'endométriose, de maladies fibreuses, d'un hémangiome, de maladies cardiovasculaires, de maladies infectieuses, de maladies néphrologiques, de maladies neurodégénératives chroniques et aigues, ainsi que de lésions du tissu nerveux, d'infections virales, pour l'inhibition de la réocclusion de vaisseaux après un traitement par cathéters à ballonnet, dans le cas de prothèses vasculaires ou après l'insertion de dispositifs mécaniques pour maintenir des vaisseaux ouverts, tels que, par exemple, des stents, en tant qu'agents immunosuppressifs, pour favoriser la cicatrisation sans marques, dans le cas de tâches dues à la vieillesse et dans le cas de la dermatite de contact.

9. Médicament destiné à l'utilisation selon la revendication 8, où
par cancer, on entend des tumeurs solides, une croissance de tumeur ou de métastases, le sarcome de Kaposi, la maladie de Hodgkin et la leucémie, par arthrite, on entend la polyarthrite rhumatoïde,
par maladies oculaires, on entend la rétinopathie diabétique, le glaucome néovasculaire,
par maladies autoimmunes, on entend le psoriasis, l'alopécie et la sclérose en plaques,
par maladies fibreuses, on entend la cirrhose du foie, des maladies prolifératives des cellules mésangiales, l'artériosclérose,
par maladies infectieuses, on entend des maladies causées par des parasites unicellulaires,
par maladies cardiovasculaires, on entend des sténoses, telles que, par exemple, une resténose provoquée par un stent, des artérioscléroses et des resténoses,
par maladies néphrologiques, on entend la glomérulonéphrite, la néphropathie diabétique, la néphrosclérose maligne, des syndromes microangiopathiques thrombiques, des rejets de greffes et la glomérulopathie,
par maladies neurodégénératives chroniques, on entend la maladie de Huntington, la sclérose latérale amyotrophique, la maladie de Parkinson, le SIDA, la démence et la maladie d'Alzheimer, par maladies neurodégénératives aigues, on entend des ischémies du cerveau et des neurotraumatismes, et par infections virales, on entend des infections par cytomégalovirus, l'herpès, l'hépatite B ou C, et des maladies dues au HIV.
